(19) Europäisches Patentamt
European Patent Office
Office européen des brevets

(11) **EP 2 564 779 B1**

(12) **EUROPEAN PATENT SPECIFICATION**

(45) Date of publication and mention of the grant of the patent:
**30.08.2017 Bulletin 2017/35**

(21) Application number: **11774701.4**

(22) Date of filing: **02.03.2011**

(51) Int Cl.:
***H01L 27/146*** *(2006.01)* ***A61B 6/00*** *(2006.01)*
***H04N 5/32*** *(2006.01)*

(86) International application number:
**PCT/JP2011/054685**

(87) International publication number:
**WO 2011/135917 (03.11.2011 Gazette 2011/44)**

(54) **RADIATION IMAGE PHOTOGRAPHY DEVICE**

VORRICHTUNG ZUR AUFNAHME VON RÖNTGENBILDERN

DISPOSITIF DE PHOTOGRAPHIE D'IMAGE RADIOGRAPHIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.04.2010 JP 2010104935**

(43) Date of publication of application:
**06.03.2013 Bulletin 2013/10**

(73) Proprietor: **Konica Minolta Medical & Graphic, Inc.**
**Tokyo 191-8511 (JP)**

(72) Inventor: **TAJIMA, Hideaki**
**Hino-shi**
**Tokyo 191-8511 (JP)**

(74) Representative: **Stanners, David Ralph**
**Urquhart-Dykes & Lord LLP**
**Altius House**
**1 North Fourth Street**
**Milton Keynes MK9 1NE (GB)**

(56) References cited:
**WO-A1-2009/005119**
**JP-A- 9 197 051**
**JP-A- 11 155 847**
**JP-A- 2007 151 761**
**JP-A- 2008 000 595**
**JP-A- 2008 132 216**
**JP-A- 2009 219 538**
**US-A1- 2003 086 523**
**US-A1- 2005 285 043**
**US-B1- 6 404 854**
**US-B1- 7 211 802**
**US-B1- 7 211 803**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 564 779 B1

Printed by Jouve, 75001 PARIS (FR)

**Description**

TECHNICAL FIELD

The present invention relates to a radiation image capturing apparatus, particularly to a radiation image capturing apparatus capable of detecting initiation of radioactive irradiation etc. by itself.

BACKGROUND ART

There has been development of various types of radiation image capturing apparatuses including a so-called direct-type radiation image capturing apparatus that generates an electric charge through a detection element in response to an irradiation dose of radiation such as X-rays and converts the electric charge into an electric signal, and a so-called indirect-type radiation image capturing apparatus that uses a scintillator etc. to convert the applied radiation into electromagnetic waves such as visible light having other wavelengths, then generates an electric charge through a photoelectric conversion element such as a photodiode in response to the energy of the electromagnetic wave having been converted and applied, and converts the electric charge into an electric signal. It should be noted that the detection element in the direct-type radiation image capturing apparatus and the photoelectric conversion element in the indirect-type radiation image capturing apparatus are collectively referred to as radiation detection elements in this invention.

This type of radiation image capturing apparatus is known as a FPD (flat panel detector) and was previously formed integrally with a supporting stand (or a bucky device) (see Patent document 1, for example), but, in recent years, a portable radiation image capturing apparatus, in which radiation detection elements and the like are stored in a housing, has been developed and put into practical use (see Patent documents 2 and 3, for example).

Such a radiation image capturing apparatus is often configured so that radioactive irradiation is performed during radiation image capturing operation in such a way that a radiation generator, which doses radioactive irradiation to the radiation image capturing apparatus, transmits a signal indicating that radiation will be performed, and the radiation image capturing apparatus side transmits a signal which permits the radiation to the radiation generator side.

However, in the case of this configuration, it is required that an interface should be constructed in a precise manner between the radiation image capturing apparatus and the radiation generator, and the radiation image capturing apparatus side should be able to accumulate electric charges in each of the radiation detection elements at the stage of radioactive irradiation, but construction of an interface between the devices is not necessarily easy.

There has been a problem that, if the interface is not constructed precisely, radioactive irradiation begins while a reset process is executed on the side of the radiation image capturing apparatus for discharging excessive electric charges remaining in each of the radiation detection elements, and electric charges generated by the radioactive irradiation, in other words, electric charges which should be read out without fail as useful information regarding the subject is reflected in the amount thereof escapes from each of the radiation detection elements in the reset process, causing decline of conversion efficiency of radiation into electric charges, namely, image data.

Thus, in recent years, various technologies have been developed aiming at a radiation image capturing apparatus which detects radioactive irradiation in itself without depending on such an interface between the radiation image capturing apparatus and a radiation generator.

For example, in the inventions described in Patent document 4 and Patent document 5, current detection unit is provided in a bias line to detect a current value of a current flowing in the bias line and detects initiation of radioactive irradiation based on increase and decrease of the value, by utilizing the fact that, once radioactive irradiation of a radiation image capturing apparatus begins and electric charges are generated in each of the radiation detection elements, the electric charges escapes from each of the radiation detection elements into the bias line connected to each of the radiation detection elements, and the current which flows in the bias line increases.

PRIOR ART DOCUMENTS

PATENT DOCUMENTS

Patent document 1: JP-A-09-73144
Patent document 2: JP-A-2006-058124
Patent document 3: JP-A-06-342099
Patent document 4: Specification of the U.S. Pat No. 7211803
Patent document 5: JP-A-2009-219538

SUMMARY OF THE INVENTION

PROBLEMS TO BE SOLVED BY THE INVENTION

However, the bias line is usually connected to an electrode of each of the radiation detection elements. Therefore, when current detection unit is provided in the bias line as described above, noise generated in the current detection unit is propagated to each of the radiation detection elements through the bias line, and a noise component caused by the noise generated in the current detection unit is superimposed on the electric charges generated in each of the radiation detection unit by radioactive irradiation, in other words, image data.

Whether or not the current detection unit is provided in a bias line, the above-mentioned problem happens as an unavoidable issue when a radiation image capturing apparatus is configured so that the current detection unit which is newly provided in the radiation image capturing apparatus is used to detect an increase of a current value of a current flowing in each line in the radiation image capturing apparatus due to radioactive irradiation.

Then, as described above, once a noise component caused by a noise generated in the current detection unit is superimposed on image data, a radiological image generated based on such image data usually suffers from deterioration of image quality. Then, with a deteriorated image quality, it becomes very difficult to see the radioactive image, and, when the radioactive image is used for, for example, diagnostic purposes and the like in medicine, a medical doctor who looks at the radioactive image may overlook an affected area captured in the image or misdiagnoses that there is a lesion in an area which is not an affected area.

However, it is not necessarily easy to remove a noise component caused by a noise generated in the current detection unit from the image data by performing image processing on the image data obtained. Although it is possible to configure the radiation image capturing apparatus so that a noise generated in the current detection unit is not propagated to the radiation detection elements by, for example, providing a new circuit or the like, new problems arise such as that control is required for the new circuit or the like and additional power is consumed due to the new circuit or the like.

The present invention has been accomplished in view of the above-mentioned problems, and aims to provide a radiation image capturing apparatus which can at least detect initiation of radioactive irradiation accurately on its own by using each existing units in the device, without providing new unit in the device. The present invention also aims to provide a radiation image capturing apparatus which is able to improve image quality of a radioactive image generated based on obtained image data.

MEANS FOR SOLVING THE PROBLEMS

In order to solve at least one of the aforementioned problems, a radiation image capturing apparatus according to claim 1.

data readout process in which image data is read out from the radiation detection elements;

switch units each connected to each of the scanning lines, discharges electric charges accumulated in the radiation detection elements to the signal lines when the on voltage is applied thereto through the scanning lines, and accumulates electric charges in the radiation detection elements when an off voltage is applied thereto through the scanning lines;

reading circuits which convert the electric charges discharged to the signal lines from the radiation detection elements into the image data and read out the image data during the image data readout process; and

a controller which controls at least the scanning drive unit and the reading circuits and causes the same to execute the data readout process from the radiation detection elements,

wherein the controller causes the reading circuits to periodically perform a readout operation before radiation image capturing operation in a state where each of the switch units is in an off state by applying the off voltage to all of the scanning lines from the scanning drive unit, causes the reading circuits to repeatedly execute a leaked data readout process in which the electric charges leaked from the radiation detection elements through the switch units are converted into leaked data, and detects initiation of irradiation at a point when the read-out leaked data exceeds a threshold value.

ADVANTAGEOUS EFFECT OF THE INVENTION

According to the radiation image capturing apparatus in the form of the present invention, electric charges leaked from the radiation detection elements through the switch unit are read out as leaked data by using the reading circuit provided in a normal radiation image capturing apparatus, and initiation of radioactive irradiation is detected based on an increase of the leaked data. Hence, without constructing an interface with a radiation generator, the radiation image capturing apparatus can at least detect initiation of radioactive irradiation accurately on its own by utilizing the properties of the switch unit that a leakage current flowing through the switch unit is increased due to radioactive irradiation.

Also, at the same time, since the radiation image capturing apparatus can at least detect initiation of radioactive irradiation accurately on its own without providing new units such as the current detection unit in the device, no additional power is consumed due to the new units such as the current detection unit, and a noise generated in the new units is not superimposed on image data read out from each of the radiation detection elements, thus making it possible to improve image quality of a radiological image which is generated based on the image data.

BRIEF DESCRIPTION OF THE DRAWINGS

Fig. 1 is a perspective view showing a radiation image capturing apparatus according to the present embodiment.
Fig. 2 is a cross-sectional view along the line X-X in Fig. 1.
Fig. 3 is a plan view showing a configuration of a substrate of the radiation image capturing apparatus.
Fig. 4 is an enlarged view illustrating configuration of radiation detection elements, TFTs, and so on formed in small regions on the substrate shown in Fig. 3.
Fig. 5 is a cross-sectional view along the line Y-Y in Fig. 4.
Fig. 6 is a side view explaining the substrate onto which a COF, a PCB substrate, and so on are attached.
Fig. 7 is a block diagram showing an equivalent circuit of the radiation image capturing apparatus.
Fig. 8 is a block diagram showing an equivalent circuit for one pixel which constructs a detecting section.
Fig. 9 is a timing chart showing timing when a voltage to be applied to each scanning line is switched between on voltage and off voltage in an image data readout process.
Fig. 10 is a timing chart showing on/off timing of an electric charge reset switch, pulse signals, and the TFTs in the image data readout process.
Fig. 11 is a graph showing changes and the like of a voltage value in a correlated double sampling circuit.
Fig. 12 is a timing chart showing on/off timing of the electric charge reset switch, the pulse signals, and the TFTs in a leaked data readout process.
Fig. 13 is a timing chart showing on/off timing of the electric charge reset switch, the pulse signals, and the TFTs in the leaked data readout process which is periodically repeated before the radiation image capturing operation.
Fig. 14 is a view explaining each electric charge leaked from each of the radiation detection elements through each of the TFTs, and is a view explaining a relation between each of the electric charges and leaked data.
Fig. 15 is a graph in which time series of read-out leaked data is plotted, and is a graph explaining that the value of leaked data increases once radioactive irradiation is initiated.
Fig. 16 is a graph showing temperature dependence of a leakage current which flows in the TFT in an off state and a current which flows in the TFT in an on state.
Fig. 17 is a graph showing an example where the maximum value among each leaked data read out in the leaked data readout process which is repeated periodically increases over time along an increase of the temperature of the TFT.
Fig. 18A is a graph showing an example of a transition of a threshold value over time calculated based on moving average of the maximum values of leaked data.
Fig. 18B is a graph showing an example of a transition of the threshold value over time calculated based on the maximum values of peak-held leaked data.
Fig. 19 is a timing chart showing on/off timing of the electric charge reset switch, the pulse signals, and the TFTs when the reset process of each of the radiation detection elements is executed in the leaked data readout process which is periodically repeated.
Fig. 20 is a timing chart showing on/off timing of the electric charge reset switch, the pulse signals, and the TFTs when the image data readout process from each of the radiation detection elemnts is executed in the leaked data readout process which is periodically repeated.
Fig. 21 is a graph showing an example of leaked data read out in each leaked data readout process when the radiation image capturing apparatus is irradiated with very weak radiation.
Fig. 22 is a view illustrating an example of a position of radioactive irradiation on a scintillator and a detecting section with a narrowed irradiation field, as well as each of the signal lines.
Fig. 23 is a graph showing a difference calculated using the example of leaked data shown in Fig. 21.
Fig. 24 is a block diagram illustrating each reading IC to which a plurality of signal lines is connected and in which a plurality of reading circuits are formed.
Fig. 25 is an illustration of the scintillator and the detecting section viewed from the side of the radiation entrance face of the device, and is a view explaining a position on the detecting section from which electromagnetic waves radiated from the scintillator can enter and a position where the electromagnetic waves do not enter.
Fig. 26 is a block diagram showing an equivalent circuit for one pixel, which constructs the detecting section in a configuration where a capacity of a capacitor of an amplifier circuit can be changed.

Fig. 27 is a schematic view explaining that a leakage current flowing in the TFT flows through a region of a semiconductor layer on a gate electrode side where density of electrons is small.
Fig. 28 is a cross-sectional view explaining a wire arranged in the TFT on the scintillator side.
Fig. 29 is a timing chart showing on/off timing of the electric charge reset switch, the pulse signals, and the TFTs in a case where time span for sending pulse signals is extended in the leaked data readout process which is repeated periodically.
Fig. 30 is a graph showing a voltage value which increases in the correlated double sampling circuit in the leaked data readout process, and a noise component which is superimposed on the voltage value.
Fig. 31 is a timing chart showing on/off timing of the electric charge reset switch, the pulse signals and the TFTs in a case where initiation of radioactive irradiation is detected in the fourth round of leakage readout process in the leaked data readout process which is repeated periodically.
Fig. 32 is a view explaining a line defect which occurs on a radioactive image in the case of Fig. 31.
Fig. 33 is a timing chart showing on/off timing of the electric charge reset switch, the pulse signals, and the TFTs in a case where initiation of radioactive irradiation is detected in the fifth round of leakage readout process in the leaked data readout process which is repeated periodically.

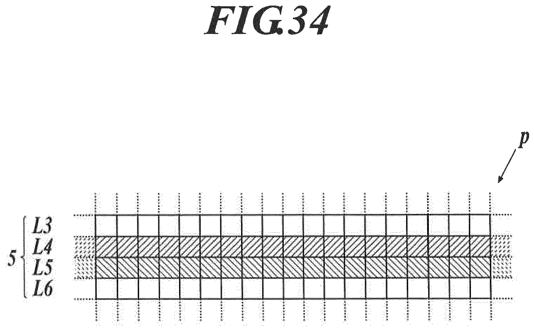

Fig. 34 is a view explaining that line defects occurs in a row on a radioactive image in the case of Fig. 33.
Fig. 35 is a timing chart showing an example of on/off timing when the reset process is executed for each of the radiation detection elements by sequentially applying an on voltage to the scanning lines except for neighboring scanning lines in the leaked data readout process which is repeated periodically.
Fig. 36 is a timing chart showing an example of on/off timing when image data readout process for each of radiation detection elements is executed by applying an on voltage to the plurality of scanning lines simultaneously in the leaked data readout process which is repeated periodically.
Fig. 37 is a timing chart of leaked data readout process and so on, the electric charge accumulation mode, and the image data readout process in a case where readout operations by the reading circuits are stopped and the electrical charge accumulation mode is executed.
Fig. 38 is a timing chart of leaked data readout process and so on, the electric charge accumulation mode, and the image data readout process in a case where readout operations by the reading circuits are continued and the electrical charge accumulation mode is executed.
Fig. 39 is a graph explaining that the leaked data read out in the case of Fig. 38 increases above the threshold value due to initiation of radioactive irradiation, and decreases to a value equal to or below the threshold value due to end of the radioactive irradiation.
Fig. 40 is a graph explaining that, when the leaked data increases due to a big noise or the like superimposed thereon, the leaked data read out in the next leaked data readout process returns to a previous value equal to or below the threshold value.
Fig. 41 is a timing chart explaining off periods of the TFTs, and explaining that the off periods of the TFTs are different time spans in the respective scanning lines.
Fig. 42 is a timing chart in a case where an offset correction value is read out by repeating the same process sequence as the process sequence in reading out the image data, after the image data readout process.
Fig. 43 is a timing chart of the leaked data readout process and the like, the electric accumulation mode, and the image data readout process, in a case where the image data readout process is executed by starting applying an on voltage from the scanning line that follows the leaked data readout process in which initiation of radioactive irradiation was detected.
Fig. 44 is a timing chart showing on/off timing of the electric charge reset switch, the pulse signals, and the TFTs in a case where the on/off operations are performed after the image data readout process at the same time as each of the previous processes in the case of Fig. 43.
Fig. 45 is a timing chart of the leaked data readout process and the like in the electric accumulation mode and the image data readout process, in a case where the image data readout process is executed by starting applying an on voltage from the first scanning line.
Fig. 46 is a timing chart of showing on/off timing of the electric charge reset switch, the pulse signals, and the TFTs in a case where the on/off operations are performed after the image data readout process at the same time as each of the previous processes in the case of Fig. 45.
Fig. 47 is a timing chart in a case where the offset correction value readout process is executed such that the off periods of the TFTs before the radiation image capturing operation and the off periods of the TFTs from the image data readout process until the offset correction value readout process become equal to each other.
Fig. 48 is a timing chart in a case where the reset process is executed for each of the radioactive detection elements after the image data readout process in the case of Fig. 47.
Fig. 49 is a timing chart in a case where the offset correction value readout process is performed immediately after the image data readout process or after lapse of a given period of time.

Fig. 50 is a table showing a relation between the off period of the TFT and a reference offset correction value, or a graph showing the relational expression of the same.

Fig. 51 is a timing chart showing on/off timing of the TFT, and a graph showing that an offset due to a lag per unit time and an offset due to a lag which is an integral value of the said offsets increase over time.

Fig. 52A is a graph explaining offsets due to lags in the respective scanning lines when the processes in Fig. 43 and the like are executed.

Fig. 52B is a graph explaining offsets due to lags in the respective scanning lines when the processes in Fig. 45 and the like are executed.

Fig. 53 is a timing chart of the leaked data readout process and the like, the electric charge accumulation mode, and the image data readout process in a third embodiment.

## DETAILED DESCRIPTION OF THE PREFERRED EMBODIMENTS

Hereinafter, embodiments of the radiation image capturing apparatus according to the present invention will be explained with reference to the drawings.

It should be noted that, although the explanation below is about a case where the radiation image capturing apparatus is a so-called indirect-type radiation image capturing apparatus which is provided with a scintillator and so on, and obtains an electric signal by converting radiation into an electromagnetic wave such as visible light having other wavelength, the present invention is also applicable to a direct-type radiation image capturing apparatus. In addition, although the explanation pertains to a case where the radiation image capturing apparatus is a portable type, the present invention is also applied to a radiation image capturing apparatus which is integrally formed with a supporting stand or the like.

### [First Embodiment]

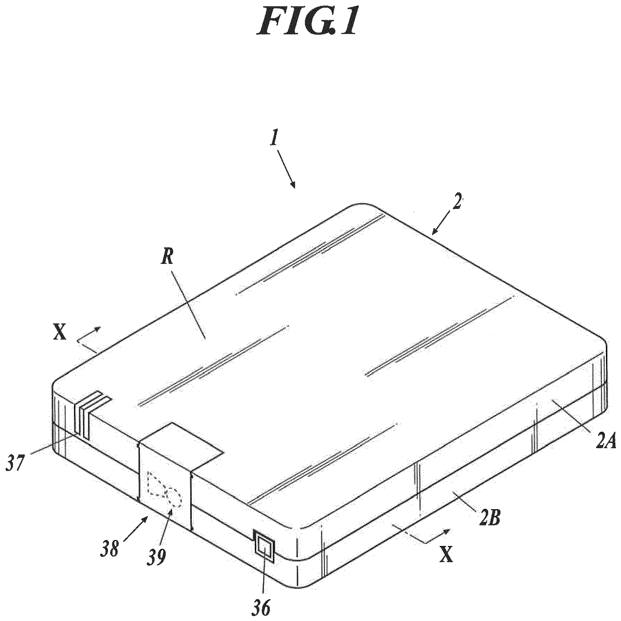

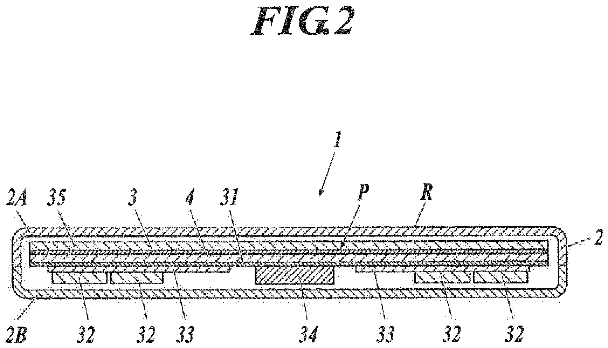

Fig. 1 is an external perspective view of a radiation image capturing apparatus according to this embodiment, and Fig. 2 is a cross-sectional view along the line X-X in Fig. 1. The radiation image capturing apparatus 1 according to this embodiment is constructed by storing a scintillator 3, a substrate 4 and so on in a case 2 as illustrated in Figs. 1 and 2.

In the case 2, at least a radiation entrance face R is formed from a material such as a carbon plate and plastic through which radiation can pass. Figs. 1 and 2 show a case where the case 2 has a so-called rectangular lunch-box shape, formed of a frame plate 2A and a back plate 2B, but the case 2 may also have a so-called monocoque shape in which the case 2 is formed integrally into a square tube shape.

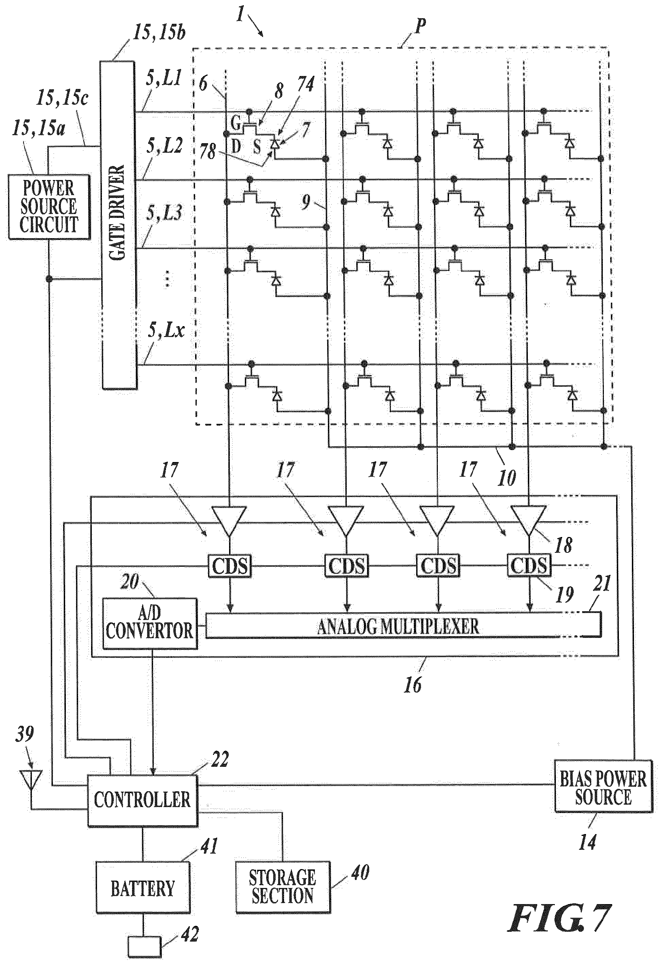

Further, as illustrated in Fig. 1, a power source switch 36, an indicator 37 constructed by LED or the like, a cover member 38 which can be opened and closed for changing a battery 41 (see Fig. 7 described later), and the like are arranged on a side surface part of the case 2. In addition, in this embodiment, an antenna device 39 is embedded in the side surface part of the cover member 38 for wirelessly sending and receiving information such as later-described image data d with an external device such as an image processing computer.

Also, the installation position of the antenna device 39 is not limited to the side surface part of the cover member 38, and the antenna device 39 may be installed at any position in the radiation image capturing apparatus 1. Further, the number of the antenna device 39 is not limited to one and may be more than one. Moreover, the antenna device 39 may be constructed to send and receive image data d and the like with the external device in a wired form such as a cable, and, in such a case, a connecting terminal or the like is provided on the side surface part or the like of the radiation image capturing apparatus 1 for establishing connection by inserting a cable or the like thereinto.

As shown in Fig. 2, in the case 2, a base 31 is located through a non-illustrated thin lead plate or the like on the lower side of the substrate 4, and a PCB substrate 33 on which electronic components 32 and the like are arranged, a buffer member 34, and so on are attached on the base 31. It should be noted that, in this embodiment, a glass substrate 35 is arranged on the radiation entrance face R of the substrate 4 and the scintillator 3 for protecting the substrate 4 and the scintillator 3.

The scintillator 3 is located to face a later-described detecting section P of the substrate 4. The scintillator 3 is formed mostly of, for example, a fluorescent material, and, once radioactive irradiation is received, the one used here as the scintillator 3 converts the radiation into an electromagnetic wave having a wavelength of between 300 and 800 nm, in other words, an electromagnetic wave which is mainly visible light.

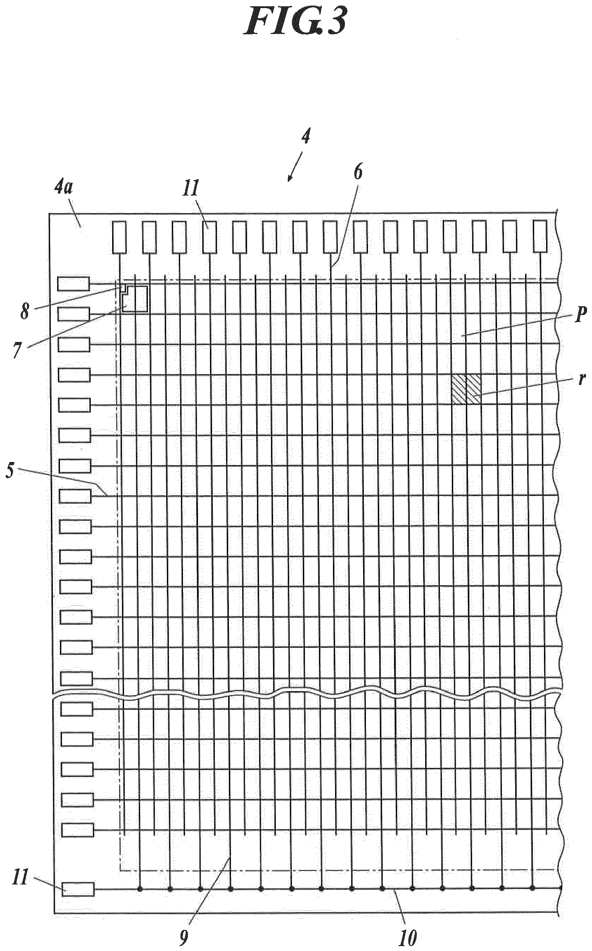

The substrate 4 in this embodiment is constructed by a glass substrate, and, as illustrated in Fig. 3, a plurality of scanning lines 5 and a plurality of signal lines 6 are arranged to intersect with each other on a surface 4a of the substrate 4 which faces the scintillator 3. Radiation detection elements 7 are respectively provided in small regions r that are partitioned by the plurality of scanning lines 5 and the plurality of signal lines 6 on the surface 4a of the substrate 4.

According to the foregoing, the entire regions r on which the plurality of radiation detection elements 7 are respectively provided in the small regions r partitioned by the scanning lines 5 and the signal lines 6 in a two-dimensional arrangement as describe above, in other words, the region indicated by a dashed line in Fig. 3, is regarded as the detecting section P.

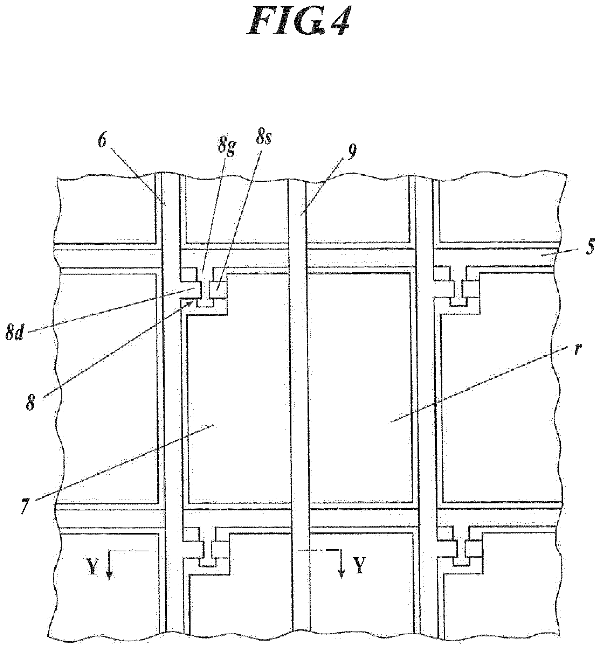

In this embodiment, although photodiodes are used as the radiation detection elements 7, other materials such as phototransistors may be used. As illustrated in the enlarged views of Figs. 3 and 4, each of the radiation detection elements 7 is connected to a source electrode 8s of a TFT 8 which serves as a switch unit. Incidentally, a drain electrode 8d of the TFT 8 is connected to the signal line 6.

Then, once an on voltage is applied by later-described scanning drive unit 15 to the scanning line 5 connected to the TFT8 and the on-voltage is applied to the gate electrode 8g through the scanning line 5, the TFT 8 enters an on state, which causes the signal line 6 to discharge an electric charge accumulated in the radiation detection element 7. Moreover, when an off voltage is applied to the scanning line 5 connected to the TFT 8 and the off voltage is applied to the gate electrode 8g through the scanning line 5, the TFT 8 enters into an off state, stops discharging of an electric charge from the radiation detection element 7 to the signal line 6, and causes the radiation detection element 7 to hold and accumulate electric charges.

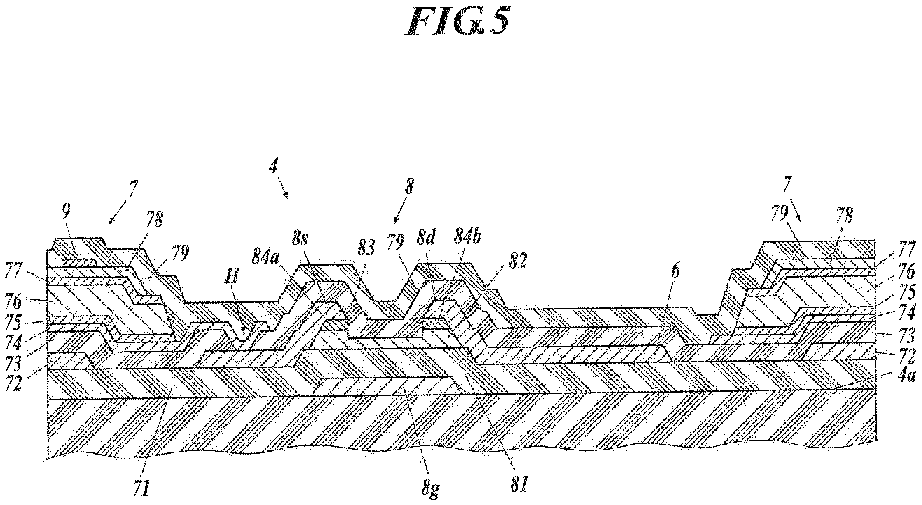

Here, the constructions of the radiation detection element 7 and the TFT 8 in this embodiment will be briefly explained using the cross-sectional view illustrated in Fig. 5. Fig. 5 is a cross-sectional view along the line Y-Y in Fig. 4.

On the surface 4a of the substrate 4, the gate electrode 8g of the TFT 8, made from Al, Cr, or the like, is formed in a laminated manner integrally with the scanning line 5, and, in an area above the gate electrode 8g on a gate insulating layer 81 made from silicon nitride ($SiN_x$) or the like laminated on the gate electrode 8g and the surface 4a, the source electrode 8s connected to a first electrode 74 of the radiation detection element 7 and the drain electrode 8d integrally formed with the signal line 6 are formed in a laminated manner through a semiconductor layer 82 made from hydrogenated amorphous silicon (a-Si) or the like.

The source electrode 8s and the drain electrode 8d are divided by a first passivation layer 83 made from nitride silicon ($SiN_x$) or the line, and the first passivation layer 83 also covers the both electrodes 8s and 8d from above. Further, ohmic contact layers 84a and 84b formed into n-type by doping VI-group elements into hydrogenated amorphous silicon are laminated respectively between the semiconductor layer 82, and the source electrode 8s and the drain electrode 8d. In this way, the TFT 8 is formed.

In the portion of the radiation detection element 7, Al, Cr or the like is laminated to form an auxiliary electrode 72 on an insulating layer 71 which is formed integrally with the gate insulating layer 81 on the surface 4a of the substrate 4, and the first electrode 74 made from Al, Cr, Mo or the like is laminated on the auxiliary electrode 72 through an insulating layer 73 which is integrally formed with the first passivation layer 83. The first electrode 74 is connected to the source electrode 8s of the TFT 8 through a hole H formed in the first passivation layer 83. Note that the auxiliary electrode 72 may not be necessarily provided.

On the first electrode 74, an n layer 75 formed into the n type by doping VI-group elements into hydrogenated amorphous silicon, an i layer 76 which is a converting layer formed from hydrogenated amorphous silicon, and a p layer 77 formed into the p type by doping III-group elements into hydrogenated amorphous silicon are formed in a laminated fashion in this order from the bottom.

Then, during the radiation image capturing operation, once radiation emitted to the radiation image capturing apparatus 1 enters from the radiation entrance face R of the case 2 and is converted into a electromagnetic wave such as visible light by the scintillator 3, and the converted electromagnetic wave is emitted from above in the drawing, the electromagnetic wave reaches the i layer 76 of the radiation detection element 7, and an electron-hole pair is generated in the i layer 76. This way, the radiation detection element 7 converts an electromagnetic wave emitted from the scintillator 3 into an electric charge (electron-hole pair).

Also, a second electrode 78 which is a transparent electrode made from ITO or the like is formed in a laminated fashion on the p layer 77, and is constructed so that an electromagnetic wave emitted reaches the i layer 76 and the like. In this embodiment, the radiation detection element 7 is formed in the way described above. It should be noted that the lamination order of the p layer 77, the i layer 76, and the n layer 75 may be upside down in the reverse order. Further, in this embodiment, it is explained that a so-called pin-type radiation detection element formed by laminating the p layer 77, the i layer 76, and the n layer 75 in this order as described above is used as the radiation detection element 7, but the radiation detection element 7 is not limited thereto.

On the upper surface of the second electrode 78 of the radiation detection element 7, a bias line 9 is connected, which applies a bias voltage to the radiation detection element 7 via the second electrode 78. It should be noted that the second electrode 78 and the bias line 9 of the radiation detection element 7, the first electrode 74 extended to the TFT 8 side, the first passivation layer 83 of the TFT 8, and the like, in other words, the upper surface areas of the radiation detection element 7 and the TFT 8 are coated by a second passivation layer 79 made from silicon nitride ($SiN_x$) from the upper side thereof.

As illustrated in Figs. 3 and 4, in this embodiment, one bias line 9 is connected to the plurality of radiation detection elements 7 which are respectively arranged in line, and each of the bias lines 9 is provided to be parallel to each of the signal lines 6. Also, each of the bias lines 9 is bundled to the wire connection 10 at a position on the outer side of the detecting section P of the substrate 4.

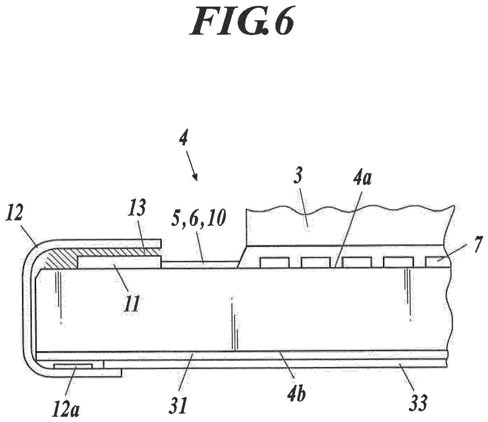

In this embodiment, as illustrated in Fig. 3, the scanning lines 5, the signal lines 6, and the wire connection 10 of the bias line 9 are connected to input/output terminals (or pads) 11 provided near an end edge part of the substrate 4, respectively. As illustrated in Fig. 6, a COF (chip on film) 12 is connected to each of the input/output terminals 11 via an anisotropic conductive adhesive material 13 such as an anisotropic conductive film or an anisotropic conductive paste, and, in the COF, a chip such as a gate IC 12a which constructs a gate driver 15b of the later-described scanning drive unit 15 is embedded in a film.

Also, the COF 12 is drawn around to a back surface 4b side of the substrate 4 and is connected to the previously-mentioned PCB substrate 33 on the back surface 4b side. This way, the portion of the substrate 4 in the radiation image capturing apparatus 1 is configured. Note that illustration of the electronic components 32 and so on is omitted in Fig. 6.

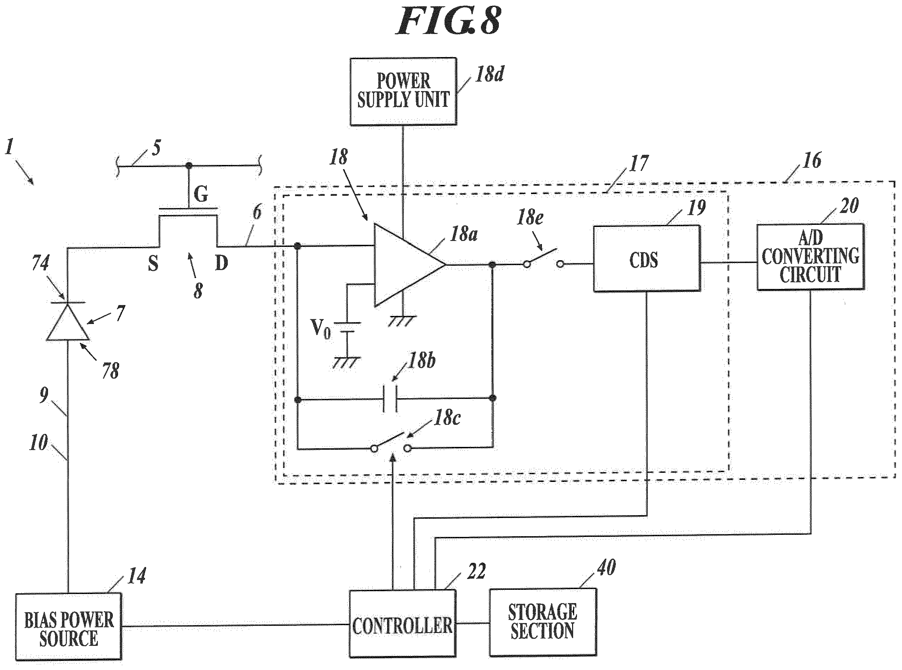

Here, the circuit construction of the radiation image capturing apparatus 1 will be explained. Fig. 7 is a block diagram showing an equivalent circuit of the radiation image capturing apparatus 1 according to the present embodiment, and Fig. 8 is a block diagram showing an equivalent circuit for one pixel which constructs the detecting section P.

As stated previously, in each of the radiation detection elements 7 of the detecting section P of the substrate 4, the bias line 9 is connected to the second electrode 78 thereof, and each of the bias lines 9 is bundled to the wire connection 10 and connected to a bias power source 14. The bias power source 14 is designed to apply a bias voltage to the second electrode 78 of each of the radiation detection elements 7 through the wire connection 10 and each of the bias lines 9. Moreover, the bias power source 14 is connected to controller 22 which will be explained later, and a bias voltage to be applied to each of the radiation detection elements 7 by the bias power source 14 is controlled by the controller 22.

As depicted in Figs. 7 and 8, in this embodiment, as will be noted from the fact that the bias line 9 is connected to the p layer 77 side of the radiation detection element 7 (see Fig. 5) via the second electrode 78, a voltage which is equal to or smaller than a voltage applied to the first electrode 74 side of the radiation detection element 7 (which is so-called reverse bias voltage) is applied as a bias voltage by the bias power source 14 to the second electrode 78 of the radiation detection element 7 through the bias line 9.

The first electrodes 74 of the radiation detection elements 7 are connected to the source electrodes 8s (denoted as S in Figs. 7 and 8) of the TFTs 8, respectively, and the gate electrodes 8g (denoted as G in Figs. 7 and 8) of the TFTs 8 are respectively connected to the lines L1 to Lx of the scanning lines 5 extending from the gate driver 15b of the later-described scanning drive unit 15. In addition, the drain electrodes 8d (denoted as D in Figs. 7 and 8) of the TFTs 8 are connected to the signal lines 6, respectively.

The scanning drive unit 15 is provided with a power source circuit 15a which supplies an on voltage and an off voltage to the gate driver 15b through a wire 15c, and the gate driver 15b which switches a voltage applied to each of the lines L1 to Lx of the scanning lines 5 between an on voltage and an off voltage and switches each of the TFTs 8 between an on state and an off state.

In this embodiment, as describe below, with the scanning drive unit 15, an on voltage is applied sequentially to each of the lines L1 to Lx of the scanning lines 5, or an off voltage is kept being applied to all of the lines L1 to Lx of the scanning line 15.

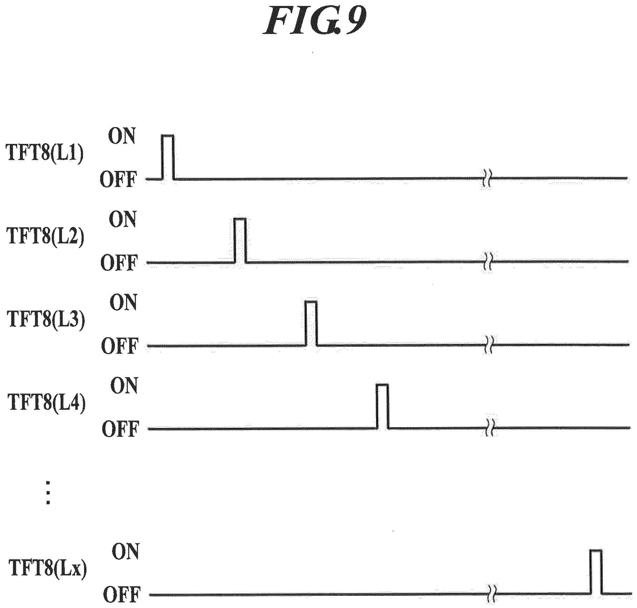

Then, after the radiation image capturing operation, at least during an image data readout process to read out the image data d from each of the radiation detection elements 7, in other words, during a process to read out electric charges which has been generated and accumulated in each of the radiation detection elements 7 due to radioactive irradiation to the radiation image capturing apparatus 1, the scanning drive unit 15 sequentially switches the scanning lines 5 among the lines L1 to Lx in which the voltage applied by the gate driver 15 is switched between the on voltage and off voltage as shown in, for example, Fig. 9, in order to read out the data, and the scanning drive unit 15 causes the image data d to be read out from each of the radiation detection elements 7 connected to each of the lines L1 to Lx of the scanning lines 15.

Further, in the present invention, before radiation image capturing operation, in other words, before radioactive irradiation to the radiation image capturing apparatus 1 is initiated, while each of the TFTs 8 is in the off state as the scanning drive unit 15 applies the off voltage to all the lines L1 to Lx of the scanning lines 5, a later-described reading circuit 17 is periodically driven to execute a leaked data readout process to convert an electric charge leaked from each of the radiation detection elements 7 through each of the TFTs 8 into leaked data Dleak, but this will be detailed later.

As illustrated in Figs. 7 and 8, each of the signal lines 6 is connected to each of the reading circuits 17 formed in each of the reading ICs 16. Note that, in this embodiment, one reading circuit 17 is provided for one signal line 6 in the reading IC 16.

The reading circuit 17 includes an amplifier circuit 18, a correlated double sampling circuit 19, and the like. In addition, in the reading IC 16, an analog multiplexer 21 and an A/D converter 20 are provided. Note that, in Figs. 7 and 8, the correlated double sampling circuit 19 is stated as CDS. Also, in Fig. 8, the analog multiplexer 21 is omitted.

In this embodiment, the amplifier circuit 18 is constructed from a charge amplifier circuit and includes an operational amplifier 18a, as well as a capacitor 18b and an electric charge reset switch 18c which are connected to the operational amplifier 18a in parallel, respectively. Also, a power supply unit 18d for supplying power to the amplifier circuit 18 is connected to the amplifier circuit 18. In addition, a switch 18e, which opens and closes in conjunction with the electric charge reset switch 18c, is provided between the operational amplifier 18a and the correlated double sampling circuit 19.

The signal line 6 is connected to an inverting input terminal on the input side of the operational amplifier 18a of the amplifier circuit 18, and a reference potential $V_0$ is applied to a non-inverting input terminal on the input side of the amplifier circuit 18. Note that the reference potential $V_0$ is set to an appropriate value, and, in this embodiment, for example, 0 [V] is applied.

Further, the electric charge reset switch 18c of the amplifier circuit 18 is connected to the controller 22 so that on/off control is executed by the controller 22, and, when the electric charge reset switch 18c is turned into the on state, the switch 18e simultaneously enters the off state, and when the electric charge reset switch 18c is turned into the off state, the switch 18e enters the on state simultaneously.

In the amplifier circuit 18, during the image data readout process or the leaked data readout process, when the electric charge reset switch 18c is in the off state and the switch 18e is in the on state, once accumulated electric charges are discharged from each of the radiation detection elements 7 to the signal line 6 through each of the TFTs 8 which has been turned into the on state (in the case of the image data readout process), or, once electric charges are leaked into the signal line 6 from each of the radiation detection elements 7 through each of the TFTs 8 which has been turned into the off state (in the case of the leaked data readout process), the electric charges flow in the signal lines 6, enters the capacitor 18b of the amplifier circuit 18, and are accumulated therein.

Also, in the amplifier circuit 18, a voltage value according to an amount of electric charges accumulated in the capacitor 18 is outputted from the output side of the operational amplifier 18a. This way, the amplifier circuit 18 performs charge-voltage conversion by outputting a voltage value in accordance with an electric charge amount outputted from each of the radiation detection elements 7.

It should be noted that the amplifier circuit 18 may be configured so as to output a current in accordance with electric charges outputted from the radiation detection elements 7. Further, when resetting the amplifier circuit 18, once the electric charge reset switch 18c is turned into the on state and the switch 18e enters in the off state simultaneously, the input side and the output side of the amplifier circuit 18 are short-circuited and electric charges accumulated in the capacitor 18b are discharged. Thereafter, the discharged electric charges pass through inside of the operational amplifier 18a from the output terminal side of the operational amplifier 18a, and exits from the non-inverting input terminal to be earthed or are flown out into the power supply unit 18d, thus resetting the amplifier circuit 18.

The correlated double sampling circuit (CDS) 19 is connected to the output side of the amplifier circuit 18. In this embodiment, the correlated double sampling circuit 19 has a sample-and-hold function, and on/off control of this sample-and-hold function of the correlated double sampling circuit 19 is conducted by a pulse signal transmitted from the controller 22.

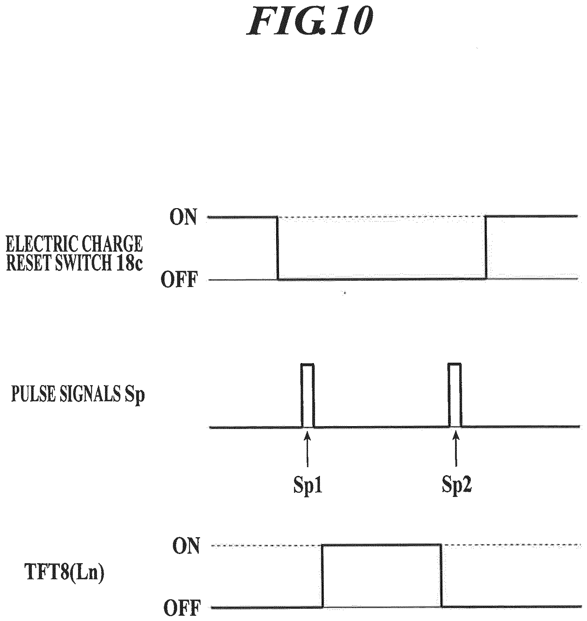

Namely, for example, during the image data readout process, the electric charge reset switch 18c of the amplifier circuit 18 of each of the reading circuits 17 is first controlled to enter the off state, as shown in Fig. 10. At this time, the moment the electric charge reset switch 18c is turned into the off state, a so-called kTC noise is generated, and an electric charge caused by the kTC noise is accumulated in the capacitor 18b of the amplifier circuit 18.

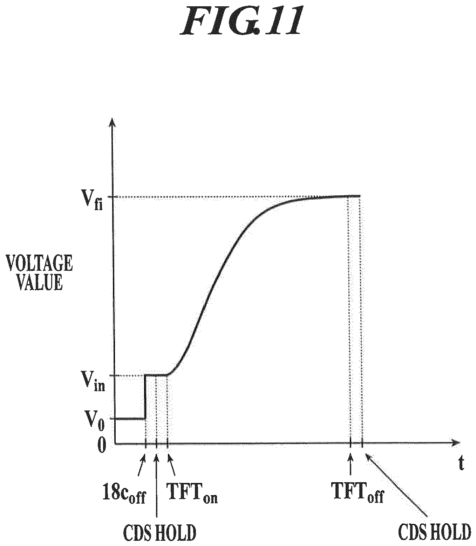

Therefore, as shown in Fig. 11, a voltage value outputted from the amplifier circuit 18 is changed from the aforementioned reference voltage $V_0$ to a voltage value Vin by an amount of electric charges caused by the kTC noise, at the moment when the electric charge reset switch 18c is turned into the off state (expressed as "18c off" in Fig. 11). At this stage, the controller 22 transmits a first pulse signal Sp1 to the correlated double sampling circuit 19 as shown in Fig. 10 to hold the voltage value Vin which is outputted from the amplifier circuit 18 at that point (expressed as "CDS hold" in Fig. 11).

Thereafter, as shown in Fig. 9, when the gate driver 15b of the scanning drive unit 15 applies an on voltage to one of the scanning lines 5 (for example, a line Ln of the scanning lines 5) so that the TFT 8 in which the gate electrode 8g thereof is connected to the scanning line 5 is turned into the on state (see Fig. 10; expressed as "TFT on" in Fig. 11), accumulated electric charges are flown into the capacitor 18b of the amplifier 18 through each of the signal lines 6 from each of the radiation detection elements 7 to which these TFTs 8 are connected, and, as shown in Fig. 11, a voltage value, which is outputted from the amplifier circuit 18 in accordance with the amount of the electric charges accumulated in the capacitor 18, increases.

Then, as shown in Fig. 10, after a lapse of a given period of time, the controller 22 switches the on voltage applied by the gate driver 15b to the scanning line 5 into the off voltage so that the TFT 8 in which the gate electrode 8g thereof is connected to the scanning line 5 enters the off state (expressed as "TFT off" in Fig. 11), and, at this stage, the controller 22 transmits a second pulse signal Sp2 to each of the correlated double sampling circuits 19 to hold a voltage value Vfi which is outputted from the amplifier circuit 18 at that point (expressed as "CDS hold" (on the right side) in Fig. 11).

Once the voltage value Vfi is held due to the second pulse signal Ps2, the correlated double sampling circuit 19 calculates a difference of the voltage values Vfi - Vin, and outputs the calculated difference Vfi - Vin to the downstream side as image data d in an analog value.

The image data d of each of the radiation detection elements 7 outputted from the correlated double sampling circuit 19 is transmitted to the analog multiplexer 21, and is transmitted sequentially from the analog multiplexer 21 to the A/D converter 20. Then, the image data d in an analog value is sequentially converted into image data d in a digital value in the A/D converter 20, outputted to and stored in storage section 40 sequentially.

Also, the controller 22 executes the above-mentioned image data readout process for reading out the image data d from each of the radiation detection elements 7 every time the scanning line 5 is switched among the respective lines L1 to Lx to which the on voltage is applied by the gate driver 5b of the scanning drive unit 15 as shown in Fig. 9.

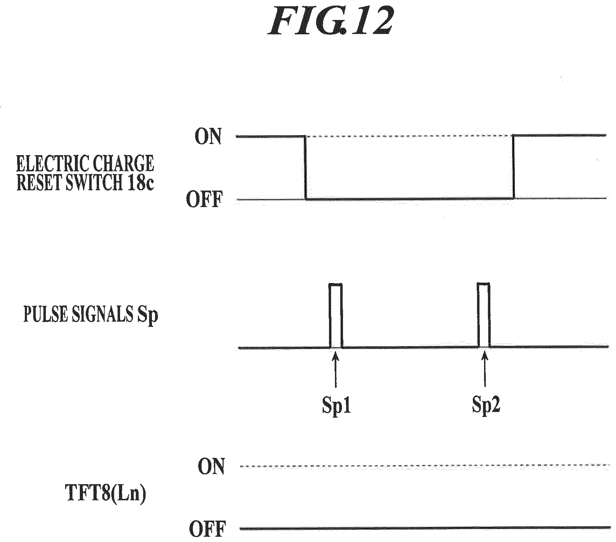

It should be noted that Fig. 10, later-explained Fig. 12 and so on only show on/off of the electric charge reset switch 18c, and does not show on/off of the switch 18e (see Fig. 8), but the switch 18e performs the off/on operation in conjunction with on/off of the electric charge reset switch 18c as explained earlier. Also, only the operations of the electric charge reset switch 18c and the like may be described in the explanation below, but the same will apply to such cases.

Meanwhile, as explained later, in the present invention, the leaked data readout process is executed when each of the TFTs 8 is in the off state for converting an electric charge leaked from each of the radiation detection elements 7 via each of the TFTs 8 into leaked data Dleak by periodically driving the reading circuits 7.

Since the leaked data readout process is executed while each of the TFTs 8 is in the off state, the off voltage is applied to all the lines L1 to Lx of the scanning lines 5 by the scanning drive unit 15 as shown in Fig. 12. This means that, unlike the case of the image data readout process shown in Fig. 10, the on/off operation of each of the TFTs 8 is not conducted in the leaked data readout process, and each of the TFTs 8 is always in the off state at least in the period of the leaked data readout process.

Then, as shown in Fig. 12, the on/off control of the electric charge reset switch 18c, transmission of the pulse signals Sp1 and Sp2 to the correlated double sampling circuits 19, and the like conducted by the controller 22 are executed similarly to the case of the image data readout process, and, as shown in Fig. 11, a voltage value outputted from the amplifier circuit 18 increases by an amount of electric charge which have been leaked from each of the radiation detection elements 7 through each of the TFTs 8 and flown and accumulated in the capacitor 18.

Note that, in the case of the leaked data readout process, although the voltage value outputted from the amplifier circuit 18 is increased, the level of the increase thereof in the case of the leaked data readout process is usually lower than the level of increase of the same in the case of the image data readout process.

Similarly to the case of the image data readout process, once the voltage value Vfi due to the second pulse signal Sp2 is held, each of the correlated double sampling circuits 19 calculates a difference of the voltage values Vfi - Vin, and outputs the calculated difference Vfi - Vin to the downstream side as leaked data Dleak in an analog value in the case of the leaked data readout process. Then, the leaked data Dleak outputted from the correlated double sampling circuit 19 is sequentially transmitted to the A/D converter 20 via the analog multiplexer 21 and converted into leaked data Dleak in a digital value.

The controller 22 is configured by a computer in which a non-illustrated CPU (central processing unit), a ROM (read only memory), a RAM (random access memory), an input/output interface and the like are connected to a bus, a FPGA (field programmable gate array) and the like. The controller 22 may also be constructed by a designated control circuit. Also, the controller 22 is designed to control operations and the like of respective members of the radiation image capturing apparatus 1. In addition, as illustrated in Fig. 7 and so on, the storage section 40 constructed by DRAM (dynamic RAM) or the like is connected to the controller 22.

Further, in this embodiment, the aforementioned antenna device 39 is connected to the controller 22, and, in addition, the battery 41 for supplying power to each of the members including the detecting section P, the scanning drive unit 15, the reading circuit 17, the storage section 40, and the bias power source 14, is connected to the controller 22. Moreover, a connecting terminal 42 is attached to the battery 41 for charging the battery 41 by supplying power to the battery 41 from non-illustrated charging equipment.

As stated previously, the controller 22 is designed to control operations of respective functional parts of the radiation image capturing apparatus 1, such as control of the bias power source 14 in order to set or change a bias voltage which is applied by the bias power source 14 to each of the radiation detection elements 7.

Below is explanation regarding the leaked data readout process, detection of initiation of radioactive irradiation, and so on by the controller 22, as well as operations of the radiation image capturing apparatus 1 according to the present invention.

[Principles of leaked data readout process and detection of initiation of radioactive irradiation]

Explained next is the leaked data readout process according to the present invention and detection of initiation of radioactive irradiation to the radiation image capturing apparatus 1 based on leaked data Dleak that is read out in the leaked data readout process.

As stated earlier, in this embodiment, the leaked data readout process begins before radioactive irradiation to the radiation image capturing apparatus 1 is initiated prior to radiation image capturing operation. This leaked data readout process is started, for example, at a point when an operator such as a radiological technologist presses the power switch 36 (see Fig. 1) of the radiation image capturing apparatus 1, when the radiation image capturing apparatus 1 is shifted to an active state, or when a signal from an external device indicating the start of the leaked data readout process is received.

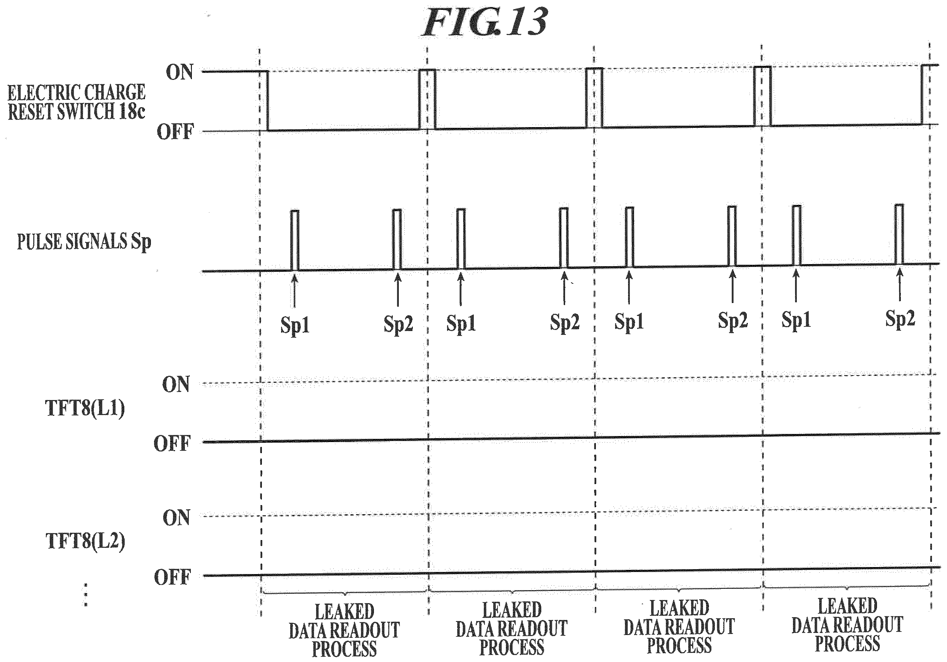

Then, in this embodiment, the controller 22 repeatedly executes the leaked data readout process shown in Fig. 12 on a periodic basis. Namely, as shown in Fig. 13, the controller 22 periodically repeats the on/off operation of the electric charge reset switch 18c of the amplifier circuit 18 and transmission of the pulse signals Sp1 and Sp2 to the correlated double sampling circuit 19, in a state where each of the TFTs 8 is turned into the off state by applying the off voltage from the scanning drive unit 15 to all the lines L1 to Lx of the scanning lines 5.

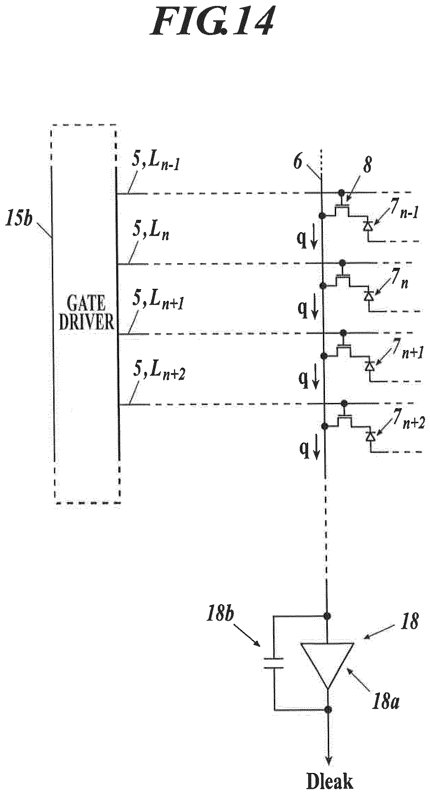

During the leaked data readout process, since the off voltage is applied to all of the lines L1 to Lx of the scanning lines 5 by the scanning drive unit 15, and each of the TFTs 8 is thus in the off state, an electric charge generated in each of the radiation detection elements 7 is accumulated in each of the radiation detection elements 7, but, as shown in Fig. 14, due to the properties of the TFTs, each of the electric charges q slowly leaks into the signal line 6 from each of the radiation detection elements 7 through each of the TFTs 8 even if the TFTs 8 are in the off state.

Then, as explained above, each of the electric charges q leaked from each of the radiation detection elements 7 flows into the capacitor 18b of the amplifier circuit 18 through the signal line 6 and is accumulated therein. Further, because a voltage value is outputted from the output side of the operational amplifier 18a in accordance with the amount of electric charges accumulated in the capacitor 18b, the voltage value outputted from the amplifier circuit 18 increases as shown in Fig. 11 after the electric charge reset switch 18c is turned into the off state, and the correlated double sampling circuit 19 outputs a difference Vfi - Vin of the voltage values Vin and Vfi as leaked data Dleak, the difference Vfi - Vin having been held according to the pulse signals Sp1 and Sp2.

As explained above, in the leaked data readout process, the sum total value of each of the electric charges q which has been leaked through each of the TFTs 8 from each of the radiation detection elements 7 connected to one signal line 6 is accumulated in the capacitor 18b of the amplifier circuit 18, and data that is equivalent of the sum total value of each of the leaked electric charges q is converted for each of the reading circuits 18 and outputted as leaked data Dleak.

Meanwhile, it is known that, in the TFT 8 which serves as the switch unit, an amount of a leakage current flowing in the TFT 8 increases when radiation is emitted or when an electromagnetic wave converted from radiation by the scintillator 3 (see Fig. 2 and so on) is emitted like this embodiment. This is considered to be because a new electron-hole pair is generated in the semiconductor layer 82 (see Fig. 5) of the TFT 8 as radiation is emitted to the TFT 8.

Then, due to radioactive irradiation (or irradiation of an electromagnetic wave converted from radiation; the same applies hereinafter), the amount of a leakage current flowing in each of the TFTs 8 increases, and, once the leakage of an electric charge from each of the radiation detection elements 7 through each of the TFTs 8 increases, the sum total value of the each of the electric charges q leaked from each of the radiation detection elements 7 connected to one signal line 6 increases, and the corresponding leaked data Dleak also increases.

Therefore, according to a time-series plot of the leaked data Dleak which is read out in the leaked data readout process which is repeated periodically as stated above, the value of the leaked data Dleak increases at time t1 when radioactive irradiation to the radiation image capturing apparatus 1 is initiated as shown in Fig. 2. 15.

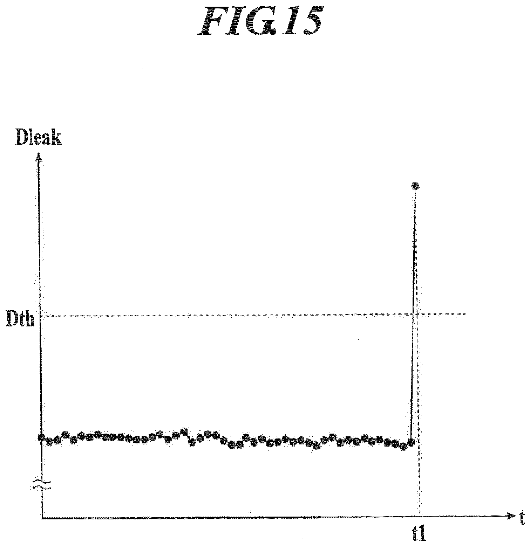

Thus, a construction may be such that the controller 22 monitors the leaked data Dleak read out in the periodically-repeated leaked data readout process shown in Fig. 13, and initiation of radioactive irradiation can be detected at a point when the leaked data Dleak which has been read out exceeds a set threshold value Dth (see Fig. 15).

As described above, the radiation image capturing apparatus 1 according to the present invention is designed so that the controller 22 causes the reading circuit 17 to execute the readout operation periodically in the state where each of the TFTs 8 is in the off state as the off voltage is applied to all of the lines L1 to Lx of the scanning lines 5 by the scanning drive unit 15 prior to radiation image capturing operation, and the controller 22 has the leaked data readout process executed for converting the electric charge q leaked from each of the radiation detection elements 7 through each of the TFTs 8 into leaked data Dleak, thus detecting that radioactive irradiation is initiated at a point when the leaked data Dleak which has been read out exceeds a threshold value Dth.

The above is the principles of the leaked data readout process and the detection of initiation of radioactive irradiation according to the present invention. With this construction, the radiation image capturing apparatus 1 is enabled to at least detect initiation of radioactive irradiation accurately on its own by using the reading circuits 17 which already exists in the radiation image capturing apparatus 1, without providing new means such as a current detection unit in the radiation image capturing apparatus 1 like the inventions described in the foregoing Patent document 4 and Patent document 5.

As shown in Fig. 14 described earlier, leaked data Dleak per reading circuit 17 is outputted from each of the reading circuits 17 as leaked data Dleak. Moreover, one reading circuit 17 is provided for every several thousands or several tens of thousands of signal lines 6 provided in the detecting section P. Therefore, in this embodiment, in a single round of the leaked data readout process, several thousands to several tens of thousands of pieces of leaked data Dleak are outputted from each of the reading circuits 17.

In this embodiment, the control means 22 is designed to extract the maximum value from among the respective leaked data Dleak read out in every leaked data readout process and determines whether the maximum value of the leaked data Dleak exceeds the threshold value Dth. With this construction, in a case where, for example, radiation is emitted only to a narrow area of the detecting section P of the radiation image capturing apparatus 1 (in other words, in a case where a radiation field is narrowed), the leaked data Dleak does not increase in an area where radiation was not emitted, but the controller 22 is able to precisely extract an increase of the leaked data Dleak in an area where radiation was emitted and detect initiation of radioactive irradiation accurately.

Note that, although depending on the performance of each of the reading circuits 17, when there is a big noise in the reading circuit 17, there may be a case that the leaked data Dleak with the noise being superimposed thereon exceeds the threshold value Dth, which may cause a false detection that radioactive irradiation has been initiated. In such a case, for example, the controller 22 may be constructed so that a sum total value (or an average value) of leaked data Dleak is calculated for each of the reading ICs 16 in which a given number of the reading circuits 17 is provided, and the controller 22 extracts the maximum value from among the sum total values (or the average values) and compares the maximum value to the threshold value Dth.

Typically, a large number of reading circuits 17, such as 128 or 256 of the same, are formed in the reading IC 16. Hence, with the aforementioned construction, a noise generated in each of the reading circuits 17 is balanced out with one another when calculating the sum total value (or the average value) of the leaked data Dleak, thus making it possible to reduce an impact of noises generated in the respective reading circuits 17 on the leaked data Dleak.

Also, instead of constructing the controller 22 to extract a maximum value of individual leaked data Dleak, or to calculate sum total values (or average values) of leaked data Dleak for each of the reading ICs 16, extract a maximum value from among them, and compare the maximum value with a threshold value Dth as explained above, the controller 22 may be constructed so as to calculate a sum total value (or an average value) of all leaked data Dleak read out in the respective reading circuits 17 in a single round of leaked data readout process, and compares the sum total value (or the average value) and the threshold value Dth. With such a construction, the process for extracting the maximum values is no longer necessary.

The explanation below is about the case where a maximum value is extracted from among leaked data Dleak which has been read out for each of the reading circuits 17, but the same explanation applies to the case where a maximum value is extracted from among sum total values (or average values) of leaked data Dleak calculated for each of the reading ICs 16, or the case where a sum total value (or an average value) of all leaked data Dleak read out in the respective reading circuits 17 is calculated.

Also, when each of the TFTs 8 is turned into the off state as the scanning drive unit 15 applies the off voltage to all of the lines L1 to Lx of the scanning lines 5 as illustrated in Fig. 13 and so on, a dark electric charge generated in each of the radiation detection elements 7 is accumulated in each of the radiation detection elements 7, and how to remove the dark electric charges will be explained later.

[How to decide threshold value]

Explained next is how to decide the aforementioned threshold value Dth which is a criterion for determining whether radioactive irradiation to the radiation image capturing apparatus 1 is initiated.

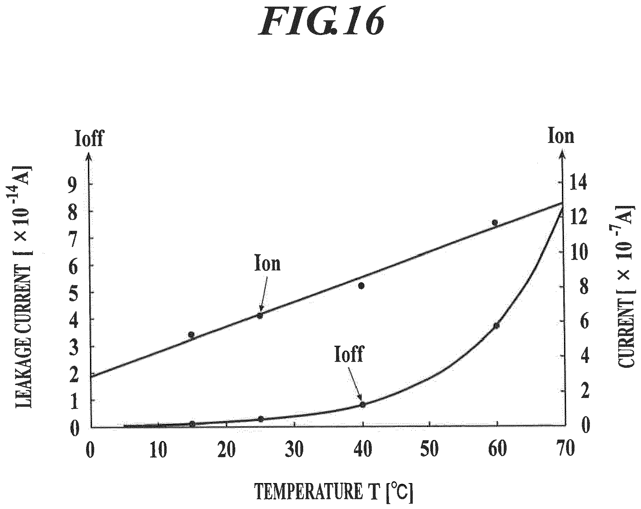

According to the studies undertaken by the present inventor, it is known that the electric charge q leaked from the radiation detection element 7 through the TFT 8 that serves as the switch unit changes at least depending on the temperature of the TFT 8. Fig. 16 is a graph showing how a leakage current Ioff flowing in the TFT 8 changes along with temperature change of the TFT 8 while the TFT 8 is in the off state (in the state where the off voltage is applied to the gate electrode 8g of the TFT 8). It should be noted that Fig. 16 also shows the temperature dependency of a current Ion flowing in the TFT 8 while the TFT 8 is in the on state (in the state where the on voltage is applied to the gate electrode 8g of the TFT 8).

Note that this experiment was conducted by measuring actual values of the leakage current Ioff while changing the temperature of the TFT 8 in a state where the reference voltage $V_0$ of 0V is applied by the amplifier circuit 18 to the drain electrode 8d of the TFT 8 (see Figs. 7 and 8) through the signal line 6, the off voltage of -10V is applied by the scanning drive unit 15 to the gate electrode 8g of the TFT 8 through the scanning line 5, and the bias voltage of -5V (reverse bias voltage) is applied to the radiation detection element 7 through the bias line 9, and, in addition, an electric charge equivalent to the bias voltage is accumulated in the radiation detection element 7, in other words, in a state where an electric charge equivalent to a saturation charge amount of a photodiode which works as the radiation detection element 7 is accumulated in this embodiment.

A controlled experiment was also conducted under similar conditions in order to obtain the temperature dependency of the current Ion flowing in the TFT 8 while the TFT 8 is in the on state (in a state where the on voltage is applied to the gate electrode 8g of the TFT 8), and the actual values of the current Ion were measured after the voltage applied to the gate electrode g of the TFT 8 by the scanning drive unit 15 is switched to the on voltage of +15V.

Although it is yet unclear why the leakage current Ioff flowing in the TFT 8 increases exponentially along the temperature rise of the TFT 8 when the TFT 8 is in the off state as shown in Fig. 16, the reason is at least thought to be an increases of carrier concentration in the semiconductor layer 82 of the TFT 8 (see Fig. 5) as atoms which construct the TFT 8 vibrate faster due to heat as the temperature of the TFT 8 rises.

Even if the leakage current Ioff flowing in the TFT 8 in the off state, in other words, the electric charge quantity of the electric charge q leaked from the radiation detection element 7 through the TFT 8 varies depending on the temperature of the TFT 8 as described above, the radiation image capturing apparatus 1 which is formed integrally with the foregoing supporting stand can be constructed so that power is always supplied thereto by a power source outside of the device, and, when the bias voltage 14, the scanning drive unit 15, and the reading IC 16 and the like including the reading circuits 17, are operated for a long period of time, the TFT 8 will be stably kept at a constant temperature.

Although the electric charge q leaked from the radiation detection element 7 through the TFT 8 having a constant temperature may have some fluctuation, the values thereof are fairly constant. Therefore, the foregoing leaked data Dleak, which is equivalent to the sum total value of each of the electric charges q leaked through the TFT 8 from each of the radiation detection elements 7 connected to a single signal line 6, may also have some fluctuation, but is almost constant. Hence, a maximum value extracted from among such leaked data Dleak may have a certain level of fluctuation but is almost constant as well.

However, once radiation is emitted to the radiation image capturing apparatus 1, each of the electric charges q leaked through each of the TFTs 8 is increased, which causes a significant increase of the maximum value extracted from among each of the leaked data Dleak that is read out in each of the reading circuits 17, as shown in Fig. 15.

Therefore, in such a case, a maximum value of the leaked data Dleak is measured in advance in a condition where the temperature of the TFT 8 is stable as power is always supplied to the radiation image capturing apparatus 1 as explained above, and, in addition, a maximum value of the leaked data Dleak is measured in advance in the case where radiation is emitted to the radiation image capturing apparatus 1, and then the threshold value Dth may be preset to a given value therebetween.

Meanwhile, in the case of the foregoing radiation image capturing apparatus 1 with a built-in battery, in order to reduce power consumption of the battery 41 (see Fig. 7) as much as possible, it is often the case that the power switch 36 (see Fig. 1) of the radiation image capturing apparatus 1 is pressed immediately before radiation image capturing operation is conducted, or the bias voltage 14, the scanning drive unit 15, the reading IC 16 and the like are launched as the radiation image capturing apparatus 1 is changed to an active state.

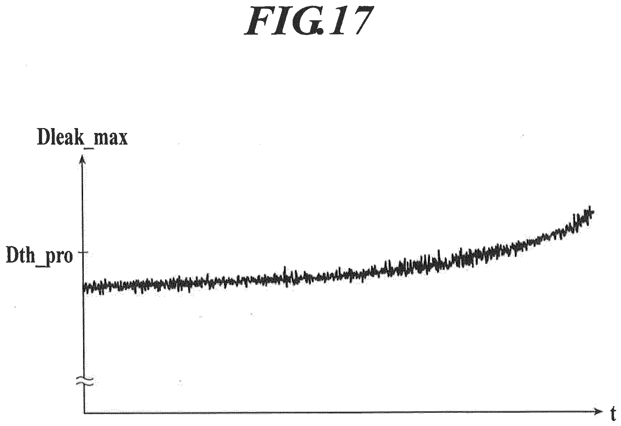

In such a case, the temperature of TFT 8 increases along a temperature rise of the substrate 4 (see Fig. 3 and so on) as the bias voltage 14, the scanning drive unit 15, the reading IC 16 and so on are launched. Therefore, when, for example, the leaked data readout process shown in Fig. 13 is periodically repeated right after the power switch 36 of the radiation image capturing apparatus 1 is pressed, a maximum value Dleaek_max among respective pieces of the leaked data Dleak read out in the respective reading circuits 17 increases gradually along the temperature rise of the TFT 8 as shown in Fig. 17, for example.

Thus, for example, when the construction is such that the threshold value Dth is set previously to a certain value Dth_pro, the value of each of the leaked data Dleak read out in each of the reading circuits 17 increases because of the temperature rise of the TFT 8 even though no radiation is emitted to the radiation image capturing apparatus 1, and the controller 22 is likely to misjudge that radioactive irradiation is initiated at a point when the maximum value Dleak_max exceeds the certain threshold value Dth_pro.

Thus, particularly when the radiation image capturing apparatus 1 is a radiation image capturing apparatus having a built-in battery as stated above, such construction is possible that the controller 22 sets the threshold value Dth while updating the same based on the history of each of the leaked data Dleak (the maximum value Dleak_max of each of the leaked data Dleak in the aforementioned case) read out in the leaked data readout process which is conducted repeatedly on a periodical basis.

To be specific, for example, such construction may be adapted that, every time the leaked data readout process is executed, an average value is calculated of the maximum values Dleak_max of the leaked data Dleak extracted in a given number of rounds, for example, 10 or 100 rounds of leaked data readout processes conducted in the past including the leaked data readout process immediately before the present leaked data readout process, in other words, an average value Dleak_ave of moving average is calculated, and the threshold value Dth is determined by adding a previously-set certain value to the average value Dleak_ave.

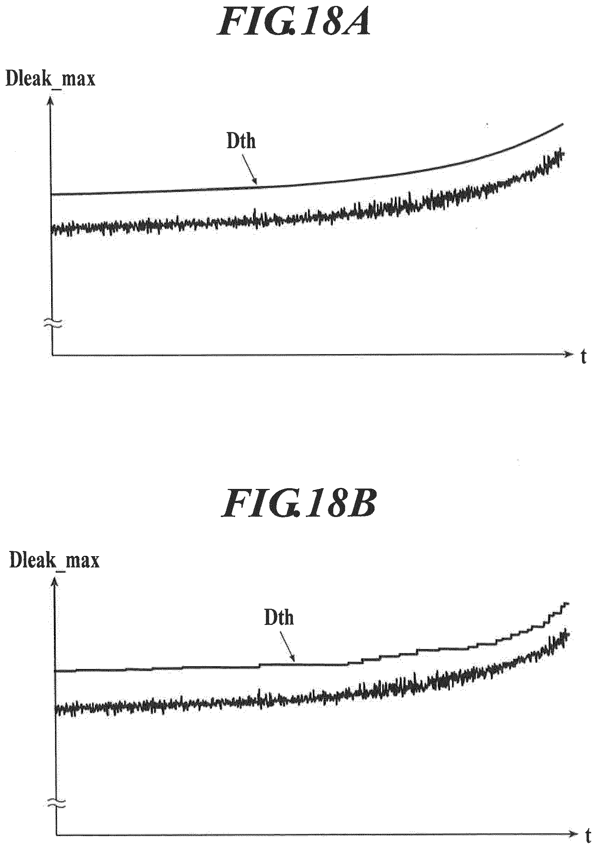

With this construction, the threshold value Dth can be set while updating the same in every leaked data readout process as shown in Fig. 18A. In addition, even when a value of each of the leaked data Dleak read out from each of the reading circuits 17 increases due to a temperature rise of the TFT 8, the threshold value Dth also increases accordingly, thus appropriately preventing false detection of initiation of radioactive irradiation.

Further, when there is a significant change of the maximum value Dleak_max of the leaked data Dleak extracted from among the leaked data Dleak read out in the current leaked data readout process as shown in Fig. 15, and the maximum value Dleak_max exceeds the threshold value Dth, initiation of radioactive irradiation can be accurately detected at a point when the current leaked data readout process is executed.

Alternatively, such construction is possible that the controller 22 is given a peak hold function or is provided with peak hold means, and, if a maximum value Dleak_max of leaked data Dleak extracted in the current round is larger than a past maximum value Dleak_max which is already held in the controller 22, the maximum value Dleak_max is updated to the maximum value Dleak_max extracted in the current round every time the leaked data readout process is executed. Then, the threshold value Dth is determined by adding a previously-set given value to the maximum value Dleak_max held in the controller 22.

With this construction, as shown in Fig 18B, the threshold value Dth can be set while updating the same in every leaked data readout process. In addition, even when a value of each of the leaked data Dleak read out in each of the reading circuits 17 increases due to a temperature rise of the TFT 8, the past maximum value Dleak_max which is held in the controller 22 is updated to the larger value, and the threshold value Dth also increases accordingly. Thus, false detection of initiation of radioactive irradiation can be prevented appropriately.

Also in this case, when there is a significant change of the maximum value Dleak_max of the leaked data Dleak extracted from among the leaked data Dleak read out in the current round of the leaked data readout process, and the maximum value Dleak_max exceeds the threshold value Dth, initiation of radioactive irradiation can be accurately detected at a point when the current leaked data readout process is executed.

[Removal of dark electric charges and so on]

As stated earlier, the leaked data readout process prior to radiation image capturing operation and detection of initiation of radioactive irradiation according to the present invention are executed while each of the TFTs 8 is in the off state by applying the off voltage from the scanning drive unit 15 to all of the lines L1 to Lx of the scanning lines 5 as shown in Fig. 13. Having said that, it is well known that, if such situation continues, so-called dark electric charges generated due to thermal excitation or the like caused by heat (temperature) of the radiation detection element 7 itself are accumulated within the radiation detection element 7, and the amount of accumulated dark electric charges increases.

In addition, each of the radiation detection elements 7 can accumulate electric charges only up to the saturation charge amount Q which is calculated from a relation Q = CV (in the case of this embodiment, V represents a difference between the reference voltage $V_0$ and the bias voltage) where a parasitic capacity of each of the radiation detection elements 7 is expressed as C. Hence, if there is a large amount of excessive electric charges accumulated and remaining in each of the radiation detection elements 7, a problem arises that a dynamic range is reduced, the dynamic range being for accumulating electric charges that are newly generated in each of the radiation detection elements 7 by radioactive irradiation, in other words, useful electric charges which carry information of a subject.

Therefore, it is necessary to remove excessive electric charges such as dark electric charges remaining in each of the radiation detection elements 7 during the leaked data readout process which is repeatedly executed on a periodic basis prior to radiation image capturing operation.

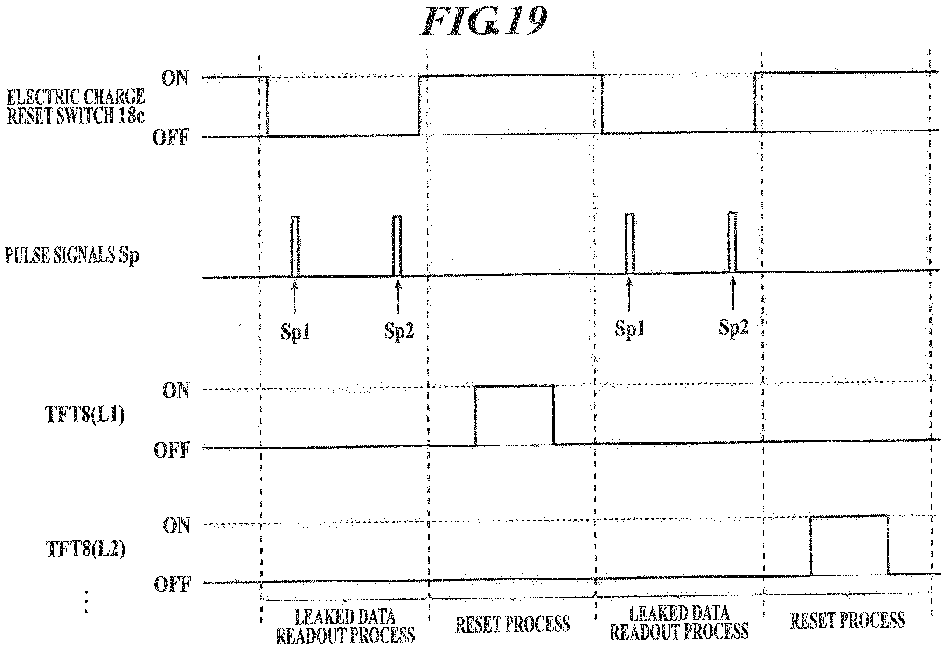

Thus, in this embodiment, although the controller 22 executes the process for reading out the leaked data Dleak during the leaked data readout process in the state where the off voltage is applied by the scanning drive unit 15 to all the lines L1 to Lx of the scanning lines 5 as explained above, a reset process for discharging and removing excessive electric charges from each of the radiation detection elements 7 is executed at the time of the periodically-executed leaked data readout process, by applying the on voltage to each of the lines L1 to Lx of the scanning lines 5 from the scanning drive unit 15 between the leaked data readout process and the next leaked data readout process, as shown in Fig. 19.

In this embodiment, for the reset process for each of the radiation detection elements 7, the on voltage is sequentially applied to each of the lines L1 to Lx of the scanning lines 5 by the scanning drive unit 15, when the electric charge reset switch 18c of the amplifier circuit 18 of the reading circuit 17 is turned into the on state, and, although not shown, the switch 18e (see Fig. 8) is turned into the off state accordingly.

So, each of the TFTs 8 connected to the lines L1 to Lx of the scanning lines 5 to which the on voltage is applied enters the on state, and excessive electric charges are discharged into the signal line 6 through each of the TFTs 8 from each of the radiation detection elements 7. Then, the electric charges discharged into the signal line 6 passes through the electric charge reset switch 18c of the amplifier circuit 18, and passes through inside of the operational amplifier 18a from the output terminal side of the operational amplifier 18a, and goes out from the non-inverting input terminal and earthed or flows out into the power supply unit 18d, thus being removed from each of the radiation detection elements 7 or the reading circuits 17.

With such a construction, since excessive electric charges such as dark electric charges are removed from each of the radiation detection elements 7, the dynamic range, which accumulates electric charges newly generated in each of the radiation detection elements 7, can be unfailingly prevented from being reduced due to dark electric charges and the like being continuously accumulated in each of the radiation detection elements 7.

Because of this, as shown in Fig. 15, it becomes possible to reliably detect initiation of radioactive irradiation by monitoring the leaked data Dleak (to be precise, it is a maximum value Dleak_max of the leaked data Dleak or a maximum value or the like of sum total values (or average values) of leaked data Dleak for each of the reading ICs 16, but will be simply referred to as the leaked data Dleak hereinbelow), and, at the same time, it becomes possible that electric charges, which have been generated in each of the radiation detection elements 7 due to radioactive irradiation, are accumulated in a large dynamic range with a plenty of space, thus enabling to obtain image data d accurately according to a dose of radiation.

It should be noted that, explained in Fig. 19 was the case where the scanning drive unit 15 applies the on voltage sequentially to each of the lines L1 to Lx of the scanning lines 5 as the reset process for each of the radiation detection elements 7 (in other words, the case where the on voltage is applied to one of the scanning lines 5 at a time, and the line L of the scanning lines 5 to which the on voltage is applied is sequentially switched), but such construction is also possible that the reset process for each of the radiation detection elements 7 is executed at the time of the periodically-conducted leaked data readout process, by applying the on voltage to all of the lines L1 to Lx of the scanning lines 5 at once from the scanning drive unit 15 between the leaked data readout process and the next leaked data readout process.

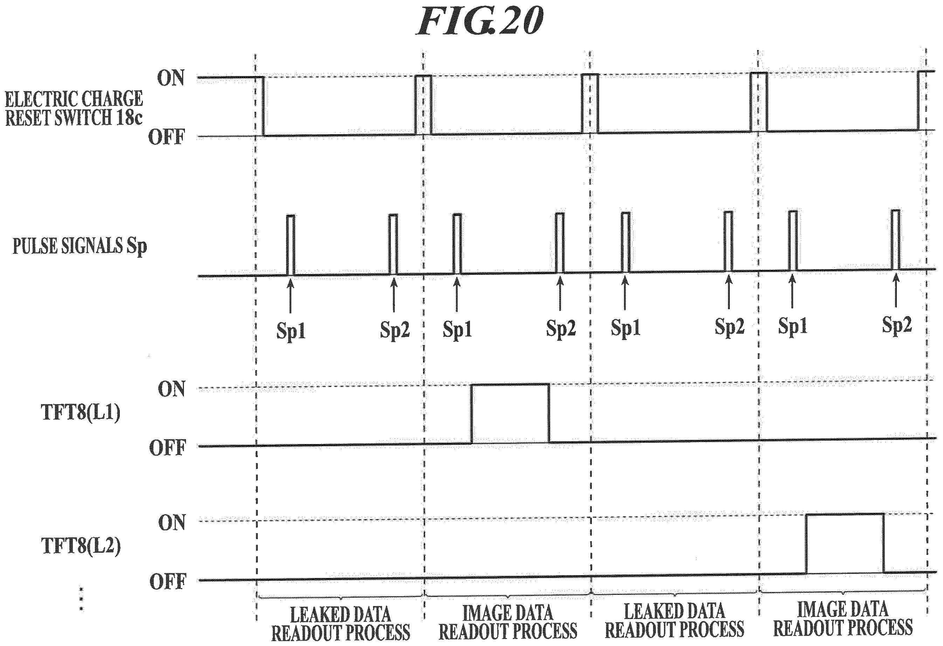

Alternatively, instead of executing the reset process for each of the radiation detection elements 7 at the time of the periodically-conducted leaked data readout process between the leaked data readout process and the next leaked data readout process, such construction may be employed that the image data readout process is executed, which is for reading out image data by converging electric charges discharged from each of the radiation detection elements 7 into image data d, by sequentially applying the on voltage to each of the lines L1 to Lx of the scanning lines 5 from the scanning drive unit 15, as shown in Fig. 20.

With this construction, excessive electric charges can also be removed from each of the radiation detection elements 7. In the case of this construction, the image data readout process is executed in the method explained using Fig. 10. In addition, although the image data d which has been read out is not used by the controller 22 as a criterion for determining whether radioactive irradiation to the radiation image capturing apparatus 1 is initiated, the image data d which has been read out can be used effectively and is used in appropriate ways.

[Improvement of S/N ratio of leaked data]

Here, improvement of the S/N ratio of the leaked data Dleak will be explained. The leaked data Dleak, which is attributable to the electric charge q (see Fig. 14) leaked from each of the radiation detection elements 7 through each of the TFTs 8 in the state where the off voltage is applied to all of the lines L1 to Lx of the scanning lines 5 from the scanning drive unit 15, is usually a small value as evident from the fact that the leakage current Ioff flowing in the TFT 8 in the off state is considerably smaller than the current Ion flowing in the TFT 8 in the on state as shown in Fig. 16.

Therefore, it may be true that the leaked data Dleak is susceptible to noises generated in the power source circuit 15a of the scanning drive unit 15 (see Fig. 7) and transferred through each of the lines L1 to Lx of the scanning lines 5 or noises generated in the reading circuits 17. Namely, in some cases, the S/N ratio of the leaked data Dleak could be bad.

[Method 1]

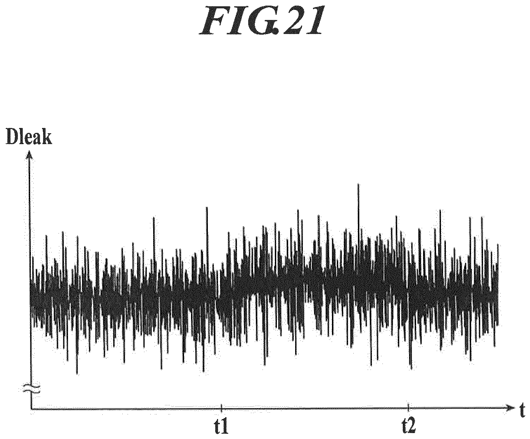

The data shown in Fig. 21 is an example of the leaked data Dleak which is read out in each of the leaked data readout processes in a case where very low radiation is emitted to the radiation image capturing apparatus 1 with a dose per unit time or a dose rate of approximately 0.5 μR/ms, and is an example where radioactive irradiation is initiated at time t1 and the irradiation is ended at time t2.

Clinically, it is generally said that 1 to 2 μR/ms is the lowest level of dose rate, and the conditions stated above correspond to a case where radiation is emitted at an even lower dose ratio, but, when radiation at such an extremely low dose ratio is emitted to the radiation image capturing apparatus 1, an increase of the leaked data Dleak due to radioactive irradiation is buried in noises as shown in Fig. 21, which at least makes it impossible to detect initiation of radioactive irradiation.

Here, a noise that is superimposed on the leaked data Dleak will be examined. At least a noise derived from the power source circuit 15a of the scanning drive unit 15 is a noise which is generated in one of the power source circuits 15a and transmitted instantly to each of the TFTs 8 through each of the lines L1 to Lx of the scanning lines 5 through the gate driver 15b, as illustrated in Fig. 7. Therefore, a noise generated in the power source circuit 15a is transmitted to all of the TFTs 8 simultaneously and superimposed on the leaked data Dleak which is read out.

Also, as depicted in Fig. 7, the bias power source 4 is connected to each of the radiation detection elements 7 through the wire connection 10 and each of the bias lines 9, and a bias voltage with a noise mixed therein generated in the bias power source 14 is applied to each of the radiation detection elements 7. Since each of the radiation detection elements 7 has a sort of capacitor-like configuration in which the i layer 76 and so on are located between the first electrode 74 and the second electrode 78 (see Fig. 5), each of the radiation detection elements 7 has a parasitic capacity. Also, when this parasitic capacity is expressed as C and the bias voltage is expressed as Vbias, an electric charge Q which is basically expressed as $Q = C \cdot (V_0 - Vbias)$ is accumulated in each of the radiation detection elements 7, and this electric charge Q is fluctuated due to the aforementioned noise in the bias voltage Vbias.

As explained above, since a noise is generated in the electric charge accumulated in each of the radiation detection elements 7 due to the noise in the bias voltage Vbias, the noise caused by the noise in the bias voltage Vbias is also superimposed on the leakage current Ioff which is generated by a tiny fraction of this electric charge flowing in the TFT 8. Thus, this noise due to the noise in the bias voltage Vbias is also superimposed on the leaked data Dleak to be read out.

Moreover, from the side of each of the reading ICs 16, a noise caused by a noise generated in each of the reading ICs 16 is superimposed on each of the TFTs 8 and the like through each of the signal lines 6. This way, the same noise caused by various kinds of noises generated in each of the functional sections in the device is superimposed on the leaked data Dleak which is read out at the same timing.

Therefore, in one round of the leaked data readout process, various types of noises such as a noise derived from the power source circuit 15a of the scanning drive unit 15 and a noise derived from the bias power source 14 are simultaneously superimposed on each piece of the leaked data Dleak which has been read out in each of the reading circuits 17. Thus, the S/N ratio of the leaked data Dleak can be improved with the construction stated below by utilizing the fact that the same noise derived from the power source circuit 15a and so on is superimposed on each of the leaked data Dleak which is read out in one round of the leaked data readout process.

[Method 1-1]

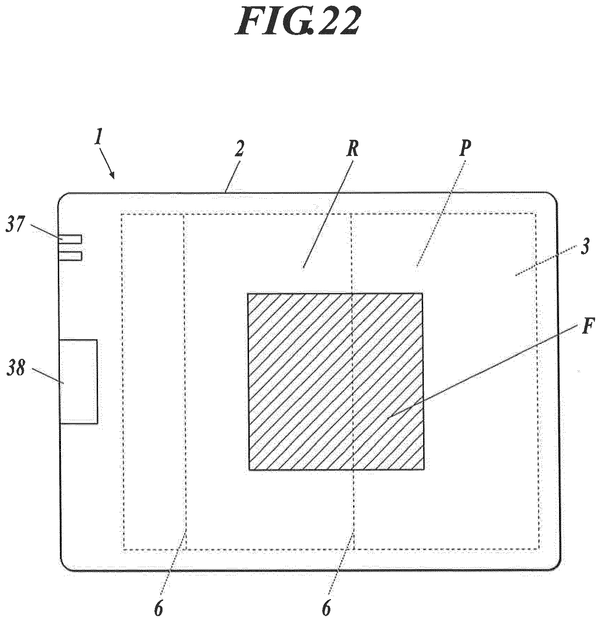

When emitting radiation to the radiation image capturing apparatus 1, radiation may be emitted not to the entire regions of the scintillator 3 and the detecting section P of the radiation image capturing apparatus 1 but to an irradiation field F which is narrowed into parts of the scintillator 3 and the detecting section P as shown in Fig. 22 when viewed from the side of the radiation entrance face R (see Figs. 1 and 2) of the radiation image capturing apparatus 1.

In particular, when a low dose rate of radiation is emitted to the radiation image capturing apparatus 1 like Schüller's projection of auditory organs, it is often the case that radiation is emitted to the narrowed irradiation field F. Note that, in Fig. 22, the signal lines 6 are arranged to extend in the vertical direction in the drawing.

When radiation is emitted in this way, once radiation is emitted to the radiation image capturing apparatus 1, the leaked data Dleak based on the electric charge q leaked through each of the TFTs 8 increases as shown in Fig. 15 in each of the radiation detection elements 7 which is provided in a location above the detecting section P corresponding to the irradiation field F of the radiation, in other words, in a location where electromagnetic waves converted by the scintillator 3 from the emitted radiation can enter.

However, in each of the radiation detection elements 7 provided in a location other than the location above the detecting section P corresponding to the irradiation field F of radiation, in other words, in a location above the detecting section P where electromagnetic waves from the scintillator 3 does not enter, the leaked data Dleak based on the electric charge q leaked through each of the TFTs 8 does not increase even when radiation is emitted to the radiation image capturing apparatus 1, because a leakage current flowing each of the TFTs 8 does not increase.

Also, as explained earlier, in the TFTs 8 connected to the radiation detection elements 7 in any location, a noise generated in the power source circuit 15a of the scanning drive unit 15 is simultaneously transmitted to each of the TFTs 8 through each of the lines L1 to Lx of the scanning lines 5. Therefore, a noise generated in the power source circuit 15a is transmitted to all of the TFTs 8 at the same time and superimposed on the leaked data Dleak to be read out.

Therefore, by utilizing this, such construction is possible that the controller 22 calculates a difference $\Delta D$ obtained by deducting leaked data Dleak, which is read out from each of the radiation detection elements 7 provided in the location above the detecting section P in which electromagnetic waves emitted from the scintillator 3 do not enter (namely, a location other than the location above the detecting section P corresponding to the irradiation field F of radiation), from leaked data Dleak which is read out from each of the radiation detection elements 7 in the location above the detecting section P in which electromagnetic waves from the scintillator 3 can enter (namely, a location above the detecting section P corresponding to the irradiation field F of radiation), and, the controller 22 detects initiation of radioactive irradiation at the point when the calculated difference $\Delta D$ exceeds the threshold value $\Delta Dth$ which is set for the said difference $\Delta D$.

It should be noted that this case presupposes that the radioactive irradiation is performed with a narrowed irradiation field F so that radiation is not emitted to the entire regions of the scintillator 3 and the detecting section P of the radiation image capturing apparatus 1 but to parts of the scintillator 3 and the detecting section P.

However, in this case, the irradiation field F of radiation emitted to the radiation image capturing apparatus 1 is usually set for the convenience of each image capture at the most appropriate position above the radiation entrance face R. Therefore, the irradiation field F may be set near the center of the radiation entrance face R as illustrated in Fig. 22, but may also be set at a position corresponding to the vicinity of edge parts of the scintillator 3 and the detecting section P, so it is impossible to specify the signal lines 6 in advance and to in advance identify the respective radiation detection elements 7 connected to those signal lines 6 as the radiation detection elements in which electromagnetic waves from the scintillator 3 do not enter.

Thus, for example, such construction is possible that the controller 22 extracts a maximum value Dleak_max and a minimum value Dleak_min from among each piece of leaked data Dleak read out from each of the signal lines 6, that is, each of the reading circuits 17, calculates a difference ΔD obtained by deducting the minimum value Dleak_min from the maximum value Dleak_max, and detects that radioactive irradiation is initiated at a point when the calculated difference ΔD exceeds a threshold value ΔDth which is set for the said difference ΔD.

However, also in this case, since an offset derived from readout characteristic of each of the reading circuits 17 is superimposed on each piece of the leaked data Dleak which is read out from each of the reading circuits 17, respective pieces of the leaked data Dleak read out from the respective reading circuits 17 become different values by the respective offsets, even when, for example, the same amount of electric charge q is leaked from each of the radiation detection elements 7 connected to each of the reading circuits 17 through the signal line 6.

Thus, for example, every time the leaked data readout process is executed, a moving average of the leaked data Dleak is calculated for each of the reading circuits 17, the leaked data Dleak having been extracted in a given number of rounds, for example, 5 or 10 rounds of the leaked data readout processes conducted in the past including the leaked data readout process immediately before the present leaked data readout process, and, the moving average is deduced from the leaked data Dleak read out in the current leaked data readout process, and the value obtained from the deduction is regarded as the leaked data Dleak read out from the reading circuit 17 in the current leaked data readout process.

Then, as described above, such construction may be adapted that the controller 22 extracts a maximum value Dleak_max and a minimum value Dleak_min from among respective pieces of leaked data Dleak which is calculated by deducting moving average from each piece of the leaked data Dleak read out from each of the signal lines 6, that is, from each of the reading circuits 17, calculates a difference ΔD obtained by deducting the minimum value Dleak_min from the maximum value Dleak_max, and detects that radioactive irradiation is initiated at a point when the calculated difference ΔD exceeds the threshold value ΔDth set for the said difference ΔD.

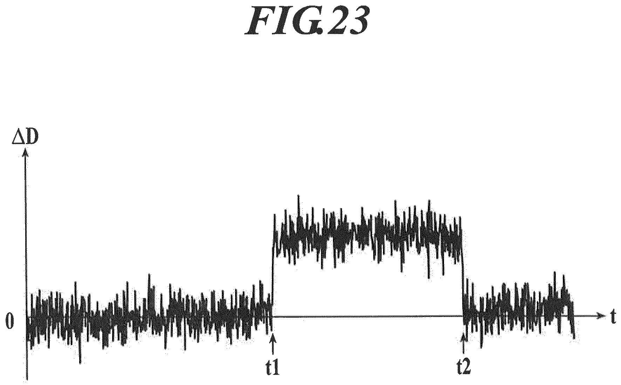

With this construction, since each of the leaked data Dleak, which is calculated by deducting moving average from leaked data Dleak readout in each of the reading circuits 7, becomes a value close to zero before radiation is emitted to the radiation image capturing apparatus 1, the difference ΔD obtained by deducting the minimum value Dleak_min from the maximum value Dleak_max becomes a value close to zero before radiation is emitted at time t1, as shown in Fig. 23.

However, for example, when radiation is emitted to the radiation image capturing apparatus 1 as depicted in Fig. 22, in the signal line 6 arranged in a location above the detecting section P corresponding to the irradiation field F of radiation, a leakage current increases which flows in each of the TFTs 8 connected to the said signal line 6, and the leaked data Dleak read out from the reading circuit 17 corresponding to the signal line 6 is increased, but, in the signal line 6 arranged in a location other than the location above the detecting section P corresponding to the irradiation field F of radiation, a leakage current which flows in each of the TFTs 8 connected to the said signal line 6 does not increase, and the leaked data Dleak read out from the reading circuit 17 corresponding to the signal line 6 is not increased.

Therefore, as shown in Fig. 23, after radiation is emitted to the radiation image capturing apparatus 1 at time t1, the difference ΔD becomes a positive value which is significantly different from zero, the difference ΔD being obtained by deducting the minimum value Dleak_min from the maximum value Dleak_max of leaked data Dleak which is calculated by deducting moving average from the leaked data Dleak read out from each of the reading circuits 17.

Hence, by setting the threshold value ΔDth to an appropriate value with respect to the difference ΔD, it becomes possible to detect initiation and end of radioactive radiation precisely as shown in Fig. 23, even in the case where very weak radiation as shown in Fig. 21 is emitted to the radiation image capturing apparatus 1.

With such a construction in which the difference ΔD is calculated as above, it at least becomes possible to remove a noise component which is derived from the power source circuit 15a and superimposed on the leaked data Dleak, and the S/N ratio of the leaked data Dleak can be improved. In addition, initiation of radioactive irradiation can be detected accurately by employing a construction so that the threshold value ΔDth is set to an appropriate value and initiation of radioactive irradiation is detected based on the calculated difference ΔD.

Note that, as stated earlier, the data shown in Fig. 21 is data in the case where radiation emitted to the radiation image capturing apparatus 1 has an extremely low dose ratio which is inconceivable in normal radiation image capturing apparatuses, and, the result shown in Fig. 23 can be obtained even for such data, so it goes without saying that the difference ΔD increases more explicitly when normal radiation is emitted with a higher dose ratio to the radiation image capturing apparatus 1.

Further, whether the dose ratio of radiation emitted to the radiation image capturing apparatus 1 is high or low, radiation may be emitted to the entire region of the radiation entrance face R (see Fig. 1 and so on) of the radiation image capturing apparatus 1 without narrowing the irradiation field F. In such a case, initiation and end of radioactive irradiation cannot be detected with the above-mentioned process procedures of [Method 1-1].

However, on the contrary, by adapting the process procedures of [Method 1-1], initiation and end of radioactive irradiation can be detected appropriately as shown in Fig. 23 even when radiation is emitted with a small dose ratio, with which initiation and end of radioactive irradiation cannot always be detected appropriately when, for example, the method stated in the explanation of the principles (see Fig. 22) is used.

Therefore, it is preferred that the actual radiation image capturing apparatus 1 be constructed so that both of the method explained in the aforementioned principles and the method described in [Method 1-1] above are used in combination, and initiation and end of radioactive irradiation be detected not only when initiation and end of radioactive irradiation is detected simultaneously by both methods, but also when initiation and end of radioactive irradiation is detected by either of the methods.

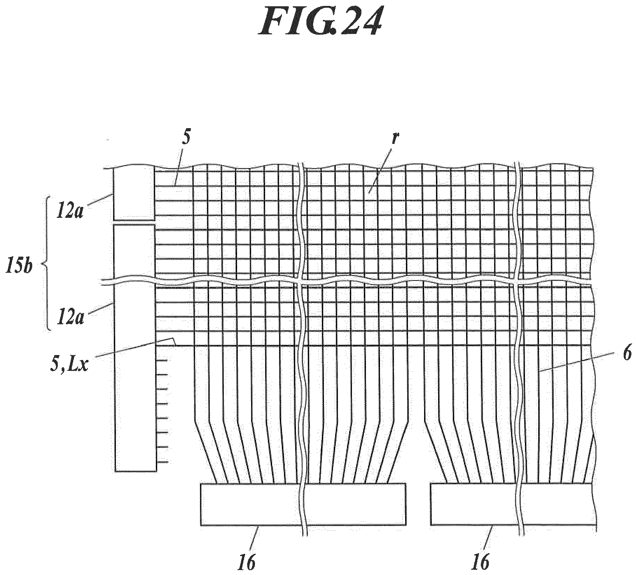

Incidentally, as shown in Fig.24, a given number of, for example, 128 or 256 of reading circuits 17 is formed in each of the reading ICs 16 (see Fig. 7). When 128 of the reading circuits 17 are formed in one reading IC 16 and there are 1024 of the signal line 6 arranged, the number of the reading ICs 16 provided is at least 8.

Then, when radiation is emitted to the radiation image capturing apparatus 1 with a narrowed irradiation field F (see Fig. 22) as mentioned above, among the 8 reading ICs 16, there may be some reading ICs 16 in which, for example, each of the radiation detection elements 7 connected to the reading IC 16 through each of the signal lines 6 becomes the radiation detection element 7 provided in a location other than the location above the detecting section P corresponding to the aforementioned irradiation field F of radiation, in other words, in a location above the detecting section P in which electromagnetic waves from the scintillator 3 do not enter.

In other words, since the irradiation field F of radiation is narrowed, it is considered that, in some reading ICs 16, radiation does not reach all of the radiation detection elements 7 connected to those reading ICs 16 (to be more precise, electromagnetic waves which have been converted from radiation in the scintillator 3 do not enter) even though radiation is emitted to the radiation image capturing apparatus 1.

Therefore, for example, instead of adapting the construction in which a maximum value and a minimum value are extracted from among respective pieces of leaked data Dleak which is calculated by deducting moving average from each piece of the leaked data Dleak read out from each of the reading circuit 17, such construction can be employed so that an average value of respective pieces of the leaked data Dleak is calculated for each of the reading ICs 16, the leaked data Dleak being calculated by deducting moving average from each piece of the leaked data Dleak read out from each of the reading circuits 17, and a maximum value and a minimum value are extracted from among the average values of the respective IC reading ICs 16.

With such a construction, since there are 8 reading ICs 16 in the above-mentioned example, the number of the average values for each of the reading ICs 16 will be 8 as well, thus making it possible to easily execute the process for extracting the maximum value and the minimum value.

On the other hand, in the actual radiation image capturing apparatus 1, there are several thousands to several tens of thousands of the signal lines 6 and the corresponding reading circuits 17, and, in any of the above-mentioned cases, moving averages must be calculated for all of them, and the moving averages must be deducted from the respective pieces of the leaked data Dleak read out from the respective reading circuits 17, so these processes can be time consuming.

Then, when it takes time to execute each of the processes stated above, a problem may occur in that determination of whether radioactive irradiation is initiated is delayed in each leaked data readout, and a line defect appears continuously on a radiological image p as described later.

Therefore, instead of deducting a moving average from each piece of leaked data Dleak read out from each of the reading circuits 17 as stated above by utilizing the fact that a given number, for example, 128 or 256 of the reading circuits 7 are formed in the reading IC 16 as shown in Fig. 24, such a construction is possible that, for example, an average value of 128 pieces of leaked data Dleak is calculated first for each of the reading ICs 16 in one round of the leaked data readout process, the 128 pieces of leaked data Dleak being outputted from the respective reading circuits 17 for one reading IC 16.

With this construction, the number of the average values of the respective pieces of the leaked data Dleak for each of the reading ICs 16 will be eight in each round of the leaked data readout processes, and 8 is equal to the number of the reading ICs 16.

Then, the construction may be such that a moving average is calculated with regard to the average values of the leaked data Dleak for each of these 8 reading ICs 16, the moving average is deducted from each of the average values, the average values from which the moving averages have been deducted, are compared to each other, a maximum value and a minimum value are extracted from among said average values, a difference $\Delta D$ is calculated by deducing the minimum value from the maximum value, and initiation of radioactive irradiation is detected at a point when the calculated difference ΔD exceeds the threshold value ΔDth.

With this construction, initiation and end of radioactive irradiation can be detected precisely as stated earlier, and, at the same time, it is no longer necessary to calculate moving average of 1024 pieces of leaked data Dleak read out in the respective reading circuits 17 in one round of the leaked data readout process, and it is only necessary to calculate moving average with respect to the average values of the leaked data Dleak for each of the 8 reading ICs 16.

Because of this, it becomes possible to rapidly conduct the series of processes including calculation of the moving average, deduction of the moving average from the average value of the leaked data Dleak, extraction of the maximum value and the minimum value, calculation of the difference ΔD, and comparison between the difference ΔD and the threshold value ΔDth, and determination of whether or not radioactive irradiation is initiated, which is performed in every round of the leaked data readout process, is done swiftly.

Also, with such a construction that the average value of the respective pieces of the leaked data Dleak is calculated for each of the reading ICs 16, since electrical noises generated in the large number of reading circuits 17 in the reading IC 16 are balanced each other out when calculating the average value of the leaked data Dleak, there is a benefit that impact can be reduced on leaked data Dleak of the electrical noises generated in the respective reading circuits 17 and the moving average thereof.

[Method 1-2]

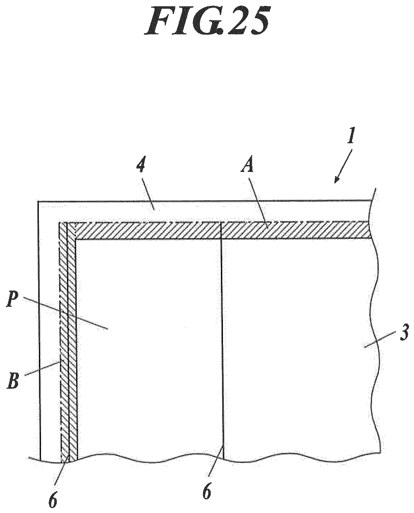

Meanwhile, as schematically illustrated in Fig. 25, in some radiation image capturing apparatus 1, the scintillator 3 is originally formed to be smaller than the detecting section P provided on the substrate 4. Note that the signal line 6 is also arranged so as to extend in the vertical direction in the drawing.

Further, when the radiation image capturing apparatus 1 is constructed like this, once radiation is emitted to the radiation image capturing apparatus 1, leaked data Dleak based on the electric charge q leaked through each of the TFTs 8 as stated above increases as shown in Fig. 15 in each of the radiation detection elements 7 provided in a location right beneath the scintillator 3 on the detecting section P, in other words, in a location where an electromagnetic wave converted at the scintillator 3 from radiation can enter.

However, in each of the radiation detection elements 7 provided in a location other than the location right beneath the scintillator 3 on the detecting section P, in other words, in a location on the detecting section P where an electromagnetic wave from the scintillator 3 does not enter, leakage current flowing in each of the TFTs 8 does not increase even when radiation is emitted to the radiation image capturing apparatus 1, so leaked data Dleak based on the electric charge q leaked through each of the TFTs 8 is not increased.

Moreover, as stated earlier, in the TFTs 8 connected to the radiation detection elements 7 in any location, noises generated in the power source circuit 15a of the scanning drive unit 15, the bias power source 14, and the like are transmitted simultaneously to each of the TFTs 8 and each of the radiation detection elements 7 through the lines L1 to Lx of the scanning lines 5. Hence, the noises generated in the power source circuit 15a and the like are transmitted to all of the TFTs 8 at the same time and superimposed on the leaked data Dleak which is read out.

Thus, by utilizing this, such construction can be employed that the controller 22 calculates a difference ΔD obtained by deducting leaked data Dleak, which is read out in each of the radiation detection elements 7 in a location above the detecting section P where a electromagnetic wave emitted from the scintillator 3 does not enter (in other words, a location other than the location immediately below the scintillator 3), from leaked data Dleak, which is read out from each of the radiation detection elements 7 provided in a location above the detecting section P where an electromagnetic wave emitted by the scintillator 3 can enter (in other words, a location immediately below the scintillator 3), and, similarly to the foregoing, initiation of radioactive irradiation is detected at a point when the calculated difference ΔD exceeds the threshold value ΔDth.

In this case, among the signal lines 6 in the locations other than the location immediately beneath the scintillator 3, in the signal lines 6 arranged in a location A other than the location right beneath the scintillator 3 marked with diagonal lines in Fig. 25, an electric charge q leaked from each of the radiation detection elements 7 in the location A flows thereinto, but an electric charge q leaked from each of the radiation detection elements 7 in the location immediately beneath the scintillator 3 also flows thereinto. Therefore, leaked data Dleak read out in each of the reading circuits 17 provided in these signal lines 6 is treated as the former leaked data Dleak among the two types of leaked data Dleak explained above, the former leaked data Dleak being read out from each of the radiation detection elements 7 in a location above the detecting section P where a electromagnetic wave emitted from the scintillator 3 can enter (in other words, a location immediately below the scintillator 3).

On the other hand, among those in a location other than a location immediately below the scintillator 3, all of the radiation detection elements 7 connected to the signal lines 6 in a location B other than the location immediately below the scintillator 3 marked with diagonal lines in Fig. 25 are in the location B other than the location immediately beneath the scintillator 3, and an electromagnetic wave emitted from the scintillator 3 does not enter in each of these radiation detection elements 7.

Therefore, leaked data which is read out for each of the signal lines 6 in this location B, in other words, leaked data which is read out for each of the reading circuits 17 provided in said signal lines 6, does not contain contribution mixed in by the electric charge q leaked from each of the radiation detection elements 7 in the location right below the scintillator 3, and, leaked data Dleak which is unrelated to radioactive irradiation or an electromagnetic wave emitted by the scintillator 3, in other words, leaked data Dleak caused by a noise in the power source circuit 15a of the scanning drive unit 15 is read out from each of these radiation detection elements 7.

Therefore, leaked data Dleak read out in each of the radiation detection elements 7 provided in the signal lines 6 which are arranged in the location B (in other words, the signal lines 6 arranged in a location other than the location immediately beneath the scintillator 3, throughout the entire length thereof) is treated as the latter leaked data Dleak among the foregoing two types of leaked data Dleak, the latter leaked data Dleak being read out from each of the radiation detection elements 7 provided in a location immediately above the detecting section P in which an electromagnetic wave emitted from the scintillator 3 does not enter (in other words, a location other than the location immediately beneath the scintillator 3).

It should be noted that, in a case where the above-mentioned construction is used and the difference ΔD is calculated as stated above, such construction is also possible that, for example, one piece of leaked data Dleak is selected from among leaked data Dleak read out in each of the reading circuits 17 which is provided in each of the signal lines 6 that are arranged in the aforementioned location B and is used as the latter leaked data Dleak read out from each of the radiation detection elements 7 provided in the location immediately above the detecting section P in which an electromagnetic wave emitted from the scintillator 3 does not enter (in other words, the location other than the location immediately beneath the scintillator 3), or, an average value of such leaked data Dleak is calculated and used as the latter leaked data Dleak.

Also, in the case where this construction is employed, if, for example, the difference ΔD is calculated in the above-mentioned way based on the data shown in Fig. 21, a noise component which is derived from the power source circuit 15a and superimposed on the leaked data Dleak is removed appropriately from the leaked data Dleak as shown in Fig. 23. Thus, an increase of the leaked data Dleak due to radioactive irradiation can be extracted as an increase of the difference ΔD.

As described above, when the radiation image capturing apparatus 1 is constructed as shown in Fig. 25, at least a noise component which is derived from the power source circuit 15a and superimposed on the leaked data Dleak can be removed by adapting the construction where the difference ΔD is calculated by executing the respective processes as described above, and the S/N ratio of the leaked data Dleak can be improved. In addition, by employing the construction in which initiation of radioactive irradiation is detected based on the calculated difference ΔD, initiation of radioactive irradiation can be accurately detected.

Note that, in the case of this [Method 1-2], since an offset derived from readout characteristic of each of the reading circuits 17 is superimposed on each piece of the leaked data Dleak which is read out from each of the reading circuits 17, it is preferred that processes are executed similarly to the foregoing [Method 1-1] in every round of the leaked data readout process, said processes including calculation for each of the reading circuits 17 of moving average of leaked data Dleak read out in each of the reading circuits 17 that is provided in each of the signal lines 6 arranged in the locations A and B, said leaked data Dleak having been read out in a given number of rounds of the past leaked data readout processes including the leaked data readout process immediately before the current round of the leaked data readout process, deduction of the moving average from the leaked data Dleak read out in the present round of the leaked data readout process, and a process which treats the value obtained from the deduction as said leaked data Dleak read out from the reading circuit 17.

Further, in this case, it is decided as necessary whether the construction should be employed to always conduct the process which treats the value obtained by deducting the moving average from the leaked data Dleak read out from each of the reading circuit 17 as the leaked data Dleak, or to conduct the process only when a dose ratio of radiation is very low.

[Method 2]

Alternatively, as a method for improving the S/N ratio of leaked data Dleak, it is also possible to adapt such construction that the capacitance of the capacitor 18 of the amplifier circuit 18 constructed by the foregoing charge amplifier can be changed, and, during the leaked data readout process which is repeatedly executed before radiation image capturing operation, the capacitance cf of the capacitor 18b of the amplifier circuit 18 can be changed to become smaller than the capacity during the image data readout process.

As explained earlier, the amplifier circuit 18 outputs a voltage value corresponding to the electric charges q which have been leaked from the radiation detection elements 7 and flown into and accumulated in the capacitor 18b, and, by changing the capacitance cf of the capacitor 18a to a smaller one, the voltage value V outputted from the amplifier circuit 18 can be increased even when the same amount of electric charges q is accumulated in the capacitor 18b in accordance with the relationship V = q/cf.

Regarding a noise component which is originally superimposed on the electric charges q leaked from the radiation detection elements 7, in other words, for example, a noise component derived from the power source circuit 15a as stated above, the noise component is increased along with an increase of the voltage value V outputted from the amplifier circuit 18, and the S/N ration is not improved, but at least a noise component generated in the reading circuit 17 including the amplifier circuit 18 does not increase even if the voltage value V is increased.

Therefore, in this case, improvement of the S/N ratio is possible at least for a noise component generated in the reading circuit 17 including the amplifier circuit 18.

Note that, when the capacitance cf of the capacitor 18b is reduced excessively, the capacitor 18b is easily saturated with each of the electric charge q leaked from each of the radiation detection elements 7, and, since saturation of the capacitor 18b may negatively affect the following readout processes in the reading circuit 17 including the said capacitor 18b, the capacitance cf of the capacitor 18b is adjusted so as to be reduced to an appropriate value. In addition, in the image readout process executed after radioactive irradiation to the radiation image capturing apparatus 1, the capacitance cf of the capacitor 18b is returned to the predetermined normal capacitance.

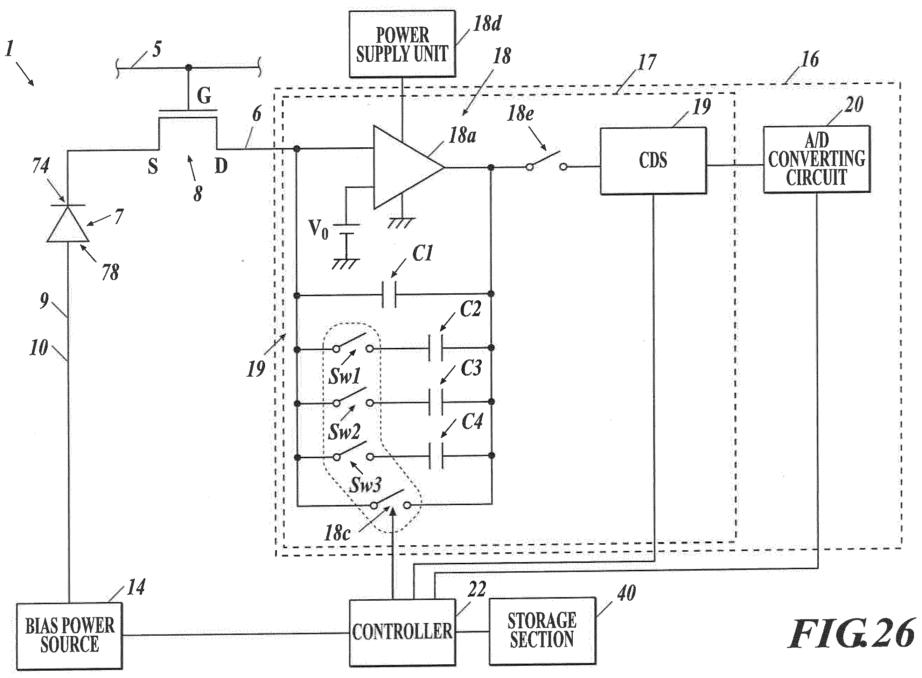

Also, such construction can be used that the capacitance of the capacitor 18b of the amplifier circuit 18 can be changed by, for example, configuring the amplifier circuit 18 of the reading circuit 17 like Fig. 26.

To be specific, instead of using one capacitor 18b as a capacitor connected in parallel to the operational amplifier 18a of the amplifier circuit 18 constructed by the charge amplifier circuit as illustrated in Fig. 8, capacitors C1 to C4 are connected in parallel to each other. Then, switches Sw1 to Sw3 are connected to the capacitors C2 to C4, respectively. Note that, such construction is possible that a switch is connected to the capacitor C1 in series as well.

Further, by switching on/off of the switches Sw1 to Sw3, the capacitance of the capacitor 18b of the amplifier circuit 18 can be changed. It should be noted that, in this case, the capacitance cf of the capacitor 18b is a sum total value of the capacitance of the capacitor C1 and the respective capacitances of the capacitors C2 to C4 connected in series to the switches in the on state out of the switches Sw1 to Sw3.

[Method 3]

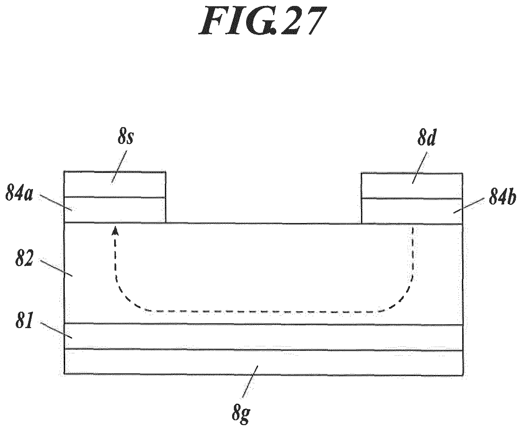

Also, the leaked data Dleak is derived from a leakage current Ioff which flows in the TFT 8 in the off state, as stated earlier. In this regard, as illustrated in Fig. 27 which schematically shows the cross-sectional construction of the TFT 8 illustrated in Fig. 5, since an off voltage is applied to the gate electrode 8g of the TFT 8, a density of electrons is thus small in the semiconductor layer 82 of the TFT 8 on the gate electrode 8g side (the lower side in Fig. 27).

It is considered that, because holes flow in this region of the semiconductor layer 82 with a small electron density on the gate electrode 8g side, the leakage current Ioff flows in the TFT 8 which is in the off state. It should be noted that, in this case, since a reverse bias voltage is applied to the second electrode 78 (illustration thereof is omitted in Fig. 27) of the radiation detection element 7, which is connected to the source electrode 8s, the leakage current Ioff flows from the drain electrode 8d side where potential is relatively high, through a region of the semiconductor layer 82 on the gate electrode 8g side, and to the source electrode 8s side where potential is relatively low.

Meanwhile, once radiation is emitted to the radiation image capturing apparatus 1 and an electromagnetic wave converted from the radiation in the scintillator 3 (illustration thereof is omitted in Fig. 27) is emitted, electron hole pairs are generated in the semiconductor layer 82 of the TFT 8 mainly on the scintillator 3 side (upper side in Fig. 27) because the scintillator 3 is provided on the upper side in the drawing.

Then, since the electron density is relatively high in the semiconductor layer 82 on the scintillator 3 side as described above, it is highly probable that holes generated therein are recombined with electrons. Therefore, as described above, as an electromagnetic wave is emitted from the scintillator 3 due to radioactive irradiation, electron hole pairs are generated within the semiconductor layer 82 of the TFT 8 and the quantity of the leakage current Ioff flowing in the TFT 8 in the off state increases, but the rate of increase of the leakage current Ioff is reduced because some holes which serve as carriers are recombined with the electrons.

Thus, by creating a region in the semiconductor layer 82 of the TFT 8 with a low electron density on the scintillator 3 side, holes which serve as carriers flow two channels, which are the region in the semiconductor layer 82 on the side of the gate electrode 8g and the region in the semiconductor layer 82 on the side of the scintillator 3, thus making it possible to increase the value of the leaked data Dleak more. Moreover, by increasing the value of the leaked data Dleak, the S/N ratio of the leaked data Dleak can be improved.

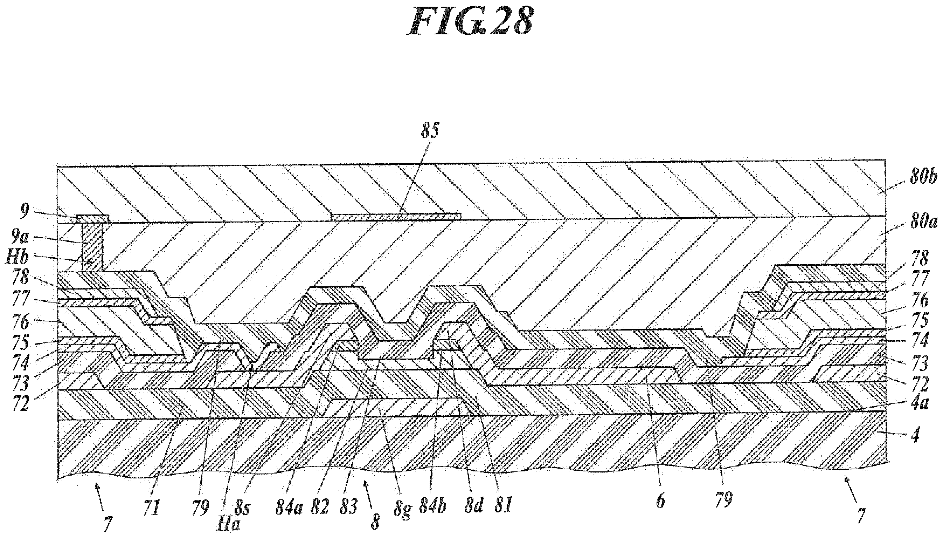

In order to create the region having a low electron density also on the scintillator 3 side in the semiconductor layer 82 of the TFT 8, a wire 85 is arranged on the side of the scintillator 3 (illustration thereof is omitted in Fig. 28; it is provided on the upper side of the drawing) of the TFT 8 as illustrated in Fig. 28, and a negative voltage can be applied to the wire 85 during the leaked data readout process which is executed repeatedly at least before radiation image capturing operation.

Specifically, the wire 85 are formed from a conductive material such as ITO which transmits an electromagnetic wave emitted from the scintillator 3, and, for example, the wires 85 are provided as many as the respective signal lines 6 in parallel with the respective signal lines 6. Further, during the leaked data readout process which is repeatedly executed at least before radiation image capturing operation, a negative voltage, which is, for example, the same as the off voltage applied each of the scanning line 5 by the scanning drive unit 15, is applied thereto.

Note that, the negative voltage to be applied to each of the wires 85 does not necessarily have to be a negative voltage which is the same value as the off voltage, and is set to a voltage with which the region having a low electron density can be created appropriately in the semiconductor layer 82 of the TFT 8 on the scintillator 3 side, as stated above. In addition, it is also possible to use such construction that an off voltage is applied to each of the wires 85 from the power source circuit 15a of the scanning drive unit 15, or that a negative voltage is applied to each of the wires 85 from other power source circuit.

Further, at least during the image data readout process which is conducted after radioactive irradiation to the radiation image capturing apparatus 1, application of the negative voltage to each of the wires 85 is stopped (in other words, a floating state is entered) or a given voltage such as 0V is applied to each of the wires 85 in order to prevent an adverse effect on reading of image data d from each of the radiation detection elements 7.

In addition, Fig. 28 shows a case where the wire 85 and the bias line 9 are formed on an upper surface of a first planarizing layer 80a (in other words, the surface on the side of the non-illustrated scintillator 3) which is formed above the radiation detection element 7 and the TFT 8 in a laminated fashion, and a second planarizing layer 80b is further formed on top thereof, but the form for forming the wires 85 is not limited to this form, and the wires 85 can be arranged at appropriate positions as long as a region having a low electron density can be formed in the semiconductor layer 82 of the TFT 8 on the scintillator 3 side.

[Method 4]

As known from comparison between the leaked data readout process shown in Fig. 12 and the image data readout process shown in Fig. 10, descriptions of this embodiment have been given so far on the assumption that the leaked data readout process is executed at the same timing with the image data readout process. In other words, the explanation was about the case where the leaked data readout process is executed so that a time span from transmission of the first pulse signal Sp1 to the correlated double sampling circuit 19 from the controller 22 until transmission of the second pulse signal Sp2 is same as the time span in the case of the image data readout process.

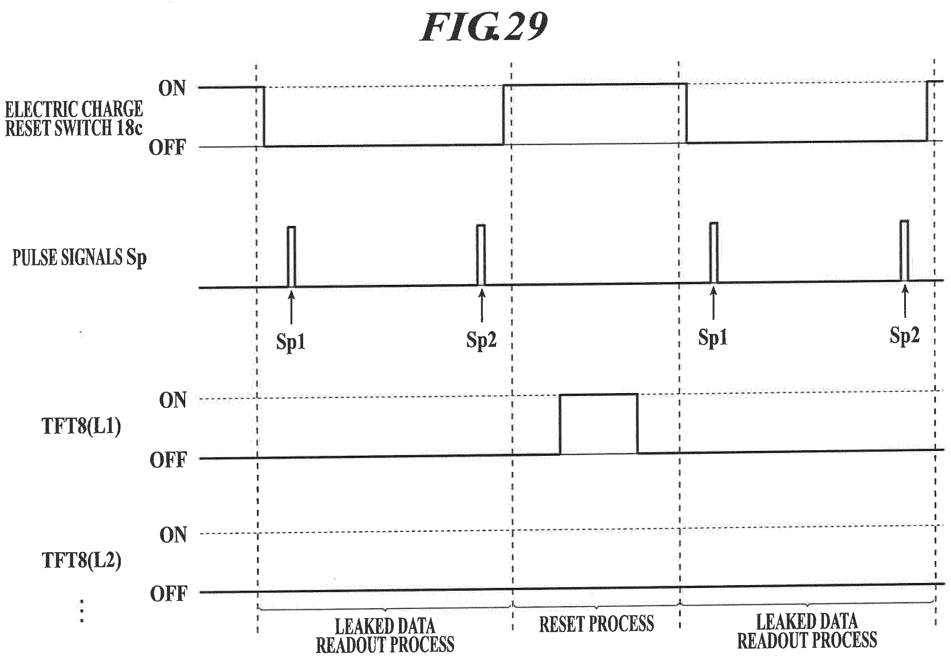

However, the leaked data readout process does not necessarily need be carried out at the same timing with the image data readout process, and, the S/N ratio of the leaked data Dleak can be improved as the time span between transmissions of the pulse signals Sp1 and Sp2 to the correlated double sampling circuit 19 from the controller 22 is controlled in the leaked data readout process so that the time span is longer than the time span of the image data readout process, as shown in Fig. 29.

Namely, as shown in Fig. 29, by extending the time span between transmissions of the pulse signals Sp1 and Sp2 during each round of the leaked data readout processes, the amount of an electric charge q leaked from each of the radiation detection elements 7 and accumulated in the capacitor 18b of the amplifier circuit 18 is increased accordingly, and the value of the leaked data Dleak is thus increased.

However, a noise component superimposed on the leaked data Dleak does not increase over time, and is a difference between a noise component, which is superimposed on the voltage value Vin from the amplifier circuit 1 and held when the first pulse signal Sp1 is transmitted to the correlated double sampling circuit 19, and a noise component, which is superimposed on the voltage value Vfi from the amplifier circuit 18 and held when the second pulse signal is transmitted, so the noise component does not increase even though the time span between transmissions of the pulse signals Sp1 and Sp2 is extended.

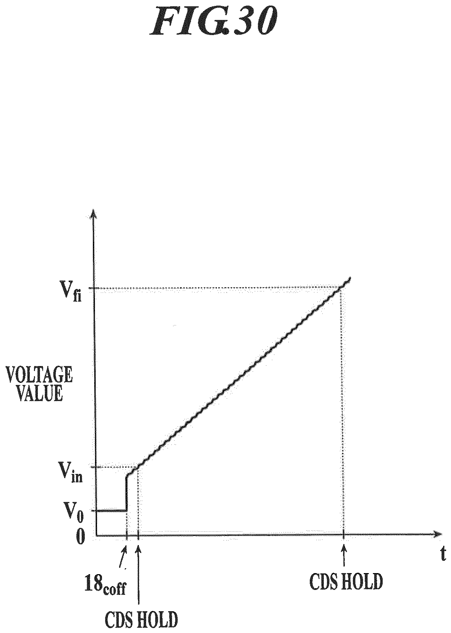

In other words, as illustrated in Fig. 30, as the time span becomes longer between transmissions of the pulse signals Sp1 and Sp2 to the correlated double sampling circuit 19 from the controller 22, the amount of an electric charge 1 leaked from each of the radiation detection elements 7 and accumulated in the capacitor 18b of the amplifier circuit 18 increases, the voltage value outputted form the amplifier circuit 18 rises, and the difference between the voltage values Vin and the voltage values Vfi is significantly increased to a large value.

On the other hand, a noise component superimposed on the leaked data Dleak can be expressed as a vibration of the aforementioned voltage value which increases and decreases minutely over time. Further, the noise component expressed as the vibration is not the one that the vibration amplitude thereof (or the amplitude of vibration in the vertical direction in Fig. 20) increases depending on time, but is the one that has almost constant amplitude of vibration without depending on time and is superimposed on a voltage value (or a voltage value leaked out as leaked data Dleak) which increases over time.

Therefore, the noise component superimposed on the leaked data Dleak does not increase even if the time span between transmissions of the pulse signals Sp1 and Sp2 is extended.

Hence, as stated above, the leaked data Dleak is increased by controlling the time span between transmissions of the pulse signals Sp1 and Sp2 during the leaked data readout process to be longer than the time span during the image data readout process, but the noise component superimposed on the leaked data Dleak does not increase, thus enabling to improve the S/N ratio of the leaked data Dleak.

It should be noted that Fig. 29 shows a case where the time span between transmissions of the pulse signals Sp1 and Sp2 during each round of the leaked data readout process is extended when the construction is employed so that the reset process of each of the radiation detection element 7 is executed between the leaked data readout process and the next round of the leaked data readout process as shown in Fig. 19, but, when such construction is employed that the image data readout process is executed between the leaked data readout processes as shown in Fig. 20, the time span between transmissions of the pulse signals SP1 and SP2 in each round of the leaked data readout processes can be extended as well.

Alternatively, such construction is also possible that the aforementioned methods 1 to 4 are executed as a combination as appropriate.

[Process for preventing continuous line defect from appearing]

The following problems may occur when the reset process for each of the radiation detection elements 7 or the image data readout process for each of the radiation detection elements 7 is executed between the leaked data readout process and the next round of the leaked data readout process as shown in Figs. 19 and 20 in order to remove excessive electric charges such as dark electric charges generated in each of the radiation detection elements 7 before conducting the leaked data readout process repeatedly executed in a periodic manner prior to radiation image capturing operation, and detection of initiation of radioactive irradiation based on the read out leaked data Dleak.

Note that the following explanation pertains to a case where the reset process of each of the radiation detection elements 7 is conducted between the leaked data readout process and the next round of the leaked data readout process, but the same explanation will be applied to the case where the image data readout process for each of the radiation detection elements 7 is executed between the leaked data readout processes.

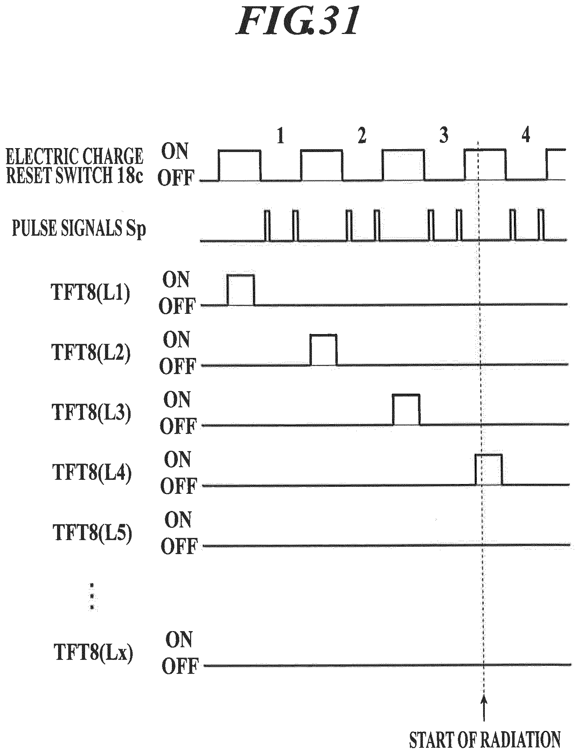

When the reset process is conducted for each of the radiation detection elements 7 between the leaked data readout processes, it is supposed that the construction is such that, for example, the first round of the leaked data readout process is executed after an on voltage is applied to the line L1 of the scanning lines 5, and the second round of the leaked data readout process is executed after the on voltage is applied to the line L2 of the scanning lines 5 as shown in Fig. 31. The numbers in the upper part of the timing chart of the electric charge reset switch 18c as shown in Fig. 31 and so on represent the round numbers of the leaked data readout processes.

When, for example, initiation of radioactive irradiation is not detected based on the leaked data Dleak read out in the third round of the leaked data readout process, but initiation of radioactive irradiation is detected based on the leaked data Dleak read out in the fourth round of the leaked data readout process, some useful electric charges generated in each of the radiation detection elements 7 due to radioactive irradiation are discharged to the signal lines 6 through each of the TFTs 8 from each of the radiation detection elements 7 connected to the line L4 of the scanning lines 5, on which the on voltage is applied during the reset process immediately before the fourth round of leaked data readout process.

Therefore, it may be difficult to say that each piece of the image data d, which is read out from each of the radiation detection elements 7 connected to the line L4 of the scanning lines 5 during the image data readout process that is executed after radioactive irradiation to the radiation image capturing apparatus 1, is always useful data.

Thus, in the case of the above-mentioned construction, the image data d may be regarded invalid, the image data d being read out from each of the radiation detection elements 7 connected to the scanning line 5 (the line L4 of the scanning lines 5 in the above example) to which the on voltage is applied during the reset process immediately before the leaked data readout process (the fourth round of the leaked data readout process in the above example) in which initiation of radioactive irradiation is detected based on the leaked data Dleak.

In the case of this construction, since invalid image data d is lined up linearly along the scanning line 5 on a radiological image p which is generated based on the image data d which has been read out, so-called line defect happens. Therefore, in such a case, for example, image data d that is regarded as invalid is abandoned with regard to each of the radiation detection elements 7 connected to the line L4 of the scanning lines 5 in which the image data d is considered invalid, and each of image data d is calculated by carrying out, for example, linear interpolation for each image data d that is read out from each of the radiation detection elements 7 connected to the line L3 and the line L5 of the scanning lines 5 which are in the vicinity of the said scanning line 5.

On one hand, when radiation is emitted to the radiation image capturing apparatus 1 from a radiation generator, once dose of radiation rises instantaneously and reaches a predetermined dose immediately after irradiation is initiated, initiation of radioactive irradiation can be detected based on the leaked data Dleak which is read out in the first round of the leaked data readout process after radioactive irradiation begins (the fourth round of leaked data readout process in the aforementioned example) as shown in Fig. 31.

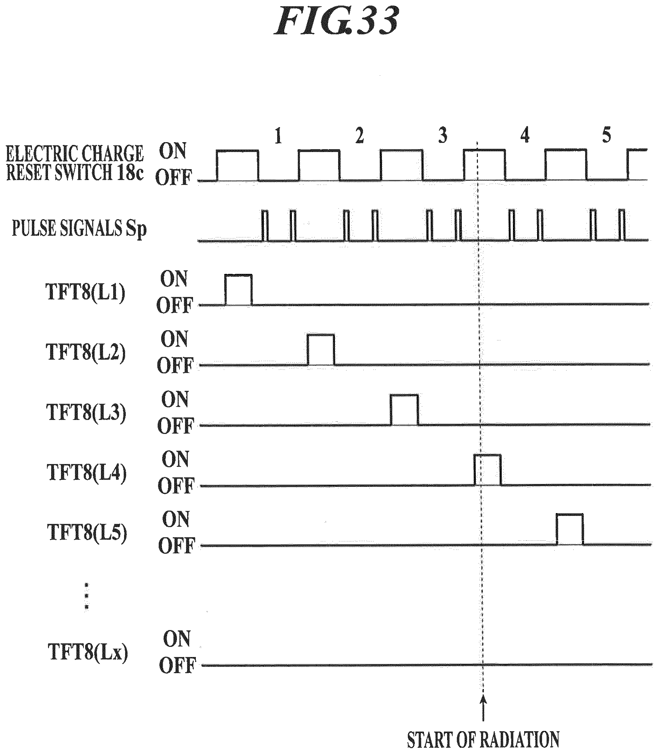

On the other hand, when a dose of radiation emitted from the radiation generator rises slowly, the leaked data Dleak read out in the fourth round of the leaked data readout process does not exceed the threshold value Dth stated earlier even though radioactive irradiation was initiated at a point when the fourth round of the leaked data readout process was actually executed as shown in Fig. 33, and, initiation of radioactive irradiation is detected only after the leaked data Dleak read out in the fifth round of the leaked data readout process exceeds the threshold value Dth after the reset process of each of the radiation detection elements 7 is executed.

In such a case, an electric charge generated in each of the radiation detection elements 7 due to radioactive irradiation is discharged to the signal line 6 through each of the TFTs8 from each of the radiation detection elements 7 which are connected to the line L5 of the scanning lines 5 to which the on voltage is applied during the reset process executed immediately before the fifth round of leaked data readout process, in addition to the reset process executed immediately before the fourth round of the leaked data readout process as stated above. Therefore, not only each piece of the image data d which is read out from each of the radiation detection elements 7 connected to the line L4 of the scanning lines 5, but also each piece of the image data d which is read out from each of the radiation detection elements 7 connected to the line L5 of the scanning lines 5 are hardly said useful and has to be regarded invalid.

Then, once the image data d from the radiation detection elements 7 respectively connected to the lines L4 and L5 of the scanning lines 5 (or each of the other following lines of the scanning lines 5) is regarded invalid as stated above, each piece of the invalid image data d is line up linearly along the lines L4 and L5 (or following lines) of the scanning lines 5 on the radiological image p, and line defects appear in a continuous fashion.

It may be advisable to employ such construction that, even through continuous line defects appear on a radiological image p as stated above, each piece of image data d from each of the radiation detection elements 7 connected to the lines L4 and L5 of the scanning lines 5 is calculated by, for example, conducting linear interpolation in each piece of image data d which is read out from each of the radiation detection elements 7 which are respectively connected to the lines L3 and L6 of the scanning lines 5 adjacent to the said scanning lines 5, similarly to the foregoing.

However, in a case where, for example, the radiological image p is used for diagnostic purposes in medicine, a small affected part which is supposed to be captured in the radiological image p may be modified by the aforementioned linear interpolation and erased from the radiological image p. Therefore, a process is required to prevent continuous line defects from appearing on the radiological image p even when the dose of radiation emitted from the radiation generator rises slowly as described earlier. This process will be explained below.

[Process 1]

As a process for preventing continuous line defects from appearing on a radiological image p, for example, the time span between transmissions of the pulse signals Sp1 and Sp2 to the correlated double sampling circuit 19 from the controller 22 during the leaked data readout process can be extended so as to be longer than the time span during the image data readout process, like the foregoing method 4 (see Fig. 29) for improving the S/N ratio of the leaked data.

With such construction, a time period required for one round of the leaked data readout process becomes longer as stated above, and, in addition, the amount of electric charges q leaked from each of the radiation detection elements 7 and accumulated in the capacitor 18b of the amplifier circuit 18 is increased and the value of the leaked data Dleak increases, so it becomes possible to enhance probability to be able to detect initiation of radioactive irradiation during one round of the leaked data readout process even when a dose of radiation emitted from radiation generator rises slowly as stated above.

[Process 2]

A reason why line defects can appear in a continuous manner on a radiological image p is considered to be, for example, sequential application of the on voltage to each of the lines L1 to Lx of the scanning lines 5 while shifting the lines to the next one during the reset process of each of the radiation detection elements 7 which is executed between the leaked data readout processes as shown in Fig. 33.

Therefore, as another process for preventing continuous line defects from appearing on a radiological image p, when the on voltage is applied sequentially to each of the lines L1 to Lx of the scanning lines 5 from the scanning drive unit 15, the reset process of each of the radiation detection elements 7 is executed by, for example, applying the on voltage to the scanning line 5 except for the scanning line 5 to which the on voltage was applied in the last reset process, instead of conducting the reset process of each of the radiation detection elements 7 by sequentially applying the on voltage to each of the lines L1 to Lx of the scanning lines 5 while shifting the lines to the next one as describe above.

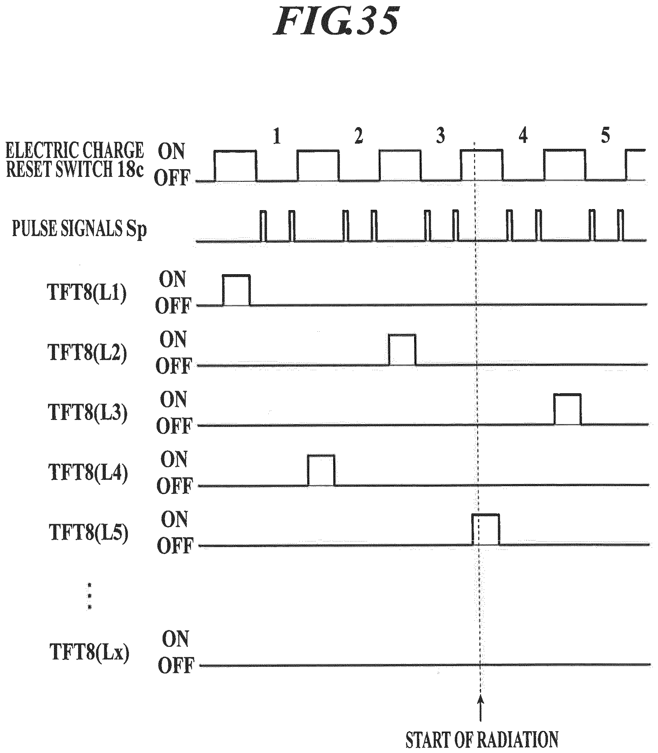

Specifically, as shown in Fig. 35, the reset process of each of the radiation detection elements 7 is executed so that a line of the scanning lines 5 to which the on voltage is applied in order to conduct the reset process is not one that is next to the line of the scanning lines 5 to which the on voltage was applied to execute the reset process right before the current round of the reset process.

With such a construction, for example, when initiation of radioactive irradiation is detected based on the leaked data Dleak read out in the fifth round of the leaked data readout process as stated above even though radioactive irradiation was actually initiated at a point when the fourth round of the leaked data readout process was executed, not only the image data d from each of the radiation detection elements 7 connected to the line L5 of the scanning line 5 in which the reset process was executed immediately before the fourth round of the leaked data readout process, but also the image data d from each of the radiation detection elements 7 connected to the line L3 of the scanning lines 5 in which the reset process was executed immediately before the fifth round of leaked data readout process, are regarded invalid.

Thus, in this case, since image data d is regarded invalid, which is read out from each of the radiation detection elements 7 respectively connected to the lines L3 and L5 of the scanning lines 5, it becomes possible for line defects on a radiological image p not to appear in a continuous fashion although not illustrated.

In the example shown in Fig. 35, however, line defects do appear at positions adjacent to each other on a radiological image p even though the line defects do not appear in a continuous fashion, and which is not necessarily preferable. Therefore, in reality, it is preferred that the line L of the scanning lines 5 to which the on voltage is applied to execute the reset process be widely spaced from the line L of the scanning lines 5 to which the on voltage is applied right after that.

Hence, for example, in a case where the aforementioned scanning drive unit 15 is constructed so that the scanning lines 5 are respectively connected to, for example, 128 terminals of each of the gate IC 12a (see Fig. 6) which constructs the gate driver 15b, the on voltage is first applied to the scanning line 5 connected to the first terminal of the first gate IC 12a to execute the reset process of each of the radiation detection elements 7, and, for the next reset process, the on voltage is applied to the scanning line 5 connected to the first terminal of the second gate IC 12a to execute the reset process.

This way, such construction can be employed to execute the reset process of each of the radiation detection elements 7 that, after the reset processes are executed as above by applying the on voltage sequentially to the scanning lines 5 connected to the first terminals of the respective ICs 12a, the on voltage is applied to the scanning lines 5 connected to the second, third, and following terminals of each of the gate ICs 12a.

Note that it is possible to adapt a construction in which a combination of the above-mentioned Process 1 and Process 2 is executed.

[Process for applying on voltage simultaneously to a plurality of scanning lines]

Explained in each of the examples was the case where the reset process of each of the radiation detection elements 7 and the image data readout process of each of the radiation detection elements 7 in the periodically-executed leaked data readout process are conducted by applying the on voltage sequentially to each of the lines L1 to Lx of the scanning lines 5 from the scanning drive unit 15, but it is also possible to have a construction so that the reset process of each of the radiation detection elements 7 and the image data readout process of each of the radiation detection elements 7 are executed by applying the on voltage to the plurality of lines L1 to Lx of the scanning lines 5 simultaneously.

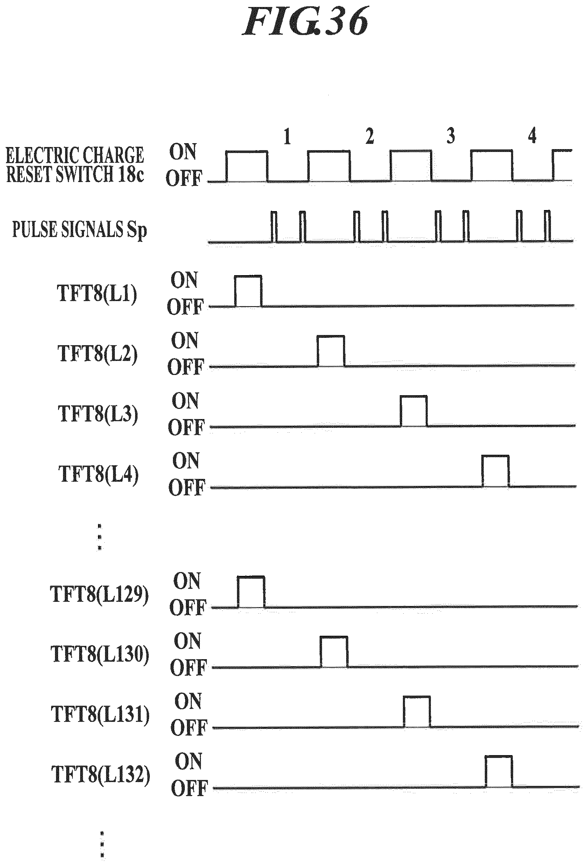

For example, as stated above, in a case where 128 of the scanning lines 5 are respectively connected to the terminals of each of the gate ICs 12a which constructs the gate driver 15b of the scanning drive unit 15, the reset process of each of radiation detection elements 7 and the like is executed by simultaneously applying the on voltage to the respective scanning lines 5 connected to the first terminals of each of the gate ICs 12a, and the next reset process is executed by simultaneously applying the on voltage to the respective scanning lines 5 connected to the second terminals of each of the gate ICs 12a, as shown in Fig. 36.

In doing so, in order to prevent line defects from appearing in a continuous fashion on a radiological image p as stated earlier, the plurality of lines L of the scanning lines 5 to which the on voltage is applied simultaneously are the plurality of scanning lines 5 which are not adjacent to each other on the detecting section P.

With this construction, the on voltage is applied to each of the scanning lines 5 in a shorter cycle, and excessive electric charges such as dark electric charges accumulated in each of the radiation detection elements 7 connected to each of the scanning lines 5 can be reduced.

Note that, Fig. 36 shows the case where the reset process of each of the radiation detection elements 7 is executed by sequentially applying the on voltage to each of the lines L1 to Lx of the scanning lines 5 while shifting the line to the next one as shown in Fig. 31 for each of the gate ICs 12a, but, as shown in Fig. 35, it is possible to adapt a construction in which the reset process of each of the radiation detection elements 7 is executed by sequentially applying the on voltage to each of the lines L1 to Lx of the scanning lines 5 so that each of the lines L1 to Lx to which the on voltage is applied in tandem is not the scanning line neighboring each other.

Further, like Process 1 described above, it is possible to extend the time span between transmissions of the pulse signals Sp1 and Sp2 to the correlated double sampling circuit 19 from the controller 22 during the leaked data readout process, and this process is combined with an appropriate process procedures for execution.

[Processes after detection of initiation of radioactive irradiation]

Described next will be processes after initiation of radioactive irradiation is detected by the controller 22 based on the leaked data Dleak which is read out in the leaked data readout process which is repeatedly executed on a periodic basis, or, because the controller 22 determines that the leaked data Dleak exceeds the threshold value Dth. It should be noted that, explained below is a case where the process shown in Fig. 31 is executed as a process before radiation image capturing operation, but, needless to say, the aforementioned methods and processes may be performed.

[Transition to electric charge accumulation mode and processes in electric charge accumulation mode]

Once initiation of radioactive irradiation is detected as above, the controller 22 applies the off voltage to all the lines L1 to Lx of the scanning lines 5 from the scanning drive unit 15, and moves to an electric charge accumulation mode while keeping each of the TFTs 8 in the off state. This electric charge accumulation mode means a mode in which an electric charge generated in each of the radiation detection elements 7 due to radioactive irradiation is accumulated in each of the radiation detection elements 7.

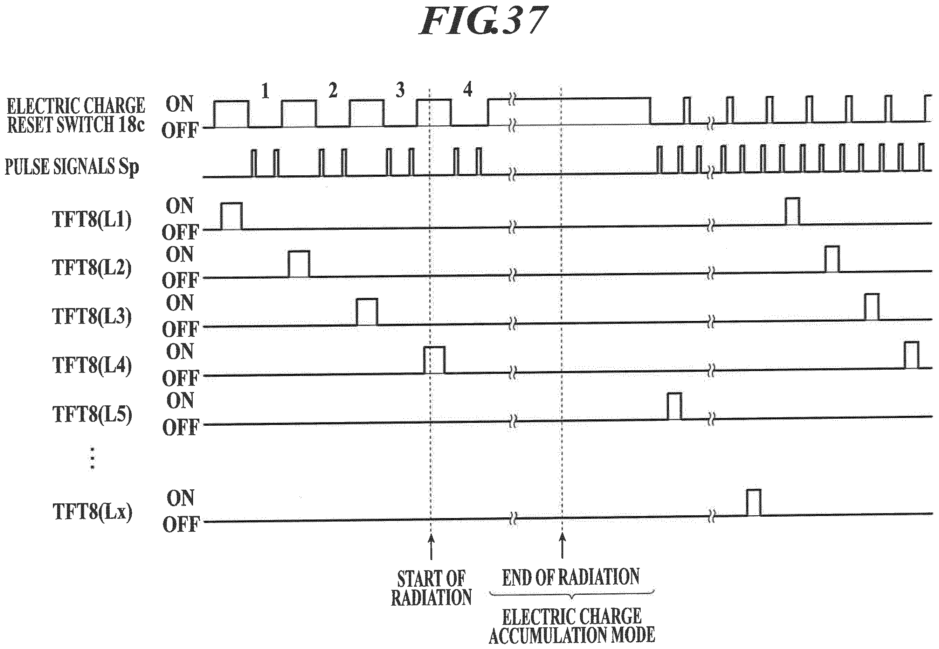

Then, as shown in Fig. 37, such construction can be adapted that, in the electric charge accumulation mode, readout operations by the reading circuits 17 are stopped and suspended for a previously-set certain period of time while the electric charge reset switch 18c of the amplifier circuit 18 in the on state, similarly to the case of the normal radiation image capturing operation.

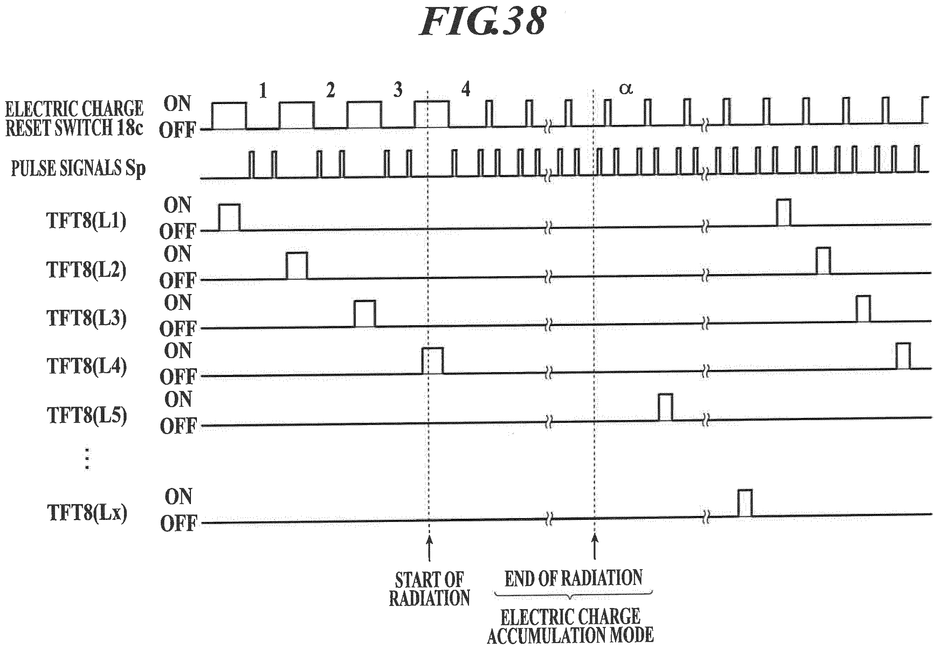

Meanwhile, as shown in Fig. 38, such construction may also be adapted that, after the controller 22 detects that radioactive irradiation is initiated in the aforementioned way, the off voltage is applied to all the lines L1 to Lx of the scanning lines 5 from the scanning drive unit 15 to move to the electric charge accumulation mode, and, at this time, the leaked data readout process is repeatedly conducted by causing the reading circuits 17 to repeatedly execute the readout operations on a periodic basis to continue monitoring of the leaked data Dleak which has been read out.

It should be noted that, when the reset process of each of the radiation detection elements 7 and the image data readout process for each of the radiation detection elements 7 are executed in the electric charge accumulation mode, a useful electric charge generated in each of the radiation detection elements 7 due to radioactive irradiation is lost, so, as shown in Fig. 38, the reset process of each of the radiation detection elements 7 and the image data readout process for each of the radiation detection elements 7 are not executed in the leaked data readout process after initiation of radioactive irradiation is detected.

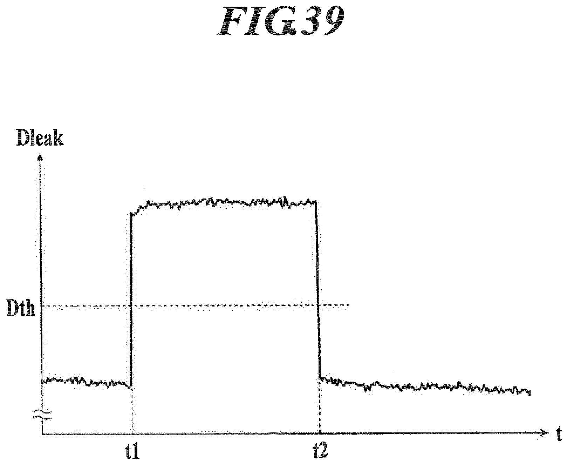

With such a construction, as shown in Fig. 39, while initiation of radioactive irradiation is detected as the leaked data Dleak read out at time t1 (in other words, the fourth round of the leaked data readout process; same as the time t1 in Fig. 15) exceeds the threshold value Dth and radiation is emitted to the radiation image capturing apparatus 1 after transition to the electric charge accumulation mode, each piece of leaked data Dleak read out in each round of the leaked data readout process after detection of initiation of radioactive irradiation (to be precise, a maximum value Dleak_max and the like among each piece of leaked data Dleak read out in each of the reading circuits 17 in each round of leaked data readout process) becomes a large value greater than the threshold value Dth.

Then, after radioactive irradiation to the radiation image capturing apparatus 1 is finished, the leaked data Dleak read out in the leakage readout process which is executed first (see $\alpha$ in Fig. 38) is reduced to a value equal to or smaller than the threshold value Dth at a time t2 when said $\alpha$th round of leaked data readout process is executed, because the amount of leakage current which flows in each of the TFTs 8 returns to the original amount at dark time due to end of radioactive irradiation.

Therefore, end of radioactive irradiation to the radiation image capturing apparatus 1 can be detected by adapting such construction that the leaked data readout process is executed periodically to monitor the leaked data Dleak which has been read out even after initiation of radioactive irradiation is detected and the electric charge accumulation mode is entered. In addition, in this case, the construction is such that end of radioactive irradiation is detected as the controller 22 determines that radioactive irradiation is ended at a point when the leaked data Dleak which has been read out becomes equal to or smaller than the threshold value Dth.

With such a construction, as shown in Fig. 38, it becomes possible to begin the image data readout process once end of radioactive irradiation is detected, thus making it possible to swiftly perform the processes after the image data readout process.

For radiation image capturing operation by using the radiation image capturing apparatus 1 in particular, it is often the case that, before generating a diagnostic radiological image by conducting full-scale image processing of the image data d in an external computer or the like, a preview image is created and displayed, and a radiological technologist or the like sees the preview image and confirms whether a subject is captured in the radiological image or whether an image of the subject is captured at an appropriate position on the radiological image.

In this case, it becomes possible to quickly judge necessity of retake and reduce a burden on a subject by retaking an image swiftly if retake is necessary, and, because of the fact that the image data readout process can be started swiftly after end of radioactive irradiation as stated above, there is a benefit that the preview image can be displayed quickly, thus enabling a radiological technologist or the like to swiftly judge necessity of retake.

Moreover, as shown in Fig. 37, when such a construction is employed that the readout operations by the reading circuits 17 are stopped and suspended for a given period of time in the electric charge accumulation mode after initiation of radioactive irradiation just like a case of normal radiation image capturing operation, there is an advantage that execution of the leaked data readout process is no longer necessary in the electric charge accumulation mode, and electric consumption of the radiation image capturing apparatus 1 can be reduced. Further, all that is needed is application of the off voltage to all the lines L1 to Lx of the scanning lines 5 and stopping the operations of the respective reading circuits 17, so there is an advantage that the control configuration becomes simple.

Note that, although Fig. 39 shows the case where the leaked data Dleak is read out by ongoingly executing the leaked data readout process after end of radioactive irradiation is detected at the time t2, this is only an experimental example to demonstrate how the leaked data Dleak changes along with radioactive irradiation, and, in reality, the leaked data readout process is stopped and the image data readout process begins immediately when end of radioactive irradiation is detected at the time t2.

[Image data readout process]

At the point when a given period of time has elapsed in the case shown in Fig. 37, and at the point when end of radioactive irradiation is detected in the case shown in Fig. 38, the controller 22 then applies the on voltage sequentially to each of the lines L1 to Lx of the scanning lines 5 from the scanning drive unit 15 as shown in Figs. 37 and 38, causes the reading circuits 17 to conduct readout operations sequentially to execute the image data readout process for reading each piece of image data d from each of the radiation detection elements 7.

In the image data readout process, as illustrated in Figs. 10 and 11, the scanning drive unit 15 and the reading circuits 17 operate, and image data d which has been read out is sequentially stored in the storage section 40 (see Fig. 7 and so on).

Note that, explained in Figs. 37 and 38 is the case where, in the image data readout process, the image data d is sequentially read out from the line L5 which follows the line L4 of the scanning lines 5 in which the reset process for each of the radiation detection elements 7 was conducted by applying the on voltage at the end of the leaked data readout process prior to radiation image capturing operation, and, after the readout process of the final line Lx of the scanning lines 5 is finished, execution of the readout process is returned to the line L1 through the line L4, but, for example, such a construction is also possible that the readout process of the image data d is executed sequentially from the first line L1 of the scanning lines 5 during the image data readout process.

[Prevention of false detection of initiation of radioactive irradiation]

Here, a process for preventing false detection of initiation of radioactive irradiation will be described. For instance, as explained as above, even if the value of the leaked data Dleak increases to a value greater than the threshold value Dth at given time t1, the leaked data Dleak may have happened to be increased for some reasons such as a large noise being mixed therein instantaneously.

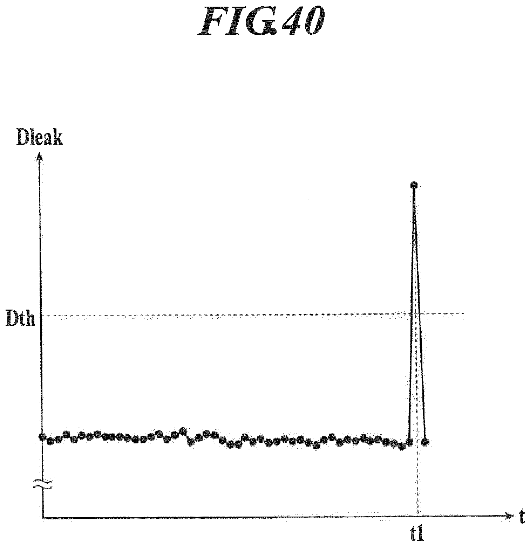

In this situation, in the case shown in Fig. 38, since the leaked data Dleak, which is read out in the leaked data readout process that follows transition to the electric charge accumulation mode, returns to the original value equal to or smaller than the threshold value Dth as shown in Fig. 40, the image data readout process is started right away. Also, in the case shown in Fig. 37, after a lapse of a given period of time from the time t1, the image data readout process is automatically started.

However, even if the image data readout process is executed in this way, only the electric charge having no information regarding the subject (in other words, a useless electric charge such as a dark electric charge) is read out from each of the radiation detection elements 7 as the image data d, and execution of the process is wasteful.

Moreover, if radiation image capturing operation is performed by emitting radiation to the radiation image capturing apparatus 1 while this kind of wasteful imaging data readout process is executed, useful image data d cannot be obtained because image data d is read out in an abnormal condition although a useful electric charge which is generated in each of the radiation detection elements 7 due to radioactive irradiation should be accumulated in each of the radiation detection elements 7. Thus, retake is required, which causes an increase of exposure dose received by the subject, imposing a burden on the subject.

Therefore, in order to prevent this kind of situation from happening and to prevent false detection of initiation of radioactive irradiation, in the case where, for example, the construction is employed that the leaked data readout process is repeatedly executed on a periodic basis to monitor the leaked data Dleak even after detection of initiation of radioactive irradiation and transition to the electric charge accumulation mode as shown in Fig. 38, when the leaked data Dleak becomes equal to or smaller than the threshold value Dth, transition to the electric charge accumulation mode may be cancelled and the state may be returned to the original state before radiation image capturing operation, said leaked data Dleak being read out in the leaked data readout process immediately after the leaked data readout process in which initiation of radioactive irradiation was detected as the leaked data Dleak that has been read out exceeded the threshold value Dth.

Namely, in the situation shown in Fig. 40, it is regarded that radioactive irradiation was not initiated and transition to the electric charge accumulation mode is cancelled, and, a state will be returned to the one before radiation image capturing operation stated above, in other words, a standby state until radioactive irradiation while the leaked data readout process is repeated periodically.

With such a construction, even if initiation of radioactive irradiation is detected by mistake because the leaked data Dleak happens to increase and exceed the threshold value Dth for some reasons such as a large noise mixed therein instantaneously, it becomes possible to accurately determine that the detection of initiation of radioactive irradiation was false and return to the standby state until radioactive irradiation while repeating the leaked data readout process periodically.

Note that, in the case shown in Fig. 37, since the false detection of initiation of radioactive irradiation causes automatic transition to the electric charge accumulation mode with the construction above, such a construction may be adapted that the leaked data readout process is continued even after detection of initiation of radioactive irradiation like the case shown in Fig. 38, and the leaked data readout process is stopped and is suspended for a given period of time at the point when it is confirmed that the detection of initiation of radioactive irradiation was not false after the leaked data Dleak that has been read out exceeds the threshold value Dth for a given number of times.

Also in this case, the construction is such that transition to the electric charge accumulation mode is cancelled and the state is returned to the original state before radiation image capturing operation, when the leaked data Dleak becomes equal to or smaller than the threshold value Dth, the leaked data Dleak being read out in the leaked data readout process immediately after the leaked data readout process in which initiation of radioactive irradiation was detected as the read out leaked data Dleak exceeded the threshold value Dth. Then, with such a construction, the effects similar to those stated earlier can be obtained.

In the case where, even when the leaked data Dleak exceeds the threshold value Dth at the time t1 due to radioactive irradiation to the radiation image capturing apparatus 1, the leaked data Dleak becomes equal to or smaller than the threshold value Dth in the next leaked data readout process for some reasons such as a large negative noise being mixed therein, the transition to the electric charge accumulation mode is cancelled and the state returns to the original state before radioactive image capture if no change is made in this construction, and this applies to both cases of the aforementioned modification examples shown in Figs. 38 and 37.

Further, the reset process of the each of the radiation detection elements 7 and the image data readout process for each of the radiation detection elements 7 are executed between the leaked data readout processes, and a useful electric charge generated in each of the radiation detection elements 7 may be lost due to radioactive irradiation during these processes.

Therefore, for example, when initiation of radioactive irradiation is detected as the leaked data Dleak exceeds the threshold value Dth and the value is reduced to the value equal to or smaller than the threshold value Dth in the next leaked data readout process as shown in Fig. 40, the construction may be such that, instead of cancelling transition to the electric charge accumulation mode promptly, the leaked data readout process is continuously executed, and transition to the electric charge accumulation mode is cancelled to return to the state before radiation image capturing operation when the read out leaked data Dleak is equal to or smaller than the threshold value Dth repeatedly in an appropriately-set plural number of rounds of the processes.

Further, in view of a relation between a period required for one round of leaked data readout process and a period of radioactive irradiation, transition to the electric charge accumulation mode is cancelled to return to the state before radiation image capturing operation when the state where the leaked data Dleak exceeds the threshold value Dth occurs continuously in a plurality of rounds of the leaked data readout processes but the leaked data Dleak returns to the previous value equal to or smaller than the threshold value Dth within a period which is sufficiently shorter than the period of radioactive irradiation and is not regarded as radioactive irradiation.

As stated above, false detection of initiation of radioactive irradiation can be adequately prevented by adapting the construction in which the leaked data readout process is executed even after initiation of radioactive irradiation is detected, and transition to the electric charge accumulation mode is cancelled to return to the state before radiation image capturing operation when the leaked data Dleak, which is read out in the leaked data readout process after the leaked data readout process where initiation of radioactive irradiation was detected, becomes a value equal to or smaller than the threshold value Dth within a period which can obviously be recognized that it is not radioactive irradiation (in other words, in a given number of rounds of the leaked data readout processes including the leaked data readout process immediately after the leaked data readout process in which initiation of radioactive irradiation was detected).

As described so far, according to the radiation image capturing apparatus 1, electric charges q leaking from the radiation detection elements 7 are read out as the leaked data Dleak through the TFTs 8 which serve as switch unit by using the reading circuits 7 provided in normal radiation image capturing apparatus 1, and initiation of radioactive irradiation is detected based on an increase of the leaked data Dleak.

Therefore, without configuring an interface with a radiation generator, it becomes possible that the radiation image capturing apparatus 1 detects at least initiation of radioactive irradiation appropriately on its own by using the characteristic of the TFTs 8 that leakage currents which flow therein increase due to radioactive irradiation.

At the same time, since it becomes possible for the radiation image capturing apparatus 1 to detect initiation of radioactive irradiation appropriately on its own without providing new means such as current detection unit, excessive power consumption due to new means such as current detection unit or superimposition of noises generated in the new means on the image data d read out from each of the radiation detection elements 7 do not happen, and a radiological image generated based on the image data d can have good image quality.

[Second embodiment]

In the aforementioned first embodiment, as stated above, respective processes were explained from the leaked data readout process before radiation image capturing operation (including the reset process of each of the radiation detection elements 7 and the image data readout process for each of the radiation detection elements 7, which are conducted during this process), the electric charge accumulation mode during radiation image capturing operation, and to the image data readout process after radiation image capturing operation.

In the second embodiment, a process for obtaining an offset correction value O will be explained, which is executed after the image data readout process in a normal radiation image capturing apparatus.

The offset correction value O is also called a dark readout value and is equivalent to an offset of the image data d, which a dark electric charge and the like which is generated and accumulated in each of the radiation detection elements 7 due to thermal excitation caused by the heat (temperature) of the radiation detection element 7 itself while each of the TFTs 8 is in the off state along with transition to the electric charge accumulation mode, and is different from the electric charge generated and accumulated in each of the radiation detection elements 7 due to radioactive irradiation. This way, this offset correction value O is read out in a state of being contained in the image data d which is read out in the image data readout process after radiation image capturing operation.

Therefore, typically, either before or after radiation image capturing operation, the radiation image capturing apparatus 1 is left without radioactive irradiation to the radiation image capturing apparatus 1 while each of the TFTs 8 is in the off state, and, thereafter, the offset correction value O is obtained from each of the radiation detection elements 7 by reading out an accumulated dark electric charge and the like from each of the radiation detection elements 7 similarly to the image data readout process, the offset correcting value O is deducted from each piece of image data d in a radiological image generating process executed in an external computer or the like, genuine image data d* derived only from electric charges generated by radioactive irradiation is calculated, and a radiological image is generated based on this genuine image data d*.

Hence, unless the offset correcting value O cannot be obtained accurately, the genuine image data d* obtained by deducting the offset correction value O from each piece of image data p is not a normal value, and a radiological image generated based thereon becomes abnormal, or the image quality thereof is deteriorated.

Thus, in this embodiment, a process for accurately obtaining the offset correction value O in the radiation image capturing apparatus 1 will be explained.

It should be noted that, explained in this embodiment is a case where the offset correction value O is obtained after radiation image capturing operation. Also, as stated above, a process for reading out the offset correction value O from each of the radiation detection elements 7 will be called an offset correction value readout process in order to discriminate the process from the image data readout process shown in Figs. 10 and 11 although both processes are executed in the similar way.

Here, assumptions for obtaining the offset correction value O will be explained.

[Assumption 1]

As stated earlier, the offset correction value O is equivalent to an electric charge (dark electric charge) generated and accumulated in each of the radiation detection elements 7 while each of the TFTs 8 is in the off state, but, to be more precise, in this embodiment and the first embodiment, the offset correction value O is equivalent to an electric charge generated and accumulated in the radiation detection element 7 during a time span from the timing when the on voltage applied to a line Ln of the scanning lines 5 is switched to the off voltage, the on voltage being applied during the reset process of each of the radiation detection elements 7 (or the image data readout process for each of the radiation detection elements 7; the same applies hereinafter) in the leaked data readout process before radiation image capturing operation, until the timing when the on voltage applied to the line Ln of the scanning lines 5 in the image data readout process after radiation image capturing operation is switched to the off voltage.

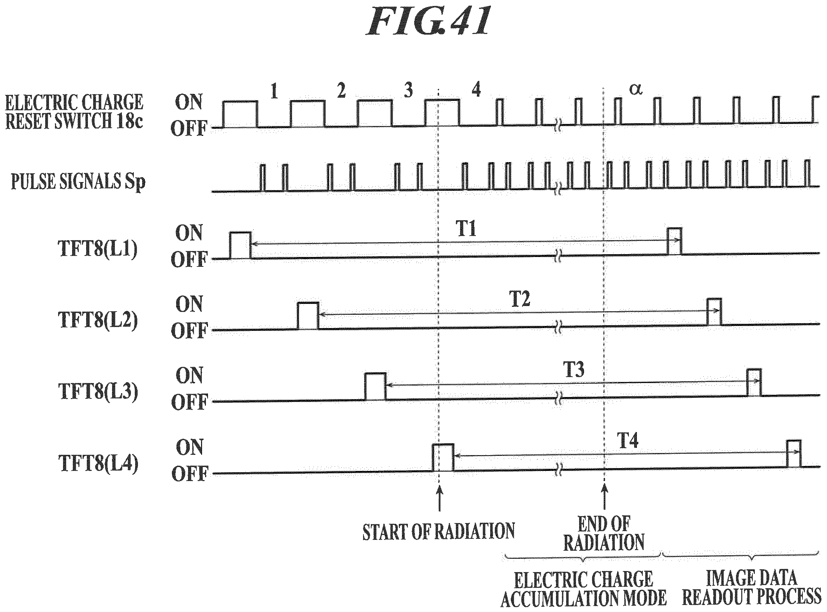

It should be noted that the aforementioned time span from the timing when the on voltage applied to a line Ln of the scanning lines 5 is switched to the off voltage until the timing when the on voltage applied to the line Ln of the scanning lines 5 in the image data readout process after radiation image capturing operation is switched to the off voltage will be referred to as a TFT 8 off period. Also, this TFT 8 off period is a time span expressed as T1 to T4 in Fig. 41 and so on described later.

[Assumption 2]

Meanwhile, the TFT 8 off period is a time span that is different in each of the lines L1 to Lx of the scanning lines 5 as expressed as T1, T2, T3 and T4 in Fig. 41 and so on, in the case where the reset process of each of the radiation detection elements 7 and the image data readout process for each of the radiation detection elements 7 are executed during the leaked data readout process before radiation image capturing operation (see Figs. 37 and 38) and in the case where the time span between transmissions of the pulse signals Sp1 and Sp2 to the correlated double sampling circuit 19 is extended in the leaked data readout process (see Fig. 29) as described in the first embodiment.

Note that Fig. 41 and so on show the case of the process explained in Fig. 38, and, although the following description will explain the process shown in Fig. 38, similar explanation also applies to the case of the processes described in Figs. 37 and 29 as a matter of course.

Although the on voltage and off voltage are also applied to each of the lines L1 to Lx of the scanning lines 5 to execute the respective processes in the case stated below similarly to the processes shown in Figs. 37 and 38, if all the lines L1 to Lx of the scanning lines 5 are depicted in the following description, the drawings will be difficult to see. Therefore, the explanation below will be provided on the assumption that each process is executed by applying the on voltage and the off voltage to each of the lines L1 to L4 of the scanning lines as shown in Fig. 41 so that the drawings is easy to see.

[Assumption 3]

From the experiment conducted by the present inventors, it is known that the offset correction value O does not necessarily increase linearly (that is, in proportion) to the TFT 8 off period. This is considered because a rate of generation of a dark electric charge generated in each of the radiation detection elements 7 is not linear with respect to the time change when the radiation image capturing apparatus 1 is left without radioactive irradiation as stated earlier. The offset correction value O becomes the same value when the TFT 8 off period is the same.

Based on the above-mentioned assumptions, a process for obtaining the offset correction value O can be constructed as the following construction examples.

[Process for obtaining offset correction value O]

[Construction example 1]

As explained in Assumption 3 above, although the offset correction value O does not increase in proportion to the TFT 8 off period, the offset correction value O becomes the same value if the TFT 8 off period is the same. Therefore, for example, such construction may be adapted so that the TFT 8 off period of each of the lines L of the scanning lines 5 becomes the same off period in both the image data readout process and the offset correction value readout process in the following way.

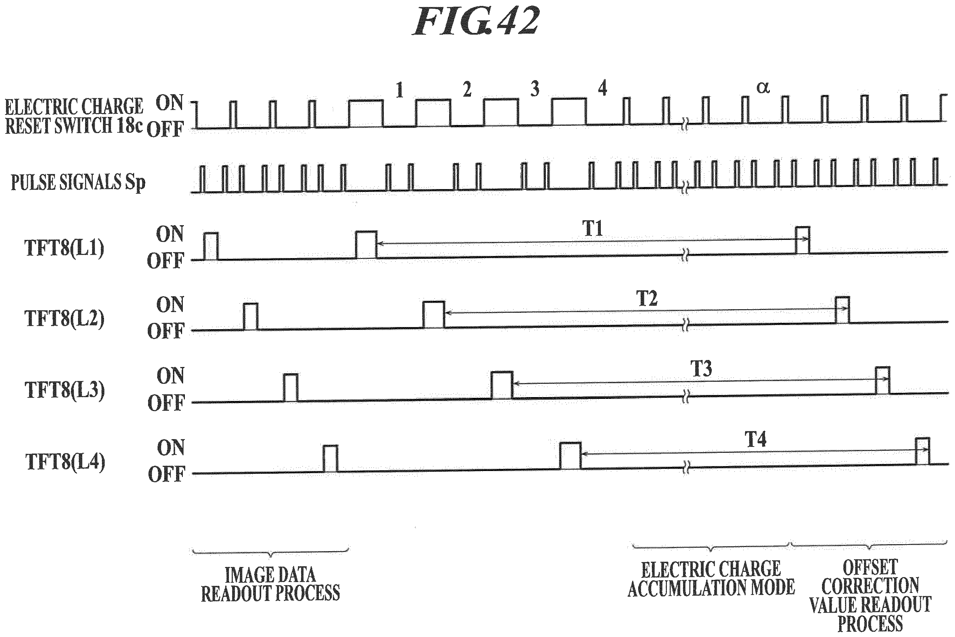

Namely, as shown in Fig. 41, after executing the leaked data readout process and the reset process for each of the radiation detection elements 7 before radiation image capturing operation, transition to the electric charge accumulation mode, and the image data readout process for each of the radiation detection elements 7 after radiation image capturing operation, the leaked data readout process and the reset process for each of the radiation detection elements 7, transition to the electric charge accumulation mode (without radiation image capturing operation), and the offset correction value readout process are executed by switching a voltage between the on voltage and the off voltage which are applied by the scanning drive unit 15 to each of the lines L1 to Lx of the scanning lines 5 simultaneously with these processes to cause the reading circuits 17 to execute the readout process sequentially, as shown in Fig. 42.

Simply speaking, the offset correction value O is read out by repeating the same process sequence as the process sequence for reading out the image data d (namely, the leaked data readout process and the like, transition to the electric charge accumulation mode, and the image data readout process), after the image readout process.

With such a construction, since the offset correction value O is read out in the same process sequence as the process sequence for reading out image data d, the TFT 8 off period when reading out the image data d and the TFT 8 off period when reading out the offset correction value O thereafter become the same time span in each one of the lines L1 to L4 of the scanning lines(in reality, the respective lines L1 to Lx of the scanning lines 5; the same applies hereinafter), even when the TFT 8 off periods T1 to T4 for the respective lines L1 to L4 of the scanning lines are different from each other as stated above.

Therefore, even if the offset correction values O are varied by the lines L1 to L4 of the scanning lines 5, the offset contained in the image data d read out in the image data readout process and the offset correction value O read out in the offset correction value readout process become the same value when looking at each of the lines L1 to L4 of the scanning lines.

Also, when looking at each of the radiation detection elements 7, the offset contained in the image data d read out from the radiation detection elements 7 in the image data readout process and the offset correction value O read out from said radiation detection element 7 in the following offset correction value readout process become the same value as well.

Therefore, by subtracting the offset correction value O read out in the offset correction value readout process from each piece of image data d read out in the image readout process during the radiological image generating process, it becomes possible to accurately calculate the genuine image data d*, which is derived only from an electric charge generated by radioactive irradiation, for each of the radiation detection elements 7. Then, it becomes possible to accurately generate a radiological image based on this genuine image data d*.

With this construction, in a case where no more imaging will be performed after the image data d read out from each of the radiation detection elements 7 in the image data readout process is sequentially stored in the storage section 40 by the controller 22 of the radiation image capturing apparatus 1 (see Fig. 7 and so on), the offset correction value readout process is executed by automatically repeating the same process sequence and the offset correction value O that has been read out is stored in the storage section 40 sequentially.

Thereafter, each piece of the image data d and each of the offset correction values O are sequentially read out from the storage section 40 at appropriate timing, and such data is transmitted through the antenna device 39 (see Figs. 1 and 7 and so on) to an external computer or the like in which image processing is conducted.

Note that Figs. 41 and 42 explained the case where initiation of radioactive irradiation is detected based on the leaked data Dleak which is read out in the leaked data readout process immediately after the reset process of each of the radiation detection elements 8 ("4" in Fig. 41 or the fourth round of the leaked data readout process) as the on voltage is applied to the final line L4 of the scanning lines 5, and, regarding the image data readout process, a case was explained where the read out process of the image data d was executed from the first line L1 of the scanning lines 5.

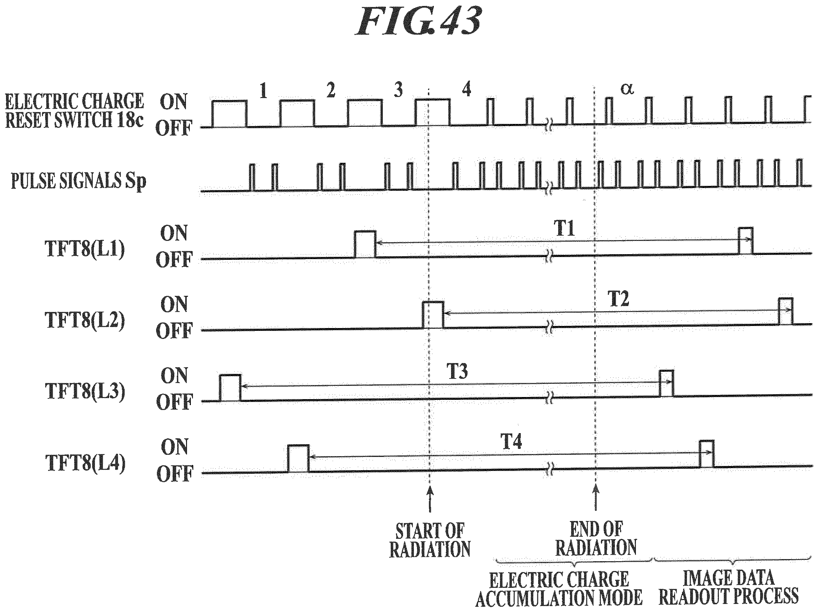

Here, as shown in Fig. 43, when, for example, initiation of radioactive irradiation is detected based on the leaked data Dleak which is read out in the leaked data readout process immediately after the reset process of each of the radiation detection elements 8 as the on voltage is applied to the line L2 in the middle of the scanning lines 5, the construction may be such that the readout process of the image data d is executed from the next line L3 of the scanning lines 5 during the image data readout process as stated above. Note that this is the case where the process is executed similarly to the case shown in Fig. 38.

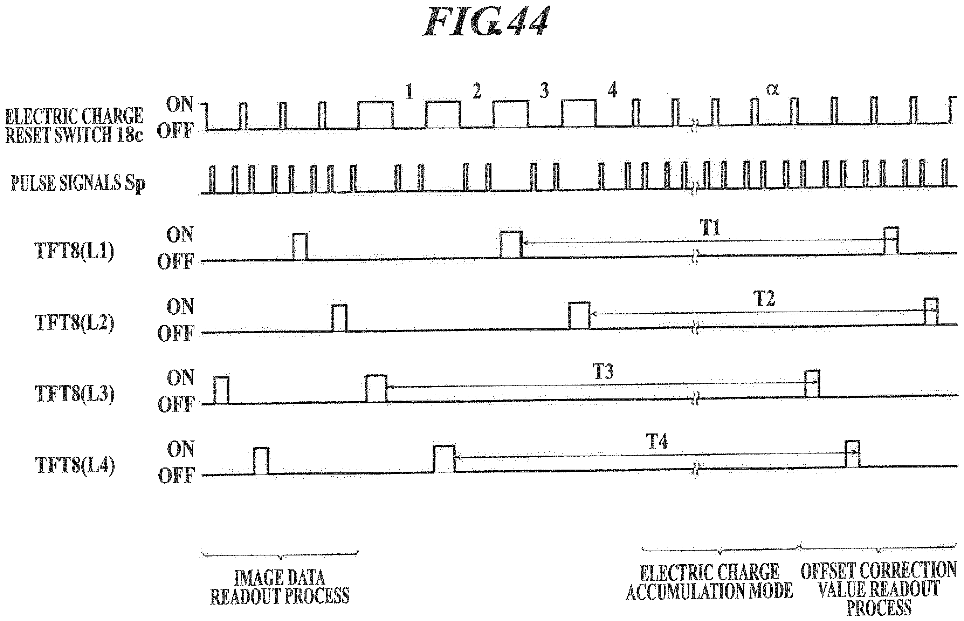

In such a case, in this [Construction example 1], as shown in Fig. 44, after the image data readout process for each of the radiation detection elements 7 is executed following radiation image capturing operation, the leaked data readout process and the reset process of each of the radiation detection elements 7, transition to the electric charge accumulation mode (without radioactive irradiation), and the offset correction value readout process are executed by switching the voltage between the on voltage and the off voltage applied by the scanning drive unit 15 to each of the lines L1 to Lx of the scanning lines 5 at the same timing with each of the processes executed until the image data readout process for each of the radiation detection elements 7 after radiation image capturing operation, thus causing the reading circuits 17 to perform the readout operations sequentially.

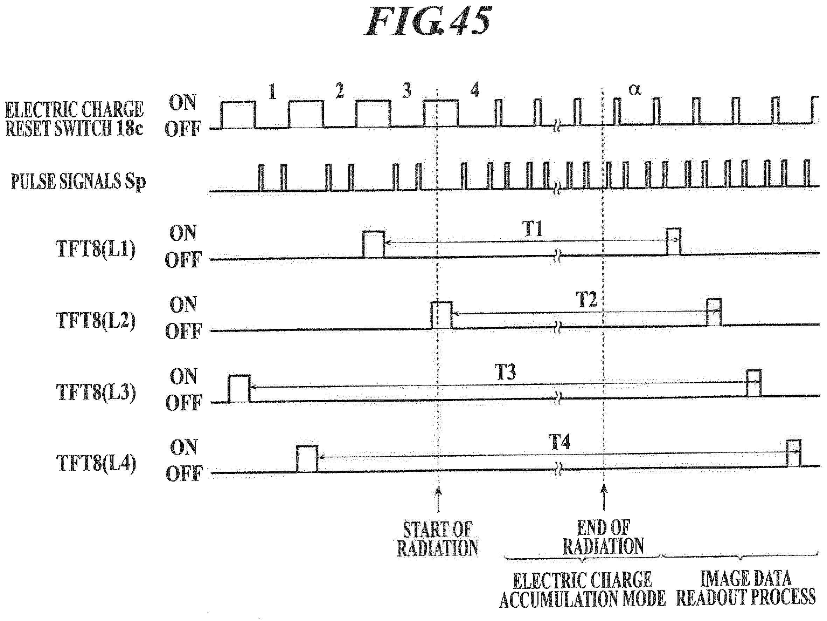

Further, as stated earlier, in this case, even when, for example, initiation of radioactive irradiation is detected based on the leaked data Dleak which is read out in the leaked data readout process immediately after the reset process of each of the radiation detection elements 8 as the on voltage is applied to the line L2 in the middle of the scanning lines 5 as shown in Fig. 45, the construction may be such that the readout process of the image data d is executed from the first line L1 of the scanning lines 5 in the image data readout process. In this case, the values of the TFT 8 off periods T1 to T4 are somewhat significantly different among the respective lines L1 to L4 of the scanning lines 5, and, in particular, the TFT 8 off periods T2 and T3 of the lines L2 and L3 of the scanning lines 5 which are next to each other on the detecting section P are considerably different from each other.

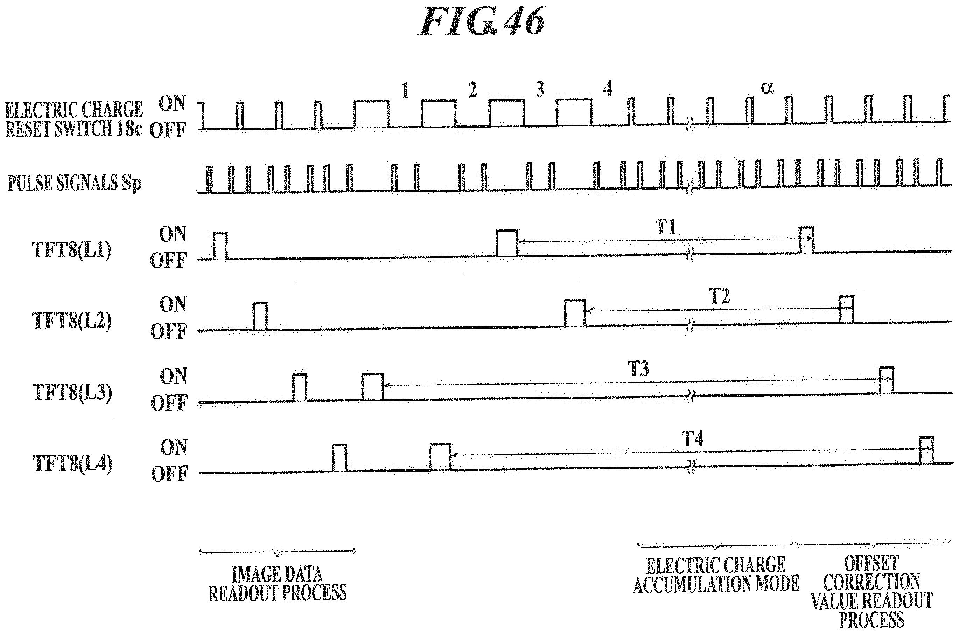

However, in this case, as shown in Fig. 46, after the image data readout process for each of the radiation detection elements 7 is executed following radiation image capturing operation, the leaked data readout process and the reset process for each of the radiation detection elements 7, transition to the electric charge accumulation mode (without radioactive irradiation), and the offset correction value readout process are executed by switching the voltage between the on voltage and the off voltage applied by the scanning drive unit 15 to each of the lines L1 to Lx of the scanning lines 5, at the same timing with each of the processes executed until the image data readout process for each of the radiation detection elements 7 following radiation image capturing operation, thus causing the reading circuit 17 to perform the readout operation sequentially.

As explained above, with both of the construction shown in Fig. 43 and the construction shown in Fig. 45, the TFT 8 off period when reading out the image data d and the TFT 8 off period when reading out the offset correction value thereafter become the same time span when looking at each of the lines L1 to L4 by constructing each of the processes after the image data readout process as shown in Figs. 44 and 46, even when the TFT 8 off periods T1 to T4 are different from each other in each of the lines L1 to L4 of the scanning lines (each of the lines L1 to Lx of the scanning lines 5 in reality; the same applies hereinafter).

Therefore, the offset contained in the image data d read out in the image data readout process, and the offset correction value O read out in the offset correction value readout process become the same value when looking at each of the lines L1 to L4 of the scanning lines, and, the offset contained in the image data d read out from each of the radiation detection elements 7 in the image data readout process and the offset correction value O read out from each of the radiation detection elements 7 in the offset correction value readout process thereafter become the same value when looking at each of the radiation detection elements 7.

Hence, by deducting the offset correction value O which was read out in the offset correction value readout process from each piece of image data d which was read out in the image readout process during the radiological image generating process, it becomes possible to accurately calculate the genuine image data d*, which is derived only from an electric charge generated by radioactive irradiation, for each of the radiation detection elements 7. Then, it becomes possible to accurately generate a radiological image based on this genuine image data d*.

It should be noted that, in the following [Construction example 2] and [Construction example 3], the constructions shown in Figs. 43 and 45 can be employed, and, with such constructions, similar effects to those described below can be obtained as well. Therefore, in the following [Construction example 2] and [Construction example 3], explanation will be omitted for the case where the constructions shown in Figs. 43 and 45 are adapted.

[Construction example 2]

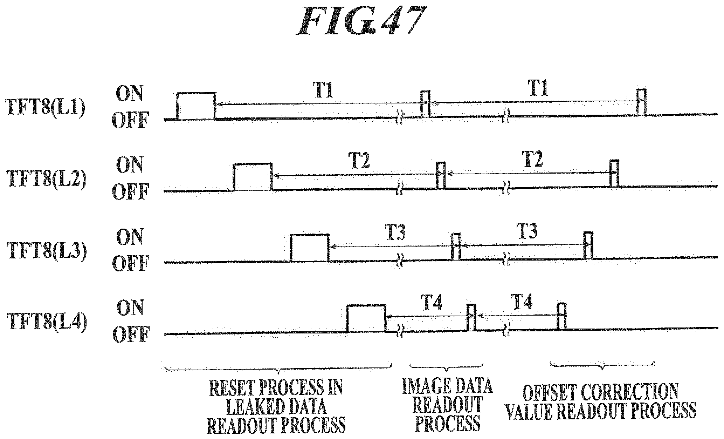

Also, although illustration of the electric charge reset switch 18c and the pulse signals Sp is omitted, such construction is possible that, for example, the offset correction value readout process is executed for each of the lines L1 to L4 of the scanning lines 4 after the image readout process is finished and without radioactive irradiation, at such timing that the TFT 8 off periods schematically shown in Fig. 47 become equal to the TFT 8 off periods T1 to T4 shown in Fig. 4, respectively, and the TFT 8 off periods schematically shown in Fig. 47 is between the timing when the on voltage applied to each of the lines L1 to L4 of the scanning lines 5 in the image data readout process is switched to the off voltage and the timing when the on voltage applied to the scanning lines 5 in the offset correction value readout process is switched to the off voltage.

In other words, simply speaking, the offset correction value readout process is executed for the lines L1 to L4 of the scanning lines 5, respectively, so that the time spans from the reset process of each of the radiation detection elements 7 before radiation image capturing operation until the image data readout process (in other words, the TFT 8 off periods T1 to T4) are equal to the time spans from the image data readout process until the offset correction value readout process (off periods).

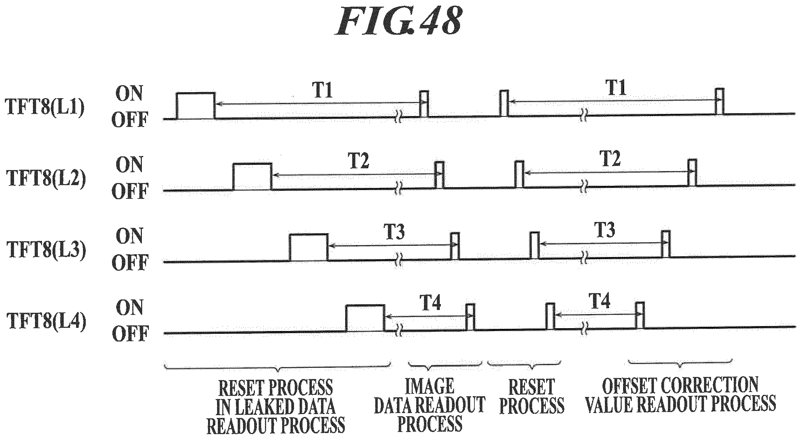

Further, as schematically shown in Fig. 48, such construction is also possible that the reset process of each of the radiation detection elements 7 is executed after the image data readout process is ended, and, thereafter, the offset correction value readout process is executed so that the time span from this reset process of each of the radiation detection elements 7 until the offset correction value readout process is equal to the time span from the reset process of each of the radiation detection elements 7 before radiation image capturing operation until the image data readout process.

With such a construction, since the TFT 8 off periods T1 to T4 during the image data readout process and the TFT 8 off periods T1 to T4 during the offset correction value readout process become the same time span, the offset contained in the image data d read out in the image data readout process and the offset correction value O read out in the offset correction value readout process become the same value in each of the radiation detection elements 7 like the foregoing.

Therefore, by deducting the offset correction value O readout in the offset correction value readout process from each piece of the image data d readout in the image readout process during the radiological image generating process, the genuine image data d* which is derived only from electric charges generated by radioactive irradiation can be calculated accurately for each of the radiation detection elements 7. Thus, a radiological image can be generated appropriately based on this genuine image data d*.

[Construction Example 3]

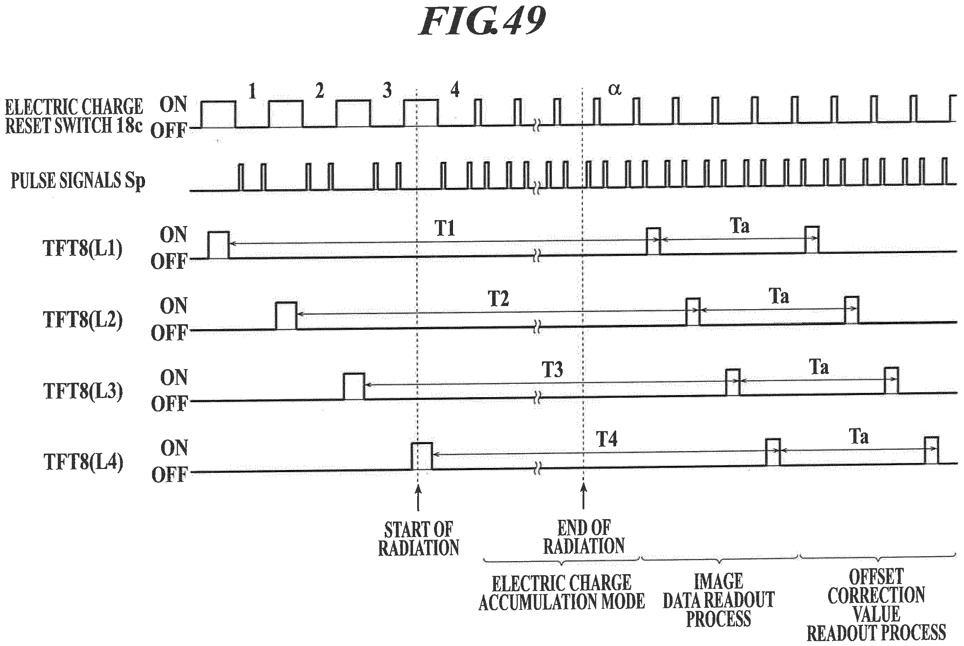

Meanwhile, as shown in Fig. 49, such construction is also possible that the offset correction value readout process is executed by sequentially applying the on voltage to each of the lines L1 to L4 of the scanning lines 5 from the scanning drive unit 15 at the same timing with the image data readout process, immediately after the image data readout process is finished or after lapse of a given period of time, in a state where no radiation is emitted. It should be noted that, like the case shown in Fig. 48, such construction is also possible that the reset process of each of the radiation detection elements 7 is executed after the image data readout process is ended, and thereafter, the offset correction value readout process is conducted.

In this case, the time spans from the image data readout process until the offset correction value readout process (in other words, the TFT8 off periods) become the same time span Ta in all of the lines L1 to L4 of the scanning lines 5.

However, in this case, since the TFT 8 off periods T1 to T4 of the respective lines L1 to L4 of the scanning lines 5 between the reset process in the leaked data readout process before radiation image capturing operation and the image data readout process are not equal to the time span Ta between the image data readout process and the offset correction value readout process, the offset contained in the image data d read out in the image data readout process and the offset correction value O read out in the offset correction value readout process do not become the same value when looking at each of the lines L1 to L4 of the scanning lines.

Thus, even if the offset correction value O read out in the offset correction value readout process is deducted from each piece of the image data d read out in the image data readout process, genuine image data d* cannot be calculated accurately. This means that the value obtained becomes a value that is different from the original genuine image data d*.

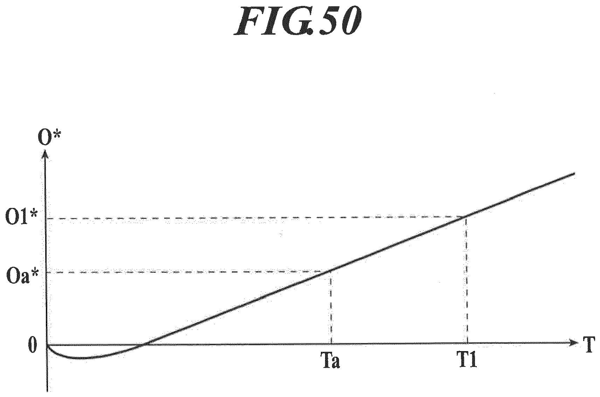

Therefore, in the case of this Construction example 3, a table or a relational expression which expresses a relation between the TFT 8 off period T and the reference offset correction value O* is experimentally obtained in advance as shown in Fig. 50, and the table or the relational expression is held in advance in an external computer or the like which performs image processing based on the image data d and the offset correction value O transmitted from the radiation image capturing apparatus 1. In this case, the experiment was undertaken in a state where, for example, temperatures and the like of the respective functional parts, the substrate 4 and so on are stabled by energizing the respective functional parts of the radiation image capturing apparatus 1 including the reading circuits 17 for a long period of time.

Then, for example, when calculating an offset (hereinafter referred to as an offset O1) contained in the image data d which is read out from each of the radiation detection elements 7 connected to the line L1 of the scanning lines 5 in the image data readout process, the computer or the like first reads out or calculates a reference offset correction value O1* (see Fig. 50) that corresponds to the off period T1 with reference to the above-mentioned table or according to the aforementioned relational expression.

However, since there are differences between the imaging conditions such as temperature of the reading circuit 17 when the table or the relational expression shown in Fig. 50 is evaluated, and the imaging conditions for the actual radiation image capturing operation, the reference offset correction value O1* read out or calculated as above cannot be used as the aforementioned offset O1 as it is.

Hence, for example, the reference offset correction value Oa* (see Fig. 50) in the off period Ta is obtained based on the foregoing table or the relational expression, and, by utilizing the fact that the ratio of the reference offset correction value O1* to the above-mentioned offset O1 is equal to the ratio of the reference offset correction ratio Oa* to the offset correction value O read out in the offset value readout process, that is to say:

$$O1^* : O1 = Oa^* : O \quad \dots(1)$$

the above mentioned offset O1 is calculated from the read-out offset correction value O according to the following expression (2) derived from the following expression (1).

$$O1 = O \times O1^* / Oa^* \quad \dots(2)$$

Then, by deducting the offset O1 calculated according to the above expression (2) from each piece of the image data d read out in the image data readout process, it becomes possible to accurately calculate the genuine image data d* which is derived only from electric charges generated by radioactive irradiation for each of the radiation detection elements 7.

Further, the similar process is carried out for the lines L2 to L4 of the scanning lines 5, and the genuine image data d* which is derived only from electric charges generated by radioactive irradiation is calculated for each of the radiation detection elements 7 accurately by calculating the offsets (in other words, offsets 02 to 04) contained in the image data d read out in the image data readout process for each of the radiation detection elements 7 that are connected to the lines L2 to L4 of the scanning lines 5, and by deducting the calculated offsets 02 to 04 from respective pieces of the image data d read out in the image data readout process.

Thus, by having such a construction, a radiological image can also be appropriately generated based on the calculated genuine image data d* in the case of Construction example 3.

Explained in the respective construction examples described above was the case where each of the processes for obtaining the offset correction value O including the offset correction value readout process is executed only once after the image data readout process, but such a construction may also be possible that, for example, the processes for obtaining the offset correction value O are executed more than once, an average of the offset correction values O obtained from the respective processes is figured out for each of the radiation detection elements 7, and the average value is used as the offset correction value O of each of the radiation detection elements 7.

[Third Embodiment]

In the second embodiment described above, various construction examples were explained regarding the case where the offset correction value O is obtained, the offset correction value O being derived only from dark electric charges or the like which are generated and accumulated in each of the radiation detection elements 7 while each of the TFTs 8 is in the off state, and the offset correction value O being generated by thermal excitation due to heat (temperature) of the radiation detection element 7 itself.

In the radiation image capturing apparatus 1 according the present invention, before radiation image capturing operation, in other words, before radioactive irradiation to the radiation image capturing apparatus 1 is initiated as stated earlier, the leaked data readout process is executed by driving the reading circuit 17, the scanning drive unit 25 and so on at timing of the on/off operation same as the case of the image data readout process (Fig. 10) as shown in Fig. 12, or the leaked data readout process is executed by transmitting the pulse signals Sp1 and Sp2 to the correlated double sampling circuit 19 from the controller 22 so that the time span between the transmissions of the pulse signals Sp1 and Sp2 becomes longer than the time span of the same in the case of the image data readout process as shown in Fig. 29.

Further, as shown in Figs. 19, 20, and 29, the reset process of each of the radiation detection elements 7 and the process for reading out the image data d from each of the radiation detection elements 7 are executed between the leaked data readout process and the next leaked data readout process when the leaked data readout process is repeated periodically before radiation image capturing operation.

Meanwhile, in the respective embodiments stated above, in the image data readout process after radiation image capturing operation, the read out process is executed at similar timing as the image data readout process under normal circumstances.

Further, in a case where initiation of radioactive irradiation is detected in the next leaked data readout process after the reset process was conducted by applying the on voltage to the line Ln of the scanning line 5, in the image data readout process after radiation image capturing operation, the process for reading out the image data d from each of the radiation detection elements 7 is executed by sequentially applying the on voltage to the next line Ln+1 and on (see Figs. 37, 38, 43 and so on), or the process for reading out the image data d from each of the radiation detection elements 7 is executed by applying the on voltage sequentially to the first line L1 and on of the scanning lines 5 (see Fig. 45 and so on).

Therefore, the TFT 8 off periods T1 to T4 are different from each other among the lines L1 to L4 of the scanning lines 5, the TFT 8 off periods T1 to T4 being from the timing when each of the TFTs 8 is turned to the off state from the on state in the reset process and the like which is executed in the leaked data readout process before radiation image capturing operation, until the timing when each of the TFTs 8 is turned to the off state from the on state in the image data readout process.

Thus, in [Construction example 1] and [Construction example 2] in the foregoing second embodiment, the construction was such that, although the TFT 8 off periods T1 to T4 until the image data readout process are different from each other among the lines L1 to L4 of the scanning lines, the TFT 8 off period until the image data read out process is equal to the following TFT 8 off period until the offset correction value readout process when looking at each of the lines L1 to L4 of the scanning lines, and the offset correction value O is read out in the offset correction value readout process, the offset correction value O being the same value as the offset derived from a dark electric charge and the like contained in the image data d read out in the image data readout process.

In addition, in [Construction example 3] of the foregoing second embodiment, the construction was such that the offset correction value readout process is executed by sequentially applying the on voltage to each of the lines L1 to L4 of the scanning lines 5 from the scanning drive unit 15 at the same timing with the image data readout process immediately after the image data readout process is finished or after lapse of a given period of time, and the offset O1 contained in the image data d read out in the image data readout process is calculated from the offset correction value O which is read out in the offset correction value readout process in the following arithmetic processing.

Incidentally, according to the study by the present inventors, it is known that, if the offset correction value O is read out in the aforementioned way after the readout process for the image data d from each of the radiation detection elements 7 is executed, a different offset due to a so-called lag is read out in addition to the foregoing offset which is derived from dark electric charges generated by thermal excitation or the like due to heat (temperature) of the radiation detection element 7 itself, when strong radiation is emitted to the radiation image capturing apparatus 1.

Then, the offset derived from dark electric charges and the like is removed relatively easily by, for example, repeating the reset process for each of the radiation detection elements 7, but it is known that the offset due to a lag has a characteristic that it is not removed easily even if the reset process for each of the radiation detection elements 7 is repeatedly executed.

This means that the offset derived from dark electric charges and the like is decreased to a value close to zero relatively quickly as the reset process for each of the radiation detection elements 7 is repeated. However, the offset due to a lag is hard to remove even if the reset process for each of the radiation detection elements 7 is repeatedly executed, and, even if the reset process is repeated, when the offset correction value readout process is executed after the radioactive image data 1 is left without radioactive irradiation, the offset correction value O is read out which is greater than the value obtained when there is only the offset derived from dark electric charges.

The reason why the offset due to a lag cannot be removed easily even if the reset process for each of radiation detection elements is repeated is considered to be because some electrons and holes generated in the radiation detection elements 7 due to strong radiation move to a sort of metastable energy level (metastable state), and the electrons and holes lose mobility in the radiation detection element 7 for a relatively long time.

Then, due to heat energy, the electrons and holes in this metastable energy state move to a conduction band having an energy level which is considered to be higher than this metastable energy with a certain probability, and the mobility thereof is restored. This is considered the reason why the offset due to a lag is not removed easily even if, for example, the reset process of each of the radiation detection elements 7 is repeated after radiation image capturing operation, and the offset due to a lag is superimposed on the offset derived from a dark electric charge and so on in the offset correction value readout process after radiation image capturing operation and read out as the offset correction value O. Hereinafter, this offset due to a lag will be expressed as Olag.

This offset Olag due to a lag is generated not only when strong radiation is emitted, but also when a normal dose of radiation including weak radiation is emitted. Having said that, when radiation that is not very strong is emitted, the percentage of the offset Olag due to a lag contained in the offset correction value O is often small enough to be ignored.

A dose of radiation emitted which increases the offset due to a lag to a non-ignorable level depends on the performance and the like of the radiation detection elements 7 such as photodiodes used in the radiation image capturing apparatus 1. Therefore, the level of radiation dose is appropriately decided for each radiation image capturing apparatus 1 for determining whether the method of the third embodiment described below should be used. Further, such construction is also possible that the image data readout process and the offset correction value readout process are always executed in the method of the third embodiment.

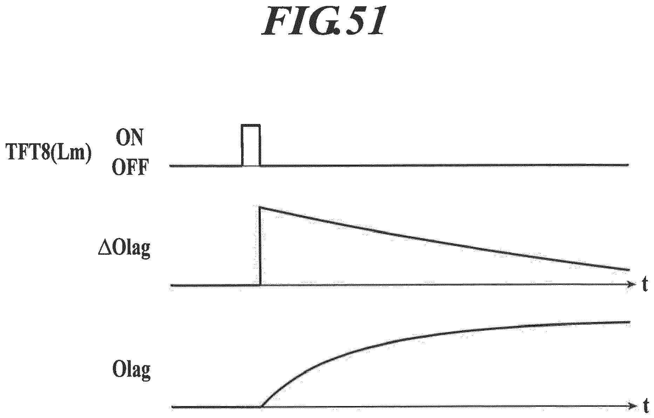

Meanwhile, according to the study by the present inventors, in the image data readout process after radiation is emitted to the radiation image capturing apparatus 1, when the on voltage is sequentially applied to each of the lines Ln of the scanning lines 5 to read out the image data d as shown in Fig. 51, the offset Olag due to a lag is generated immediately after the on voltage applied to each of the lines Ln of the scanning lines 5 is switched to the off voltage.

Then, when the offset Olag due to a lag generated per unit time is expressed as ΔOlag, this offset ΔOlag due to a lag per unit time reaches the largest value at a point when the voltage applied to each of the lines Ln of the scanning lines 5 is switched from the on voltage to the off voltage, and then is decreased gradually after that, as shown in Fig. 51. Therefore, the offset Olag due to a lag, which can be expressed as an integral value per unit time of the offset ΔOlag due to a lag per unit time, becomes a value which increases over time as shown in Fig. 51.

Further, since the offset Olag due to a lag increases over time in this manner, the following problem happens.

As explained earlier, the image data d read out in the image data readout process after radiation image capturing operation includes the genuine image data d* derived from an electric charge generated in each of the radiation detection elements 7 due to radioactive irradiation, and an offset caused by a dark electric charge and so on (hereinafter referred to as Od). Therefore, the following relation holds.

$$d = d^* + Od \ldots (3)$$

In addition, the offset correction value O read out in the offset correction value readout process includes the offset Od derived from a dark electric charge and the like, and the offset Olag due to a lag. Therefore, the following relation holds.

$$O = Od + Olag \ldots (4)$$

Therefore, where the offset correction value O is deducted from the image data d according to normal image processing method, the offset Od derived of a dark electric charge and the like is balanced out, resulting in the following equation:

$$d - O = (d^* + Od) - (Od + Olag)$$

$$\therefore d - O = d^* - Olag \quad \ldots(5)$$

Now, consider the case where strong radiation is emitted to the radiation image capturing apparatus 1 evenly, in other words, a same dose of strong radiation is emitted to a front face of the radiation entrance face R (see Fig. 1). In this case, the data that is finally obtained from each of the radiation detection elements 7 should be the same. It should be noted that, in this case, abnormality of radiation detection elements 7 and the offset of each of the reading circuits 17 are not taken into consideration.

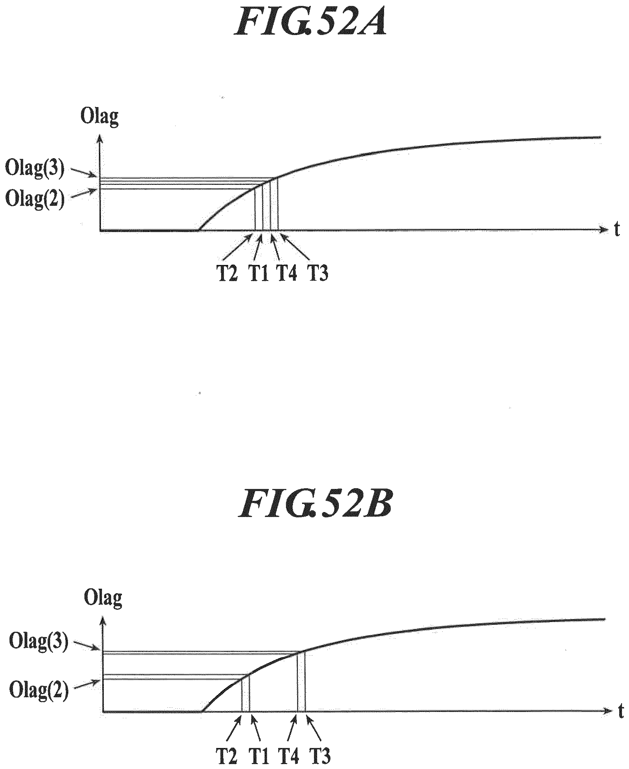

In this case, the genuine image data d* derived from an electric charge which is generated in each of the radiation detection elements 7 due to radioactive irradiation become the same value. However, when each of the processes is executed, for example, as shown in Figs. 43 and 44, the values of the offsets Olag(1) to Olag(4) caused by lags for the respective lines L1 to L4 of the scanning lines 5 are different from each other as shown in Fig. 52A because the TFT 8 off periods T1 to T4 are different from each other among the respective lines L1 to L4 of the scanning lines 5.

Therefore, when the process is executed to deduct the offset correction value O from the image data d as stated earlier, although the value of d* is the same in the equation (5) above, the value of Olag is varied among the respective lines L1 to L4 of the scanning lines 5, so the value d - O calculated by deducting the offset correction value O from the image data d is also varied among the respective lines L1 to L4 of the scanning lines 5.

Moreover, if each of the processes is executed as shown in Figs. 43 and 44, the TFT 8 off period T2 of the line L2 of the scanning lines 5 becomes the shortest, and the TFT 8 off period T3 of the neighboring line L3 of the scanning lines 5 becomes the longest amongst the respective lines L1 to 14 of the scanning lines 5. Therefore, as shown in Fig. 52A, among the offsets Olag(1) to Olag(4) caused by lags, the offset Olag(2) caused by a lag becomes the smallest value, and the offset Olag(3) caused by a lag becomes the largest value.

Thus, when a radiological image is generated based on the calculated value d - O, although the entire region of a radiological image should have the same lightness (brightness) as the image was captured by emitting strong radiation equally to the radiation image capturing apparatus 1, the lightness of the radiological image is slightly different in different regions of the image, and, in addition, the lightness becomes uneven at positions corresponding to the lines L2 and L3 of the scanning lines 5, respectively, on the radiological image.

In a case where each of the processes is executed as shown in Figs. 45 and 46, since the TFT 8 off periods T2 and T3 are more significantly different from each other between the line L2 of the scanning lines 5 where the off period is the shortest and the line L3 of the scanning lines 5 where the off period is the longest, a difference between the offsets Olag(2) and Olag(3) becomes larger as shown in Fig. 52B, thus causing the aforementioned phenomena to happen in more remarkable manner.

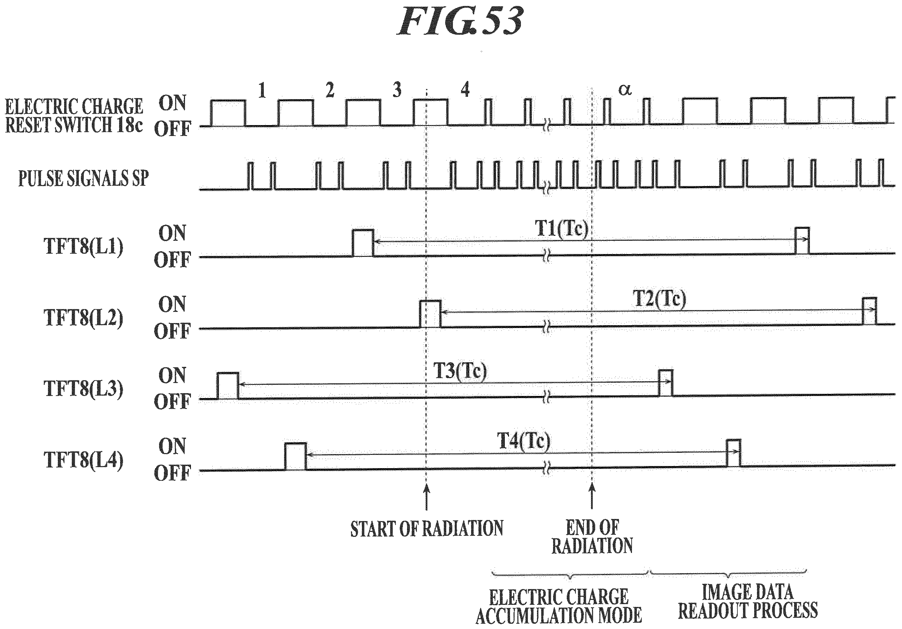

Thus, in this embodiment, as one of the ways to prevent this, for example, such construction can be applied that it is possible to change the timing for sequentially applying the on voltage to each of the lines L1 to L4 of the scanning lines 5 (which is same in the case of the lines L1 to Lx of the scanning lines 5; the same applies hereinafter) in the image data readout process after radiation image capturing operation so that the TFT 8 off periods T1 to T4 become the same time span Tc in all the lines L1 to L4 of the scanning lines 5, as shown in Fig. 53.

With such a construction, all of the TFT 8 off periods T1 to T4 before and after the image data readout process become the same time span Tc, in the case where the process sequence in reading out the image data d are equalized to the process sequence until the offset correction value O is read out after the image data readout process as stated in [Construction example 1] in the second embodiment above, and in the case where the offset correction readout process is executed so that the TFT 8 off periods T1 to T4 until the image data readout process are equalized to the TFT 8 off periods T1 to T4 until the offset correction value readout process in each of the lines L1 to L4 of the scanning lines 5 as stated in [Construction 2].

Therefore, like the foregoing examples, when strong radiation is emitted evenly to the radiation image capturing apparatus 1, all the offsets Olag(1) to Olag(4) due to lags become the same value as evident from Figs. 51, 52A and 52B. Moreover, since the genuine image data d* derived from an electric charge which is generated in each of the radiation detection elements 7 due to radioactive irradiation becomes the same value, the value d - O calculated according to the equation (5) above becomes the same value in all the lines L1 to 14 of the scanning lines 5.

Therefore, if a radiological image is generated based on the calculated value d - O, the entire region of the radiological image has the same lightness when imaging is conducted by emitting strong radiation evenly to the radiation image capturing apparatus 1. This way, above-mentioned uneven lightness on the radiological image can be prevented.

It should be noted that, in this case, if the construction is such that the readout process of the image data d is executed from the first line L1 of the scanning lines 5 in the image data readout process when initiation of radioactive irradiation is detected based on the leaked data Dleak read out in the leaked data readout process immediately after the reset process executed for each of the radiation detection elements 8 as the on voltage is applied to the line L2 in the middle of the scanning lines 5 as shown in Fig. 45, the TFT 8 off periods T1 to T4 cannot be the same time span Tc for each of the lines L1 to L4 of the scanning lines 5 as stated above.

Therefore, when a construction is employed so that the TFT 8 off periods T1 to T4 for the respective lines L1 to L4 of the scanning lines 5 become the same time span Tc, if, for example, initiation of radioactive irradiation is detected based on the leaked data Dleak which is read out in the leaked data readout process immediately after the reset process is executed for the each of the radiation detection elements 8 as the on voltage is applied to the line L2 in the middle of the scanning lines 5 as shown in Fig. 43, the readout process of the image data d is executed from the next line L3 of the scanning lines 5.

Further, in the case of [Construction example 3] in the aforementioned second embodiment, effects similar to the foregoing can be achieved by equalizing the time span Ta from the image data readout process until the offset correction value readout process to the aforementioned time span Tc. Moreover, in this case, since all of the TFT 8 off periods T1 to T4 before and after the image data readout process become the same time span Tc, it is no longer necessary to calculate the offset Od (O1 in the equation) caused by a dark electric charge in accordance with the equation (2) stated above based on the foregoing table or the relational expression.

It should be noted that, it is often the case that the offset Olag due to a lag causes a problem when strong radiation is emitted but does not cause problems when small or normal dose of radiation is emitted.

Accordingly, such construction is possible that the timing for applying the on voltage or the off voltage to each of the lines L1 to Lx of the scanning lines 5 in the image data readout process after radiation image capturing operation is switched between a normal timing mode (the case of the second embodiment) and the variable timing mode (the case of the third embodiment) depending on the dose of radiation emitted to the radiation image capturing apparatus 1.

With such a construction, when timing is changed for sequentially applying the on voltage to each of the lines L1 to Lx of the scanning lines 5 in the image data readout process after radiation image capturing operation like this embodiment, time required for each of the processes in the radiation image capturing apparatus 1 becomes slightly longer than the case of the normal timing, but, when weak or normal dose of radiation is emitted, it becomes possible to prevent the time required for such processes from being extended, by executing the image data readout process at normal timing.

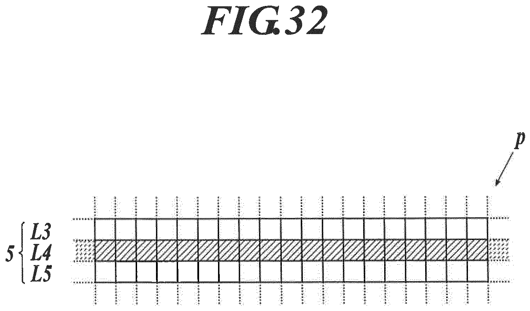

It should be noted that, in the aforementioned embodiments, the case was explained where the image data d is regarded invalid as shown in Figs. 31 and 32, the image data d being read out from each of the radiation detection elements 7 connected to the line L4 of the scanning lines 5 to which the on voltage is applied in the reset process immediately before the fourth round of the leaked data readout process in which initiation of radioactive irradiation is detected based on the leaked data Dleak, and, the image data d is calculated by performing linear interpolation using surrounding pieces of image data d.

However, such construction may be employed that, without making such image data d invalid, the image data d is restored by modifying the image data d.

## INDUSTRIAL APPLICABILITY

The present invention is applicable in the fields in which radiation image capturing operation is conducted (especially in medical fields).

## DESCRIPTION OF THE NUMERALS

| | |
|---|---|
| 1 | Radiation image capturing apparatus |
| 3 | Scintillator |
| 5, L1 to Lx | Scanning lines |
| 6 | Signal lines |
| 7 | Radiation detection elements |
| 8 | TFT (Switch unit) |
| 15 | Scanning drive unit |
| 16 | Reading IC |
| 17 | Reading circuit |
| 18 | Amplifier circuit |

| | |
|---|---|
| 18a | Operation amplifier |
| 18b, C1 to C4 | Capacitors |
| 19 | Correlated double sampling circuit |
| 22 | Controller |
| 85 | Wire |
| cf | Capacitance |
| d | Image data |
| Dleak | Leaked data |
| Dth | Threshold value |
| O | Offset correction value |
| P | Detecting section |
| q | Electric charge |
| r | Region |
| T1 to T4 | TFT off periods (time spans) |
| Tc | Same time span |
| V | Voltage value |
| Vfi - Vin | Difference |
| Vin, Vfi | Voltage value |
| ΔD | Difference |
| ΔDth | Threshold value |

## Claims

**1.** A radiation image capturing apparatus, comprising:

a detecting section (P) that includes:

a plurality of scanning lines (5) and a plurality of signal lines (6) arranged to intersect with each other, and a plurality of radiation detection elements (7) that are two-dimensionally aligned, each element being individually aligned_in respective regions partitioned by the plurality of scanning lines (5) and the plurality of signal lines (6) ; a scanning drive unit (15) that applies a voltage to each of the scanning lines (5) while switching the voltage between an on voltage and an off voltage;

switch units (8) each connected to each of the scanning lines (5), the switch units (8) configured to discharge electric charges accumulated in the radiation detection elements (7) to the signal lines (6) when the on voltage is applied thereto through the scanning lines, and accumulate electric charges in the radiation detection elements (7) when an off voltage is applied thereto through the scanning lines (5);
reading circuits (17) which convert the electric charges discharged to the signal lines (6) from the radiation detection elements (7) into image data and reads out the image data during an image data readout process in which the image data is read out from the radiation detection elements; and
a controller (22) which controls at least the scanning drive unit (15) and the reading circuits (17) and causes the same to execute the image data readout process from the radiation detection elements,
wherein the controller (22) causes the reading circuits (17) to periodically perform a readout operation before radiation image capturing operation in a state where each of the switch units (8) is in an off state by applying the off voltage to all of the scanning lines (5) from the scanning drive unit (15), causes the reading circuits (17) to repeatedly execute a leaked data readout process in which the electric charges leaked from the radiation detection elements through the switch units (8) are converted into leaked data, and detects initiation of irradiation at a point when the read-out leaked data exceeds a threshold value, and

wherein, in the leaked data readout process repeatedly executed before the radiation image capturing operation, the controller (22) causes the scanning drive unit (15) to apply the on voltage to each of the scanning lines (5) to execute a reset process for removing an excessive electric charge from each of the radiation detection elements (7), between the leaked data readout process and the next leaked data readout process, and wherein, when applying the on voltage to each of the scanning lines (5) in the reset process before the radiation image capturing operation, the scanning drive unit (15) executes the reset process-by applying the on voltage to the scanning lines other than the scanning lines which are respectively, in the detecting section (P), adjacent to the scanning lines (5) to which the on voltage was applied in the previous reset process.

2. The radiation image capturing apparatus of claim 1, wherein, when applying the on voltage to each of the scanning lines in the reset process before the radiation image capturing operation, the scanning drive unit (15) applies the on voltage simultaneously to the plurality of scanning lines which are not adjacent to each other in the detecting section (P), and executes the reset process.

3. The radiation image capturing apparatus of claim 1, wherein, in the leaked data readout process repeatedly executed before the radiation image capturing operation, the controller (22) causes the scanning drive unit (15) to apply the on voltage to each of the scanning lines (5) to execute the image data readout process in order to remove an excessive electric charge from each of the radiation detection elements between the leaked data readout process and the next leaked data readout process.

4. The radiation image capturing apparatus of claim 3, wherein, when applying the on voltage to each of the scanning lines (5) in the image data readout process before the radiation image capturing operation, the scanning drive unit executes the image data readout process by applying the on voltage to the scanning lines other than the scanning lines which are respectively, in the detecting section (P), adjacent to the scanning lines (5) to which the on voltage was applied in the previous image data readout process.

5. The radiation image capturing apparatus of claim 3, wherein, when applying the on voltage to each of the scanning lines (5) in the image data readout process before the radiation image capturing operation, the scanning drive unit (15) applies the on voltage simultaneously to the plurality of scanning lines which are not adjacent to each other on the detecting section (P), and executes the image data readout process.

6. The radiation image capturing apparatus of any one of claims 1 to 5, wherein the controller (22) sets the threshold value while updating the same based on a history of each piece of the leaked data read out in the leaked data readout process which is periodically repeated.

7. The radiation image capturing apparatus of any one of claims 1 to 6, wherein the controller (22) extracts a maximum value and a minimum value from among respective pieces of the leaked data read out in the same leaked data readout process for each of the reading circuits (17), calculates a difference obtained by deducting the minimum value from the maximum value, and detects initiation of irradiation at a point when the calculated difference exceeds the threshold value.

8. The radiation image capturing apparatus of claim 7, further comprising
a plurality of reading ICs in each of which a prescribed number of the reading circuits (17) are formed,
wherein the controller (22) calculates an average value of the respective pieces of leaked data read out for each of the reading circuits in the same leaked data readout process for each of the reading ICs, instead of the respective pieces of leaked data read out in the same leaked data readout process for each of the reading circuits, and extracts the maximum value and the minimum value from among the average values of the respective pieces of the leaked data for each of the reading ICs.

9. The radiation image capturing apparatus of any one of claims 1 to 6, wherein the controller (22) calculates, for each of the reading circuits (17), a moving average of the respective pieces of the leaked data read out for each of the reading_circuits in a predetermined number of rounds of past leaked data readout processes including the leaked data readout process immediately before the current round of the leaked data readout process, extracts a maximum value and a minimum value from among values obtained by deducting the moving average from the respective pieces of the leaked data_currently read out for each of the reading circuits, calculates a difference by deducting the minimum value from the maximum value, and detects initiation of radioactive irradiation at a point when the calculated difference exceeds the threshold value.

10. The radiation image capturing apparatus of claim 9, further comprising
a plurality of reading ICs in each of which a prescribed number of the reading circuits (17) are formed,
wherein the controller (22) calculates a moving average of the average values of the respective pieces of the leaked data read out in a predetermined number of rounds of past leaked data readout processes including the leaked data readout process immediately before the current round of the leaked data readout process for each of the reading ICs, instead of calculating the moving average for each of the reading circuits, and
extracts the maximum value and the minimum value from among respective values obtained by deducting the moving average of the average values from the average value of the respective pieces of the leaked data currently read out for each of the reading circuits, for each of the reading ICs.

**11.** The radiation image capturing apparatus of any one of claims 1 to 10, wherein the reading circuit (17) includes:

an amplifier circuit which converts the electric charges discharged from the radiation detection element or the electric charges leaked from the radiation detection element through the switch unit (8) into a voltage value and outputs the same; and
a correlated double sampling circuit which holds the voltage value outputted by the amplifier circuit before the electric charges flow into the amplifier circuit, holds the voltage value outputted by the amplifier circuit after the electric charges flow into the amplifier circuit, and outputs a difference between the former voltage value and the latter voltage value as the image data or the leaked data,
wherein the correlated double sampling circuit is controlled so that a time span between the two holding operations during the leaked data readout process is longer than a time span between the two holding operations during the image data readout process.

**12.** The radiation image capturing apparatus of any one of claim 1 to 11, wherein, once the controller detects initiation of radioactive irradiation, the controller moves to an electric charge accumulation mode while maintaining a state where the respective switch units is turned in the off state by applying the off voltage to all of the scanning lines from the scanning drive unit, causes the reading circuits (17) to execute the leaked data readout process repeatedly by causing the reading circuits to carry out readout operations periodically, and, once end of radioactive irradiation is detected at a point when the read-out leaked data becomes the threshold value or smaller, the controller causes the scanning drive unit to apply the on voltage sequentially to the respective scanning lines, and causes the reading circuits to perform readout operations sequentially and execute the image data readout process for reading out image data from the respective radiation detection elements.

**13.** The radiation image capturing apparatus of_any one of claims 1 to 12, wherein, after the image data readout process is ended, in a state where no radiation is emitted, the controller (22):

• switches the voltage applied by the scanning drive unit to each of the scanning lines between the on voltage and the off voltage at the same timing as

(a) the leaked data readout process, before the radiation image capturing operation,
(b) transition to the electric charge accumulation mode, and
(c) the image data readout process; and

• executes

(a') the leaked data readout process,
(b') transition to the electric charge accumulation mode, and
(c') an offset correction value readout process for reading out an offset correction value from each of the radiation detection elements.

## Patentansprüche

**1.** Strahlungsbilderfassungsvorrichtung, die Folgendes beinhaltet:

einen Detektierabschnitt (P) der Folgendes umfasst:

eine Vielzahl von Abtastleitungen (5) und eine Vielzahl von Signalleitungen (6), die angeordnet sind, um sich gegenseitig zu schneiden, und
eine Vielzahl von Strahlungsdetektionselementen (7), die zweidimensional ausgerichtet sind, wobei jedes Element individuell in jeweiligen Regionen ausgerichtet ist, die durch die Vielzahl von Abtastleitungen (5) und die Vielzahl von Signalleitungen (6) aufgeteilt sind;

eine Abtastansteuereinheit (15), die an jede der Abtastleitungen (5) eine Spannung angelegt, während die Spannung zwischen einer Ein-Spannung und einer Aus-Spannung geschaltet wird;
Schalteinheiten (8), die jeweils mit jeder der Abtastleitungen (5) verbunden sind, wobei die Schalteinheiten (8) konfiguriert sind, um elektrische Ladungen, die in den Strahlungsdetektionselementen (7) akkumuliert wurden, zu den Signalleitungen (6) zu entladen, wenn die Ein-Spannung über die Abtastleitungen daran angelegt wird, und elektrische Ladungen in den Strahlungsdetektionselementen (7) zu akkumulieren, wenn eine Aus-Spannung über die Abtastleitungen (5) daran angelegt wird;
Leseschaltungen (17), welche die von den Strahlungsdetektionselementen (7) zu den Signalleitungen (6) entladenen elektrischen Ladungen zu Bilddaten konvertieren und die Bilddaten während eines Bilddatenausleseprozesses lesen, bei dem die Bilddaten aus den Strahlungsdetektionselementen ausgelesen werden; und
eine Steuereinheit (22), die mindestens die Abtastansteuereinheit (15) und die Leseschaltungen (17) steuert und diese veranlasst, den Bilddatenausleseprozess aus den Strahlungsdetektionselementen auszuführen,
wobei die Steuereinheit (22) die Leseschaltungen (17) veranlasst, vor einem Strahlungsbilderfassungsvorgang in einem Status, bei dem jede der Schalteinheiten (8) in einem Aus-Status ist, durch Anlegen der Aus-Spannung an alle Abtastleitungen (5) von der Abtastansteuereinheit (15) periodisch einen Auslesevorgang durchzuführen, die Leseschaltungen (17) veranlasst, wiederholt einen Geleckte-Daten-Ausleseprozess durchzuführen, bei dem die aus den Strahlungsdetektionselementen durch die Schalteinheiten (8) geleckten elektrischen Ladungen zu geleckten Daten konvertiert werden, und Initiierung der Bestrahlung an einem Punkt detektiert, wenn die ausgelesenen geleckten Daten einen Schwellenwert überschreiten, und
wobei im Geleckte-Daten-Ausleseprozess, der wiederholt vor dem Strahlungsbilderfassungsvorgang ausgeführt wird, die Steuereinheit (22) die Abtastansteuereinheit (15) veranlasst, die Ein-Spannung an jede der Abtastleitungen (5) anzulegen, um zwischen dem Geleckte-Daten-Ausleseprozess und dem nächsten Geleckte-Daten-Ausleseprozess einen Rücksetzprozess zum Entfernen einer übermäßigen elektrischen Ladung von jedem der Strahlungsdetektionselemente (7) auszuführen, und wobei, wenn die Ein-Spannung an jede der Abtastleitungen (5) im Rücksetzprozess vor dem Strahlungsbilderfassungsvorgang angelegt wird, die Abtastansteuereinheit (15) den Rücksetzprozess durch Anlegen der Ein-Spannung an die Abtastleitungen ausführt, die sich von den Abtastleitungen unterscheiden, die jeweils im Detektierabschnitt (P) an die Abtastleitungen (5) angrenzen, an die die Ein-Spannung im vorangegangenen Rücksetzprozess angelegt wurde.

2. Strahlungsbilderfassungsvorrichtung gemäß Anspruch 1, wobei, wenn die Ein-Spannung an jede der Abtastleitungen im Rücksetzprozess vor dem Strahlungsbilderfassungsvorgang angelegt wird, die Abtastansteuereinheit (15) die Ein-Spannung gleichzeitig an die Vielzahl von Abtastleitungen anlegt, die im Detektierabschnitt (P) nicht aneinander angrenzen, und den Rücksetzprozess ausführt.

3. Strahlungsbilderfassungsvorrichtung gemäß Anspruch 1, wobei im Geleckte-Daten-Ausleseprozess, der wiederholt vor dem Strahlungsbilderfassungsvorgang ausgeführt wird, die Steuereinheit (22) die Abtastansteuereinheit (15) veranlasst, die Ein-Spannung an jede der Abtastleitungen (5) anzulegen, um zwischen dem Geleckte-Daten-Ausleseprozess und dem nächsten Geleckte-Daten-Ausleseprozess den Bilddatenausleseprozess auszuführen, um eine übermäßige elektrische Ladung von jedem der Strahlungsdetektionselemente zu entfernen.

4. Strahlungsbilderfassungsvorrichtung gemäß Anspruch 3, wobei, wenn die Ein-Spannung an jede der Abtastleitungen (5) im Bilddatenausleseprozess vor dem Strahlungsbilderfassungsvorgang angelegt wird, die Abtastansteuereinheit den Bilddatenausleseprozess durch Anlegen der Ein-Spannung an die Abtastleitungen ausführt, die sich von den Abtastleitungen unterscheiden, die jeweils im Detektierabschnitt (P) an die Abtastleitungen (5) angrenzen, an die die Ein-Spannung im vorangegangenen Bilddatenausleseprozess angelegt wurde.

5. Strahlungsbilderfassungsvorrichtung gemäß Anspruch 3, wobei, wenn die Ein-Spannung an jede der Abtastleitungen (5) im Bilddatenausleseprozess vor dem Strahlungsbilderfassungsvorgang angelegt wird, die Abtastansteuereinheit (15) die Ein-Spannung gleichzeitig an die Vielzahl von Abtastleitungen anlegt, die auf dem Detektierabschnitt (P) nicht aneinander angrenzen, und den Bilddatenausleseprozess ausführt.

6. Strahlungsbilderfassungsvorrichtung gemäß einem der Ansprüche 1 bis 5, wobei die Steuereinheit (22) den Schwellenwert einstellt während sie denselben basierend auf einer Geschichte für jeden Teil der im Geleckte-Daten-Ausleseprozess, der periodisch wiederholt wird, ausgelesenen geleckten Daten aktualisiert.

7. Strahlungsbilderfassungsvorrichtung gemäß einem der Ansprüche 1 bis 6, wobei die Steuereinheit (22) einen Maximalwert und einen Minimalwert aus jeweiligen Teilen der im gleichen Geleckte-Daten-Ausleseprozess für jede der Leseschaltungen (17) ausgelesenen geleckten Daten extrahiert, eine Differenz berechnet, die durch Subtrahieren des Minimalwerts vom Maximalwert erhalten wird, und Initiierung der Bestrahlung an einem Punkt detektiert, wenn die berechnete Differenz den Schwellenwert überschreitet.

8. Strahlungsbilderfassungsvorrichtung gemäß Anspruch 7, die ferner die Folgendes beinhaltet:

eine Vielzahl von Lese-ICs, in denen jeweils eine vorgeschriebene Anzahl der Leseschaltungen (17) gebildet sind,
wobei die Steuereinheit (22) einen Durchschnittswert der jeweiligen Teile der für jede der Leseschaltungen im gleichen Geleckte-Daten-Ausleseprozess ausgelesenen geleckten Daten für jede der Lese-ICs berechnet, anstatt der jeweiligen Teile der im gleichen Geleckte-Daten-Ausleseprozess ausgelesenen geleckten Daten für jede der Leseschaltungen, und den Maximalwert und den Minimalwert aus den Durchschnittswerten der jeweiligen Teile der geleckten Daten für jede der Lese-ICs extrahiert.

**9.** Strahlungsbilderfassungsvorrichtung gemäß einem der Ansprüche 1 bis 6, wobei die Steuereinheit (22), für jede der Leseschaltungen (17), einen gleitenden Durchschnitt der jeweiligen Teile der für jede der Leseschaltungen in einer vorbestimmten Anzahl von Runden von vergangenen Geleckte-Daten-Ausleseprozessen, einschließlich des Geleckte-Daten-Ausleseprozesses unmittelbar vor der aktuellen Runde des Geleckte-Daten-Ausleseprozesses, ausgelesenen geleckten Daten berechnet, einen Maximalwert und einen Minimalwert aus Werten extrahiert, die durch Subtrahieren des gleitenden Durchschnitts von den jeweiligen Teilen der für jede der Leseschaltungen aktuell ausgelesenen geleckten Daten erhalten werden, eine Differenz durch Subtrahieren des Minimalwerts vom Maximalwert berechnet und Initiierung der radioaktiven Bestrahlung an einem Punkt detektiert, wenn die berechnete Differenz den Schwellenwert überschreitet.

**10.** Strahlungsbilderfassungsvorrichtung gemäß Anspruch 9, die ferner die Folgendes beinhaltet:

eine Vielzahl von Lese-ICs, in denen jeweils eine vorgeschriebene Anzahl der Leseschaltungen (17) gebildet sind,
wobei die Steuereinheit (22) einen gleitenden Durchschnitt der Durchschnittswerte der jeweiligen Teile der in einer vorbestimmten Anzahl von Runden von vergangenen Geleckte-Daten-Ausleseprozessen, einschließlich des Geleckte-Daten-Ausleseprozesses unmittelbar vor der aktuellen Runde des Geleckte-Daten-Ausleseprozesses, ausgelesenen geleckten Daten für jede der Lese-ICs berechnet, anstatt den gleitenden Durchschnitt für jede der Leseschaltungen zu berechnen, und
den Maximalwert und den Minimalwert aus den jeweiligen Werten extrahiert, die durch Subtrahieren des gleitenden Durchschnitts der Durchschnittswerte vom Durchschnittswert der jeweiligen Teile der für jede der Leseschaltungen aktuell ausgelesenen geleckten Daten erhalten werden, für jede der Lese-ICs.

**11.** Strahlungsbilderfassungsvorrichtung gemäß einem der Ansprüche 1 bis 10,
wobei die Leseschaltung (17) Folgendes umfasst:

eine Verstärkerschaltung, die die vom Strahlungsdetektionselement entladenen elektrischen Ladungen oder die vom Strahlungsdetektionselement durch die Schalteinheit (8) geleckten elektrischen Ladungen zu einem Spannungswert konvertiert und denselben ausgibt; und
eine korrelierte Doppelsamplingschaltung, die den von der Verstärkerschaltung ausgegebenen Spannungswert hält, bevor die elektrischen Ladungen in die Verstärkerschaltung fließen, den von der Verstärkerschaltung ausgegebenen Spannungswert hält, nachdem die elektrischen Ladungen in die Verstärkerschaltung fließen, und eine Differenz zwischen dem ersteren Spannungswert und dem letzteren Spannungswert als die Bilddaten oder die geleckten Daten ausgibt,
wobei die korrelierte Doppelsamplingschaltung gesteuert wird, sodass eine Zeitspanne zwischen den zwei Haltevorgängen während des Geleckte-Daten-Ausleseprozesses länger ist als eine Zeitspanne zwischen den zwei Haltevorgängen während des Bilddatenausleseprozesses.

**12.** Strahlungsbilderfassungsvorrichtung gemäß einem der Ansprüche 1 bis 11, wobei, sobald die Steuereinheit Initiierung der radioaktiven Bestrahlung detektiert, die Steuereinheit auf einen Modus der Akkumulation der elektrischen Ladung übergeht, während ein Status aufrechterhalten wird, bei dem die jeweiligen Schalteinheiten durch Anlegen der Aus-Spannung an alle Abtastleitungen von der Abtastansteuereinheit auf den Aus-Status geschaltet werden, die Leseschaltungen (17) veranlasst, den Geleckte-Daten-Ausleseprozess wiederholt auszuführen, indem die Leseschaltungen veranlasst werden, Auslesevorgänge periodisch vorzunehmen, und, sobald das Ende der radioaktiven Bestrahlung an einem Punkt detektiert wird, wenn die ausgelesenen geleckten Daten der Schwellenwert oder kleiner werden, die Steuereinheit die Abtastansteuereinheit veranlasst, die Ein-Spannung sequenziell an die jeweiligen Abtastleitungen anzulegen und die Leseschaltungen veranlasst, Auslesevorgänge sequenziell durchzuführen und den Bilddatenausleseprozess zum Auslesen der Bilddaten aus den jeweiligen Strahlungsdetektionselementen auszuführen.

**13.** Strahlungsbilderfassungsvorrichtung gemäß einem der Ansprüche 1 bis 12, wobei, nachdem der Bilddatenausleseprozess beendet wurde, in einem Status, im dem keine Strahlung emittiert wird, die Steuereinheit (22):

- die durch die Abtastansteuereinheit an jede der Abtastleitungen angelegte Spannung zwischen der EinSchaltung und der Aus-Schaltung schaltet, zur gleichen Zeit wie

  (a) der Geleckte-Daten-Ausleseprozess, vor dem Strahlungsbilderfassungsvorgang,
  (b) der Übergang auf den Modus der Akkumulation der elektrischen Ladung, und
  (c) der Bilddatenausleseprozess; und

- Folgendes ausführt

  (a') den Geleckte-Daten-Ausleseprozess,
  (b') den Übergang auf den Modus der Akkumulation der elektrischen Ladung, und
  (c') einen Offsetkorrekturwertausleseprozess zum Auslesen eines Offsetkorrekturwerts aus jedem der Strahlungsdetektionselemente.

## Revendications

**1.** Appareil de capture d'image radiographique, comprenant :

une section de détection (P) qui comporte :

une pluralité de lignes de balayage (5) et une pluralité de lignes de signal (6) disposées de telle façon qu'elles se croisent les unes les autres, et
une pluralité d'éléments détecteurs de radiation (7) qui sont alignés dans deux dimensions, chaque élément étant aligné individuellement dans des régions respectives divisées par la pluralité de lignes de balayage (5) et la pluralité de lignes de signal (6) ;

une unité de commande de balayage (15) qui applique une tension à chacune des lignes de balayage (5) tandis qu'elle fait basculer la tension entre une tension d'activation et une tension de désactivation ;
des unités de commutation (8) connectées chacune à chacune des lignes de balayage (5), les unités de commutation (8) étant configurées pour décharger des charges électriques accumulées dans les éléments détecteurs de radiation (7) vers les lignes de signal (6) lorsque la tension d'activation leur est appliquée par le biais des lignes de balayage et pour accumuler des charges électriques dans les éléments détecteurs de radiation (7) lorsqu'une tension de désactivation leur est appliquée par le biais des lignes de balayage (5) ;
des circuits de lecture (17) qui convertissent les charges électriques déchargées vers les lignes de signal (6) à partir des éléments détecteurs de radiation (7) en données d'image et qui lisent les données d'image pendant un processus de lecture de données d'image dans lequel les données d'image sont lues à partir des éléments détecteurs de radiation ; et
un contrôleur (22) qui commande au moins l'unité de commande de balayage (15) et les circuits de lecture (17) et leur fait exécuter le processus de lecture de données d'image à partir des éléments détecteurs de radiation,
dans lequel le contrôleur (22) fait périodiquement effectuer aux circuits de lecture (17) une opération de lecture avant une opération de capture d'image de radiation dans un état où chacune des unités de commutation (8) se trouve dans un état désactivé en appliquant la tension de désactivation à toutes les lignes de balayage (5) à partir de l'unité de commande de balayage (15), fait exécuter de manière répétée aux circuits de lecture (17) un processus de lecture de données de fuite dans lequel les charges électriques qui ont fui des éléments détecteurs de radiation par le biais des unités de commutation (8) sont converties en données de fuite, et détecte le début de l'irradiation à un point où les données de fuite lues dépassent une valeur seuil, et
dans lequel, dans le processus de lecture de données de fuite exécuté de manière répétée avant l'opération de capture d'image de radiation, le contrôleur (22) fait appliquer la tension d'activation à chacune des lignes de balayage (5) par l'unité de commande de balayage (15) afin d'exécuter un processus de remise à zéro destiné à éliminer une charge électrique excessive de chacun des éléments détecteurs de radiation (7), entre le processus de lecture de données de fuite et le processus de lecture de données de fuite suivant, et dans lequel, lors de l'application de la tension d'activation à chacune des lignes de balayage (5) dans le processus de remise à zéro avant l'opération de capture d'image de radiation, l'unité de commande de balayage (15) exécute le processus de remise à zéro en appliquant la tension d'activation aux lignes de balayage autres que les lignes de balayage qui sont respectivement, dans la section de détection (P), adjacentes aux lignes de balayage (5) auxquelles la tension d'activation a été appliquée dans le processus de remise à zéro précédent.

**2.** Appareil de capture d'image radiographique selon la revendication 1, dans lequel, lors de l'application de la tension d'activation à chacune des lignes de balayage dans le processus de remise à zéro avant l'opération de capture d'image de radiation, l'unité de commande de balayage (15) applique la tension d'activation simultanément à la pluralité de lignes de balayage qui ne sont pas adjacentes les unes aux autres dans la section de détection (P), et exécute le processus de remise à zéro.

**3.** Appareil de capture d'image radiographique selon la revendication 1, dans lequel, dans le processus de lecture de données de fuite exécuté de manière répétée avant l'opération de capture d'image de radiation, le contrôleur (22) fait appliquer la tension d'activation à chacune des lignes de balayage (5) par l'unité de commande de balayage (15) pour exécuter le processus de lecture de données d'image afin d'éliminer une charge électrique excessive de chacun des éléments détecteurs de radiation entre le processus de lecture de données de fuite et le processus de lecture de données de fuite suivant.

**4.** Appareil de capture d'image radiographique selon la revendication 3, dans lequel, lors de l'application de la tension d'activation à chacune des lignes de balayage (5) dans le processus de lecture de données d'image avant l'opération de capture d'image de radiation, l'unité de commande de balayage exécute le processus de lecture de données d'image en appliquant la tension d'activation aux lignes de balayage autres que les lignes de balayage qui sont respectivement, dans la section de détection (P), adjacentes aux lignes de balayage (5) auxquelles la tension d'activation a été appliquée dans le processus de lecture de données d'image précédent.

**5.** Appareil de capture d'image radiographique selon la revendication 3, dans lequel, lors de l'application de la tension d'activation à chacune des lignes de balayage (5) dans le processus de lecture de données d'image avant l'opération de capture d'image de radiation, l'unité de commande de balayage (15) applique la tension d'activation simultanément à la pluralité de lignes de balayage qui ne sont pas adjacentes les unes aux autres dans la section de détection (P), et exécute le processus de lecture de données d'image.

**6.** Appareil de capture d'image radiographique selon l'une quelconque des revendications 1 à 5, dans lequel le contrôleur (22) fixe la valeur seuil tout en l'actualisant sur la base d'un historique de chaque élément des données de fuite lues dans le processus de lecture de données de fuite qui est répété périodiquement.

**7.** Appareil de capture d'image radiographique selon l'une quelconque des revendications 1 à 6, dans lequel le contrôleur (22) extrait une valeur maximum et une valeur minimum à partir des éléments respectifs des données de fuite lues dans le même processus de lecture de données de fuite pour chacun des circuits de lecture (17), calcule une différence obtenue en soustrayant la valeur minimum de la valeur maximum, et détecte le début de l'irradiation à un point où la différence calculée dépasse la valeur seuil.

**8.** Appareil de capture d'image radiographique selon la revendication 7, comprenant en outre :

une pluralité de circuits intégrés de lecture dans chacun desquels est formé un nombre prescrit des circuits de lecture (17),
dans lequel le contrôleur (22) calcule une valeur moyenne des éléments respectifs des données de fuite lues pour chacun des circuits de lecture dans le même processus de lecture de données de fuite pour chacun des circuits intégrés de lecture, au lieu des éléments respectifs des données de fuite lues dans le même processus de lecture de données de fuite pour chacun des circuits de lecture, et extrait la valeur maximum et la valeur minimum parmi les valeurs moyennes des éléments respectifs des données de fuite pour chacun des circuits intégrés de lecture.

**9.** Appareil de capture d'image radiographique selon l'une quelconque des revendications 1 à 6, dans lequel le contrôleur (22) calcule, pour chacun des circuits de lecture (17), une moyenne mobile des éléments respectifs des données de fuite lues pour chacun des circuits de lecture dans un nombre prédéterminé de cycles de processus antérieurs de lecture de données de fuite incluant le processus de lecture de données de fuite qui précède immédiatement le cycle en cours du processus de lecture de données de fuite, extrait une valeur maximum et une valeur minimum parmi les valeurs obtenues en soustrayant la moyenne mobile des éléments respectifs des données de fuite en cours de lecture pour chacun des circuits de lecture, calcule une différence en soustrayant la valeur minimum de la valeur maximum, et détecte le début de l'irradiation radioactive à un point où la différence calculée dépasse la valeur seuil.

**10.** Appareil de capture d'image radiographique selon la revendication 9, comprenant en outre :

une pluralité de circuits intégrés de lecture dans chacun desquels est formé un nombre prescrit des circuits de lecture (17),
dans lequel le contrôleur (22) calcule une moyenne mobile des valeurs moyennes des éléments respectifs des données de fuite lues dans un nombre prédéterminé de cycles de processus antérieurs de lecture de données de fuite incluant le processus de lecture de données de fuite qui précède immédiatement le cycle en cours du processus de lecture de données de fuite pour chacun des circuits intégrés de lecture, au lieu de calculer la moyenne mobile pour chacun des circuits de lecture, et
extrait la valeur maximum et la valeur minimum parmi les valeurs respectives obtenues en soustrayant la moyenne mobile des valeurs moyennes de la valeur moyenne des éléments respectifs des données de fuite en cours de lecture pour chacun des circuits de lecture, pour chacun des circuits intégrés de lecture.

**11.** Appareil de capture d'image radiographique selon l'une quelconque des revendications 1 à 10, dans lequel le circuit de lecture (17) comporte :

un circuit amplificateur qui convertit les charges électriques déchargées de l'élément détecteur de radiation ou les charges électriques ayant fui de l'élément détecteur de radiation par le biais de l'unité de commutation (8) en une valeur de tension, et qui délivre cette valeur en sortie ; et
un circuit de double échantillonnage corrélé qui maintient la valeur de tension délivrée en sortie par le circuit amplificateur avant que les charges électriques s'écoulent dans le circuit amplificateur, qui maintient la valeur de tension délivrée en sortie par le circuit amplificateur après que les charges électriques se sont écoulées dans le circuit amplificateur, et qui délivre en sortie une différence entre la première valeur de tension et la seconde valeur de tension en tant que données d'image ou en tant que données de fuite,
dans lequel le circuit de double échantillonnage corrélé est commandé de telle façon qu'un intervalle de temps entre les deux opérations de maintien pendant le processus de lecture de données de fuite est plus long qu'un intervalle de temps entre les deux opérations de maintien pendant le processus de lecture de données d'image.

**12.** Appareil de capture d'image radiographique selon l'une quelconque des revendications 1 à 11, dans lequel, une fois que le contrôleur a détecté le début de l'irradiation radioactive, le contrôleur passe en mode d'accumulation de charges électriques tout en maintenant un état dans lequel les unités de commutation respectives sont mises à l'état désactivé en appliquant la tension de désactivation à toutes les lignes de balayage à partir de l'unité de commande de balayage, fait exécuter le processus de lecture de données de fuite de manière répétée par les circuits de lecture (17) en faisant effectuer périodiquement des opérations de lecture par lesdits circuits de lecture et, une fois que la fin de l'irradiation radioactive est détectée à un point où les données de fuite lues deviennent égales ou inférieures à la valeur seuil, le contrôleur fait appliquer séquentiellement la tension d'activation aux lignes de balayage respectives par l'unité de commande de balayage et fait effectuer séquentiellement des opérations de lecture par les circuits de lecture et exécuter le processus de lecture de données d'image pour lire des données d'image à partir des éléments détecteurs de radiation respectifs.

**13.** Appareil de capture d'image radiographique selon l'une quelconque des revendications 1 à 12, dans lequel, après la fin du processus de lecture de données d'image, dans un état où aucune radiation n'est émise, le contrôleur (22) :

- fait basculer la tension appliquée par l'unité de commande de balayage à chacune des lignes de balayage entre la tension d'activation et la tension de désactivation en même temps que

  (a) le processus de lecture de données de fuite, avant l'opération de capture d'image de radiation,
  (b) le passage en mode d'accumulation de charges électriques, et
  (c) le processus de lecture de données d'image ; et

- exécute

  (a') le processus de lecture de données de fuite,
  (b') le passage en mode d'accumulation de charges électriques, et
  (c') un processus de lecture de valeur de correction de décalage pour lire une valeur de correction de décalage à partir de chacun des éléments détecteurs de radiation.

FIG.1

1
2
R
X
37
2A
2B
38
39
X
36

FIG.2

1
2A
35
3
4
31
P
R
2
2B
32
32
33
34
33
32
32

FIG.3

4
4a
11
6
8
7
P
r
5
11
9
10

FIG.4

6
8g
8s
9
5
8d
r
8
7
Y
Y

FIG.5

77
76
75
74
73
72
4a
78
7
79
6
82
84b
8d
8
79
83
8s
84a
4
H
79
78
7
9
81
8g
71
77
76
75
74
73
72

# FIG.6

4
3
4a
12
13
5,6,10
7
11
12a
31
4b
33

1
P
15,15b
15,15c
15,15a
POWER SOURCE CIRCUIT
GATE DRIVER
5,L1
5,L2
5,L3
5,Lx
6
8
74
7
G
D
S
78
9
17
10
18
CDS
19
20
A/D CONVERTOR
21
ANALOG MULTIPLEXER
16
39
22
CONTROLLER
BIAS POWER SOURCE
14
41
BATTERY
STORAGE SECTION
40
42

FIG.7

FIG.8
POWER SUPPLY UNIT
18d
1
5
G
6
18
17
16
18e
19
20
18a
S
D
8
74
7
78
CDS
A/D CONVERTING CIRCUIT
$V_0$
18b
18c
9
10
14
22
40
BIAS POWER SOURCE
CONTROLLER
STORAGE SECTION

# FIG.9

TFT8(L1)
ON
OFF
TFT8(L2)
ON
OFF
TFT8(L3)
ON
OFF
TFT8(L4)
ON
OFF
TFT8(Lx)
ON
OFF

# *FIG.10*

ON
ELECTRIC CHARGE RESET SWITCH 18c
OFF
PULSE SIGNALS Sp
Sp1
Sp2
ON
TFT8(Ln)
OFF

# FIG.11

$V_{fi}$
VOLTAGE VALUE
$V_{in}$
$V_0$
0
t
$18c_{off}$
$TFT_{on}$
$TFT_{off}$
CDS HOLD
CDS HOLD

*FIG.12*

ON
ELECTRIC CHARGE RESET SWITCH 18c
OFF
PULSE SIGNALS Sp
Sp1
Sp2
ON
TFT8(Ln)
OFF

FIG.13
ELECTRIC CHARGE RESET SWITCH 18c
ON
OFF
PULSE SIGNALS Sp
Sp1
Sp2
Sp1
Sp2
Sp1
Sp2
Sp1
Sp2
TFT8(L1)
ON
OFF
TFT8(L2)
ON
OFF
...
LEAKED DATA READOUT PROCESS
LEAKED DATA READOUT PROCESS
LEAKED DATA READOUT PROCESS
LEAKED DATA READOUT PROCESS

# FIG.14

15b
GATE DRIVER
5, $L_{n-1}$
5, $L_n$
5, $L_{n+1}$
5, $L_{n+2}$
6
8
$7_{n-1}$
$7_n$
$7_{n+1}$
$7_{n+2}$
q
q
q
q
18b
18
18a
Dleak

FIG.15

Dleak
Dth
t
t1

# FIG.16

Ioff
Ion
LEAKAGE CURRENT [ × 10 -14A]
CURRENT [ × 10 -7A]
9
8
7
6
5
4
3
2
1
0
14
12
10
8
6
4
2
0
Ion
Ioff
0
10
20
30
40
50
60
70
TEMPERATURE T [℃]

# FIG.17

Dleak_max
Dth_pro
t

FIG.18A

Dleak_max
Dth
t

FIG.18B

Dleak_max
Dth
t

FIG.19

ELECTRIC CHARGE RESET SWITCH 18c
ON
OFF
PULSE SIGNALS Sp
Sp1
Sp2
Sp1
Sp2
TFT8(L1)
ON
OFF
TFT8(L2)
ON
OFF
...
LEAKED DATA READOUT PROCESS
RESET PROCESS
LEAKED DATA READOUT PROCESS
RESET PROCESS

FIG.20
ELECTRIC CHARGE RESET SWITCH 18c
ON
OFF
PULSE SIGNALS Sp
Sp1
Sp2
Sp1
Sp2
Sp1
Sp2
Sp1
Sp2
TFT8(L1)
ON
OFF
TFT8(L2)
ON
OFF
...
LEAKED DATA READOUT PROCESS
IMAGE DATA READOUT PROCESS
LEAKED DATA READOUT PROCESS
IMAGE DATA READOUT PROCESS

FIG.21

Dleak
t
t1
t2

FIG.22

1
2
R
P
37
3
F
38
6
6

*FIG.23*

ΔD
0
t
t1
t2

# FIG.24

5
r
12a
15b
12a
5,Lx
6
16
16

FIG.25

1
4
A
P
3
B
6
6

POWER SUPPLY UNIT
CDS
A/D CONVERTING CIRCUIT
BIAS POWER SOURCE
CONTROLLER
STORAGE SECTION
G
S
D
$V_0$
C1
C2
C3
C4
Sw1
Sw2
Sw3
1
5
6
7
8
9
10
14
16
17
18
18a
18c
18d
18e
19
20
22
40
74
78


FIG.26

# FIG.27

8s
8d
84a
84b
82
81
8g

80b
80a
78
77
76
75
74
73
72
4a
4
7
79
6
81
8d
84b
8g
8
83
82
84a
8s
Ha
79
71
7
85
9
9a
Hb
78
77
76
75
74
73
72

FIG.28

# FIG.29

ELECTRIC CHARGE RESET SWITCH 18c
ON
OFF
PULSE SIGNALS Sp
Sp1
Sp2
Sp1
Sp2
TFT8(L1)
ON
OFF
TFT8(L2)
ON
OFF
...
LEAKED DATA READOUT PROCESS
RESET PROCESS
LEAKED DATA READOUT PROCESS

# FIG.30

$V_{fi}$
VOLTAGE VALUE
$V_{in}$
$V_0$
0
t
$18_{coff}$
CDS HOLD
CDS HOLD

# FIG.31

1
2
3
4
ELECTRIC CHARGE RESET SWITCH 18c
ON
OFF
PULSE SIGNALS Sp
TFT8(L1)
ON
OFF
TFT8(L2)
ON
OFF
TFT8(L3)
ON
OFF
TFT8(L4)
ON
OFF
TFT8(L5)
ON
OFF
TFT8(Lx)
ON
OFF
START OF RADIATION

FIG.32

p
5
L3
L4
L5

*FIG.33*

1
2
3
4
5
ELECTRIC CHARGE RESET SWITCH 18c
ON
OFF
PULSE SIGNALS Sp
TFT8(L1)
ON
OFF
TFT8(L2)
ON
OFF
TFT8(L3)
ON
OFF
TFT8(L4)
ON
OFF
TFT8(L5)
ON
OFF
TFT8(Lx)
ON
OFF
START OF RADIATION

# FIG.34

p
5
L3
L4
L5
L6

FIG.35

1
2
3
4
5
ELECTRIC CHARGE RESET SWITCH 18c
ON
OFF
PULSE SIGNALS Sp
TFT8(L1)
ON
OFF
TFT8(L2)
ON
OFF
TFT8(L3)
ON
OFF
TFT8(L4)
ON
OFF
TFT8(L5)
ON
OFF
TFT8(Lx)
ON
OFF
START OF RADIATION

# FIG.36

1
2
3
4
ELECTRIC CHARGE RESET SWITCH 18c
ON
OFF
PULSE SIGNALS Sp
TFT8(L1)
ON
OFF
TFT8(L2)
ON
OFF
TFT8(L3)
ON
OFF
TFT8(L4)
ON
OFF
TFT8(L129)
ON
OFF
TFT8(L130)
ON
OFF
TFT8(L131)
ON
OFF
TFT8(L132)
ON
OFF

# FIG.37

ELECTRIC CHARGE RESET SWITCH 18c
PULSE SIGNALS Sp
TFT8(L1)
TFT8(L2)
TFT8(L3)
TFT8(L4)
TFT8(L5)
TFT8(Lx)
ON
OFF
1
2
3
4
START OF RADIATION
END OF RADIATION
ELECTRIC CHARGE ACCUMULATION MODE

FIG.38
ELECTRIC CHARGE RESET SWITCH 18c
PULSE SIGNALS Sp
TFT8(L1)
TFT8(L2)
TFT8(L3)
TFT8(L4)
TFT8(L5)
...
TFT8(Lx)
ON
OFF
1
2
3
4
α
START OF RADIATION
END OF RADIATION
ELECTRIC CHARGE ACCUMULATION MODE

# FIG.39

Dleak
Dth
t1
t2
t

FIG.40

Dleak
Dth
t
t1

# FIG.41

ELECTRIC CHARGE RESET SWITCH 18c
ON
OFF
PULSE SIGNALS Sp
TFT8(L1)
TFT8(L2)
TFT8(L3)
TFT8(L4)
1
2
3
4
α
T1
T2
T3
T4
START OF RADIATION
END OF RADIATION
ELECTRIC CHARGE ACCUMULATION MODE
IMAGE DATA READOUT PROCESS

# FIG.42

ELECTRIC CHARGE RESET SWITCH 18c
ON
OFF
1
2
3
4
α
PULSE SIGNALS Sp
TFT8(L1)
T1
TFT8(L2)
T2
TFT8(L3)
T3
TFT8(L4)
T4
IMAGE DATA READOUT PROCESS
ELECTRIC CHARGE ACCUMULATION MODE
OFFSET CORRECTION VALUE READOUT PROCESS

FIG.43

ELECTRIC CHARGE RESET SWITCH 18c
ON
OFF
PULSE SIGNALS Sp
TFT8(L1)
ON
OFF
TFT8(L2)
ON
OFF
TFT8(L3)
ON
OFF
TFT8(L4)
ON
OFF
1
2
3
4
α
T1
T2
T3
T4
START OF RADIATION
END OF RADIATION
ELECTRIC CHARGE ACCUMULATION MODE
IMAGE DATA READOUT PROCESS

FIG. 44
ELECTRIC CHARGE RESET SWITCH 18c
ON
OFF
1
2
3
4
α
PULSE SIGNALS Sp
TFT8(L1)
ON
OFF
T1
TFT8(L2)
ON
OFF
T2
TFT8(L3)
ON
OFF
T3
TFT8(L4)
ON
OFF
T4
IMAGE DATA READOUT PROCESS
ELECTRIC CHARGE ACCUMULATION MODE
OFFSET CORRECTION VALUE READOUT PROCESS

FIG.45
ELECTRIC CHARGE RESET SWITCH 18c
PULSE SIGNALS Sp
TFT8(L1)
TFT8(L2)
TFT8(L3)
TFT8(L4)
ON
OFF
1
2
3
4
α
T1
T2
T3
T4
START OF RADIATION
END OF RADIATION
ELECTRIC CHARGE ACCUMULATION MODE
IMAGE DATA READOUT PROCESS

FIG.46
ELECTRIC CHARGE RESET SWITCH 18c
ON
OFF
PULSE SIGNALS Sp
TFT8(L1)
TFT8(L2)
TFT8(L3)
TFT8(L4)
1
2
3
4
α
T1
T2
T3
T4
IMAGE DATA READOUT PROCESS
ELECTRIC CHARGE ACCUMULATION MODE
OFFSET CORRECTION VALUE READOUT PROCESS

FIG.47
TFT8(L1)
TFT8(L2)
TFT8(L3)
TFT8(L4)
ON
OFF
T1
T2
T3
T4
RESET PROCESS IN LEAKED DATA READOUT PROCESS
IMAGE DATA READOUT PROCESS
OFFSET CORRECTION VALUE READOUT PROCESS

TFT8(L1)
TFT8(L2)
TFT8(L3)
TFT8(L4)
ON
OFF
T1
T2
T3
T4
RESET PROCESS IN LEAKED DATA READOUT PROCESS
IMAGE DATA READOUT PROCESS
RESET PROCESS
OFFSET CORRECTION VALUE READOUT PROCESS

FIG.48

FIG.49
ELECTRIC CHARGE RESET SWITCH 18c
ON
OFF
1
2
3
4
α
PULSE SIGNALS Sp
TFT8(L1)
T1
Ta
TFT8(L2)
T2
TFT8(L3)
T3
TFT8(L4)
T4
START OF RADIATION
END OF RADIATION
ELECTRIC CHARGE ACCUMULATION MODE
IMAGE DATA READOUT PROCESS
OFFSET CORRECTION VALUE READOUT PROCESS

*FIG.50*

O*
O1*
Oa*
0
Ta
T1
T

*FIG.51*

TFT8(Lm)
ON
OFF
ΔOlag
t
Olag
t

# FIG.52A

Olag
Olag(3)
Olag(2)
t
T2 T1 T4 T3

# FIG.52B

Olag
Olag(3)
Olag(2)
t
T2 T1 T4 T3

FIG.53

1
2
3
4
α
ELECTRIC CHARGE RESET SWITCH 18c
ON
OFF
PULSE SIGNALS SP
TFT8(L1)
TFT8(L2)
TFT8(L3)
TFT8(L4)
T1(Tc)
T2(Tc)
T3(Tc)
T4(Tc)
START OF RADIATION
END OF RADIATION
ELECTRIC CHARGE ACCUMULATION MODE
IMAGE DATA READOUT PROCESS

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 9073144 A
- JP 2006058124 A
- JP 6342099 A
- US 7211803 B
- JP 2009219538 A